# EUROPÄISCHE PATENTSCHRIFT

(11) **EP 1 430 308 B1**
(45) Veröffentlichungstag und Bekanntmachung des Hinweises auf die Patenterteilung: **22.11.2006**
(21) Anmeldenummer: 02777190.6
(22) Anmeldetag: 24.09.2002
(51) Int. Cl.: G01N 33/68, A61P 25/00

(54) **SCREENINGVERFAHREN FÜR VERSCHIEDENE INDIKATIONEN MIT BNPI UND/ODER DNPI**
SCREENING METHOD FOR DIFFERENT INDICATIONS USING BNPI AND/OR DNPI
PROCEDE DE CRIBLAGE POUR DIFFERENTES INDICATIONS A L'AIDE DE PROTEINES BNPI OU DNPI

(30) Priorität: 24.09.2001 DE 10147006; 25.09.2001 DE 10147028
(43) Veröffentlichungstag der Anmeldung: 23.06.2004
(60) Teilanmeldung: 04025751.1
(73) Patentinhaber: Grünenthal GmbH, 52078 Aachen (DE)
(72) Erfinder: WEIHE, Eberhard, 35037 Marburg (DE); SCHÄFER, Martin, K.-H., 35041 Marburg (DE)
(74) Vertreter: Graf von Stosch, Andreas
(86) Internationale Anmeldenummer: PCT/EP2002/010707
(87) Internationale Veröffentlichungsnummer: WO 2003/029828

(56) Entgegenhaltungen:
- WO-A-01/57190
- WO-A-01/64835
- WO-A-02/08384
- WO-A-96/34288
- US-A- 5 985 604
- FUJIYAMA F ET AL: "Immunocytochemical localization of candidates for vesicular glutamate transporters in the rat cerebral cortex." THE JOURNAL OF COMPARATIVE NEUROLOGY. UNITED STATES 2 JUL 2001, Bd. 435, Nr. 3, 2. Juli 2001 (2001-07-02), Seiten 379-387, XP008014105 ISSN: 0021-9967
- DENTON TRAVIS T ET AL: "Synthesis and preliminary evaluation of trans-3,4-conformationally-re stricted glutamate and pyroglutamate analogues as novel EAAT2 inhibitors." BIOORGANIC & MEDICINAL CHEMISTRY LETTERS, Bd. 12, Nr. 21, 2002, Seiten 3209-3213, XP001148334 4 November, 2002 ISSN: 0960-894X
- HERZOG ET AL.: J. O. NEUROSCIENCE, Bd. 20, 2004, Seiten 1752-1760,
- MENTIS ET AL.: PNAS, Bd. 102, Nr. 20, 2005, Seiten 7344-7349,
- DANIELS ET AL.: J. O. NEUROSCIENCE, Bd. 24, Nr. 46, 2004, Seiten 10466-10474,
- HYDE: BIOCHEM. J., Bd. 373, 2003, Seiten 1-18,

### SEQUENZPROTOKOLL

<110> GRÜNENTHAL GmbH
<120> Screeningverfahren für verschiedene Indikationenen mit BNPI und/oder DNPI
<130> GRA 3070-PCT
<140> PCT/EP 02/10707
   <141> 2002-09-24
<150> DE10147006.1
   <151> 2001-09-24
<160> 14
<170> Patent In Ver. 2.1
<210> 1
   <211> 2366
   <212> DNA
   <213> Homo Sapiens
<400> 1
   ccggcggcag gagccgccac catggagttc cgccaggagg agtttcggaa gctagcgggt 60
   cgtgctctcg ggaagctgca ccgccttctg gagaagcggc aggaaggcgc ggagacgctg 120
   gagctgagtg cggatgggcg cccggtgacc acgcagaccc gggacccgcc ggtggtggac 180
   tgcacctgct tcggcctccc tcgccgctac attatcgcca tcatgagtgg tctgggcttc 240
   tgcatcagct ttggcatccg ctgcaacctg ggcgtggcca tcgtctccat ggtcaataac 300
   agcacgaccc accgcggggg ccacgtggtg gtgcagaaag cccagttcag ctgggatcca 360
   gagactgtcg gcctcataca cggctccttt ttctggggct acattgtcac tcagattcca 420
   ggaggattta tctgtcaaaa atttgcagcc aacagagttt tcggctttgc tattgtggca 480
   acatccactc taaacatgct gatcccctca gctgcccgcg tccactatgg ctgtgtcatc 540
   ttcgtgagga tcctgcaggg gttggtagag ggggtcacat accccgcctg ccatgggatc 600
   tggagcaaat gggccccacc cttagaacgg agtcgcctgg cgacgacagc cttttgtggt 660
   tcctatgctg gggcggtggt cgcgatgccc ctcgccgggg tccttgtgca gtactcagga 720
   tggagctctg ttttctacgt ctacggcagc ttcgggatct tctggtacct gttctggctg 780
   ctcgtctcct acgagtcccc cgcgctgcac cccagcatct cggaggagga gcgcaagtac 840
   atcgaggacg ccatcggaga gagcgcgaaa ctcatgaacc ccctcacgaa gtttagcact 900
   ccctggcggc gcttcttcac gtctatgcca gtctatgcca tcatcgtggc caacttctgc 960
   cgcagctgga cgttctacct gctgctcatc tcccagcccg cctacttcga agaagtgttc 1020
   ggcttcgaga tcagcaaggt aggcctggtg tccgcgctgc cccacctggt catgaccatc 1080
   atcgtgccca tcggcggcca gatcgcggac ttcctgcgga gccgccgcat catgtccacc 1140
   accaacgtgc gcaagttgat gaactgcgga ggcttcggca tggaagccac gctgctgttg 1200
   gtggtcggct actcgcactc caagggcgtg gccatctcct tcctggtcct agccgtgggc 1260
   ttcagcggct tcgccatctc tgggttcaac gtgaaccacc tggacatagc cccgcgctac 1320
   gccagcatcc tcatgggcat ctccaacggc gtgggcacac tgtcgggcat ggtgtgcccc 1380
   atcatcgtgg gggccatgac taagcacaag actcgggagg agtggcagta cgtgttccta 1440
   attgcctccc tggtgcacta tggaggtgtc atcttctacg gggtctttgc ttctggagag 1500
   aagcagccgt gggcagagcc tgaggagatg agcgaggaga agtgtggctt cgttggccat 1560
   gaccagctgg ctggcagtga cgacagcgaa atggaggatg aggctgagcc cccgggggca 1620
   ccccctgcac ccccgccctc ctatggggcc acacacagca catttcagcc ccccaggccc 1680
   ccaccccctg tccgggacta ctgaccatgt gcctcccact gaatggcagt ttccaggacc 1740
   tccattccac tcatctctgg cctgagtgac agtgtcaagg aaccctgctc ctctctgtcc 1800
   tgcctcaggc ctaagaagca ctctcccttg ttcccagtgc tgtcaaatcc tctttccttc 1860
   ccaattgcct ctcaggggta gtgaagctgc agactgacag tttcaaggat acccaaattc 1920
   ccctaaaggt tccctctcca cccgttctgc ctcagtggtt tcaaatctct cctttcaggg 1980
   ctttatttga atggacagtt cgacctctta ctctctcttg tggttttgag gcacccacac 2040
   cccccgcttt cctttatctc cagggactct caggctaacc tttgagatca ctcagctccc 2100
   atctcctttc agaaaaattc aaggtcctcc tctagaagtt tcaaatctct cccaactctg 2160
   ttctgcatct tccagattgg tttaaccaat tactcgtccc cgccattcca gggattgatt 2220
   ctcaccagcg tttctgatgg aaaatggcgg tttcaagtcc ccgattccgt gcccacttca 2280
   catctcccct accagcagat tctgcgaaag caccaaattt ctcaagaccc tcttctccct 2340
   agcttagcat aatgtctggg gaaaca 2366
<210> 2
   <211> 560
   <212> PRT
   <213> Homo Sapiens
<400> 2
   Met Glu Phe Arg Gln Glu Glu Phe Arg Lys Leu Ala Gly Arg Ala Leu
   1               5                   10                  15
   Gly Lys Leu His Arg Leu Leu Glu Lys Arg Gln Glu Gly Ala Glu Thr
               20                  25                  30
   Leu Glu Leu Ser Ala Asp Gly Arg Pro Val Thr Thr Gln Thr Arg Asp
           35                  40                  45
   Pro Pro Val Val Asp Cys Thr Cys Phe Gly Leu Pro Arg Arg Tyr Ile
       50                  55                  60
   Ile Ala Ile Met Ser Gly Leu Gly Phe Cys Ile Ser Phe Gly Ile Arg
   65                  70                  75                  80
   Cys Asn Leu Gly Val Ala Ile Val Ser Met Val Asn Asn Ser Thr Thr
                   85                  90                  95
   His Arg Gly Gly His Val Val Val Gln Lys Ala Gln Phe Ser Trp Asp
               100                 105                 110
   Pro Glu Thr Val Gly Leu Ile His Gly Ser Phe Phe Trp Gly Tyr Ile
           115                 120                 125
   Val Thr Gln Ile Pro Gly Gly Phe Ile Cys Gln Lys Phe Ala Ala Asn
   130 135 140
   Arg Val Phe Gly Phe Ala Ile Val Ala Thr Ser Thr Leu Asn Met Leu
   145 150 155 160
   Ile Pro Ser Ala Ala Arg Val His Tyr Gly Cys Val Ile Phe Val Arg
   165 170 175
   Ile Leu Gln Gly Leu Val Glu Gly Val Thr Tyr Pro Ala Cys His Gly
   180 185 190
   Ile Trp Ser Lys Trp Ala Pro Pro Leu Glu Arg Ser Arg Leu Ala Thr
   195 200 205
   Thr Ala Phe Cys Gly Ser Tyr Ala Gly Ala Val Val Ala Met Pro Leu
   210 215 220
   Ala Gly Val Leu Val Gln Tyr Ser Gly Trp Ser Ser Val Phe Tyr Val
   225 230 235 240
   Tyr Gly Ser Phe Gly Ile Phe Trp Tyr Leu Phe Trp Leu Leu Val Ser
   245 250 255
   Tyr Glu Ser Pro Ala Leu His Pro Ser Ile Ser Glu Glu Glu Arg Lys
   260 265 270
   Tyr Ile Glu Asp Ala Ile Gly Glu Ser Ala Lys Leu Met Asn Pro Leu
   275 280 285
   Thr Lys Phe Ser Thr Pro Trp Arg Arg Phe Phe Thr Ser Met Pro Val
   290 295 300
   Tyr Ala Ile Ile Val Ala Asn Phe Cys Arg Ser Trp Thr Phe Tyr Leu
   305 310 315 320
   Leu Leu Ile Ser Gln Pro Ala Tyr Phe Glu Glu Val Phe Gly Phe Glu
   325 330 335
   Ile Ser Lys Val Gly Leu Val Ser Ala Leu Pro His Leu Val Met Thr
   340 345 350
   Ile Ile Val Pro Ile Gly Gly Gln Ile Ala Asp Phe Leu Arg Ser Arg
   355 360 365
   Arg Ile Met Ser Thr Thr Asn Val Arg Lys Leu Met Asn Cys Gly Gly
   370 375 380
   Phe Gly Met Glu Ala Thr Leu Leu Leu Val Val Gly Tyr Ser His Ser
   385                 390                 395                 400
   Lys Gly Val Ala Ile Ser Phe Leu Val Leu Ala Val Gly Phe Ser Gly
                   405                 410                 415
   Phe Ala Ile Ser Gly Phe Asn Val Asn His Leu Asp Ile Ala Pro Arg
               420                 425                 430
   Tyr Ala Ser Ile Leu Met Gly Ile Ser Asn Gly Val Gly Thr Leu Ser
           435                 440                 445
   Gly Met Val Cys Pro Ile Ile Val Gly Ala Met Thr Lys His Lys Thr
       450                 455                 460
   Arg Glu Glu Trp Gln Tyr Val Phe Leu Ile Ala Ser Leu Val His Tyr
   465                 470                 475                 480
   Gly Gly Val Ile Phe Tyr Gly Val Phe Ala Ser Gly Glu Lys Gln Pro
                   485                 490                 495
   Trp Ala Glu Pro Glu Glu Met Ser Glu Glu Lys Cys Gly Phe Val Gly
               500                 505                 510
   His Asp Gln Leu Ala Gly Ser Asp Asp Ser Glu Met Glu Asp Glu Ala
           515                 520                 525
   Glu Pro Pro Gly Ala Pro Pro Ala Pro Pro Pro Ser Tyr Gly Ala Thr
       530                 535                 540
   His Ser Thr Phe Gln Pro Pro Arg Pro Pro Pro Pro Val Arg Asp Tyr
   545                 550                 555                 560
<210> 3
   <211> 2716
   <212> DNA
   <213> Homo Sapiens
<400> 3
   cgataagctt gatatcgaat tccggactct tgctcgggcg ccttaacccg gcgttcggtt 60
   catcccgcag cgccagttct gcttaccaaa agtggcccac taggcactcg cattccacgc 120
   ccggctccac gccagcgagc cgggcttctt acccatttaa agtttgagaa taggttgaga 180
   tcgtttcggc cccaagacct ctaatcattc gctttaccgg ataaaactgc gtggcggggg 240
   tgcgtcgggt ctgcgagagc gccagctatc ctgagggaaa cttcggaggg aaccagctac 300
   tagatggttc gattagtctt tcgcccctat acccaggtcg gacgaccgat ttgcacgtca 360
   ggaccgctac ggacctccac cagagtttcc tctggcttcg ccctgcccag gcgatcggcg 420
   ggggggaccc gcggggtgac cggcggcagg agccgccacc atggagttcc gccaggagga 480
   gtttcggaag ctagcgggtc gtgctctcgg gaagctgcac cgccttctgg agaagcggca 540
   ggaaggcgcg gagacgctgg agctgagtgc ggatgggcgc ccggtgacca cgcagacccg 600
   ggacccgccg gtggtggact gcacctgctt cggcctccct cgccgctaca ttatcgccat 660
   catgagtggt ctgggcttct gcatcagctt tggcatccgc tgcaacctgg gcgtggccat 720
   cgtctccatg gtcaataaca gcacgaccca ccgcgggggc cacgtggtgg tgcagaaagc 780
   ccagttcagc tgggatccag agactgtcgg cctcatacac ggctcctttt tctggggcta 840
   cattgtcact cagattccag gaggatttat ctgtcaaaaa tttgcagcca acagagtttt 900
   cggctttgct attgtggcaa catccactct aaacatgctg atcccctcag ctgcccgcgt 960
   ccactatggc tgtgtcatct tcgtgaggat cctgcagggg ttggtagagg gggtcacata 1020
   ccccgcctgc catgggatct ggagcaaatg ggccccaccc ttagaacgga gtcgcctggc 1080
   gacgacagcc ttttgtggtt cctatgctgg ggcggtggtc gcgatgcccc tcgccggggt 1140
   ccttgtgcag tactcaggat ggagctctgt tttctacgtc tacggcagct tcgggatctt 1200
   ctggtacctg ttctggctgc tcgtctccta cgagtccccc gcgctgcacc ccagcatctc 1260
   ggaggaggag cgcaagtaca tcgaggacgc catcggagag agcgcgaaac tcatgaaccc 1320
   cctcacgaag tttagcactc cctggcggcg cttcttcacg tctatgccag tctatgccat 1380
   catcgtggcc aacttctgcc gcagctggac gttctacctg ctgctcatct cccagcccga 1440
   ctacttcgaa gaagtgttcg gcttcgagat cagcaaggta ggcctggtgt ccgcgctgcc 1500
   ccacctggtc atgaccatca tcgtgcccat cggcggccag atcgcggact tcctgcggag 1560
   ccgccgcatc atgtccacca ccaacgtgcg caagttgatg aactgcggag gcttcggcat 1620
   ggaagccacg ctgctgttgg tggtcggcta ctcgcactcc aagggcgtgg ccatctcctt 1680
   cctggtccta gccgtgggct tcagcggctt cgccatctct gggttcaacg tgaaccacct 1740
   ggacatagcc ccgcgctacg ccagcatcct catgggcatc tccaacggcg tgggcacact 1800
   gtcgggcatg gtgtgcccca tcatcgtggg ggccatgact aagcacaaga ctcgggagga 1860
   gtggcagtac gtgttcctaa ttgcctccct ggtgcactat ggaggtgtca tcttctacgg 1920
   ggtctttgct tctggagaga agcagccgtg ggcagagcct gaggagatga gcgaggagaa 1980
   gtgtggcttc gttggccatg accagctggc tggcagtgac gacagcgaaa tggaggatga 2040
   ggctgagccc ccgggggcac cccctgcacc cccgccctcc tatggggcca cacacagcac 2100
   atttcagccc cccaggcccc caccccctgt ccgggactac tgaccatgtg cctcccactg 2160
   aatggcagtt tccaggacct ccattccact catctctggc ctgagtgaca gtgtcaagga 2220
   accctgctcc tctctgtcct gcctcaggcc taagaagcac tctcccttgt tcccagtgct 2280
   gtcaaatcct ctttccttcc caattgcctc tcaggggtag tgaagctgca gactgacagt 2340
   ttcaaggata cccaaattcc cctaaaggtt ccctctccac ccgttctgcc tcagtggttt 2400
   caaatctctc ctttcagggc tttatttgaa tggacagttc gacctcttac tctctcttgt 2460
   ggttttgagg cacccacacc ccccgctttc ctttatctcc agggactctc aggctaacct 2520
   ttgagatcac tcagctccca tctcctttca gaaaaattca aggtcctcct ctagaagttt 2580
   caaatctctc ccaactctgt tctgcatctt ccagattggt ttaaccaatt actcgtcccc 2640
   gccattccag ggattgattc tcaccagcgt ttctgatgga aaatggcggg aattcctgca 2700
   gcccggggga tccact                                                 2716
<210> 4
   <211> 560
   <212> PRT
   <213> Homo Sapiens
<400> 4
   Met Glu Phe Arg Gln Glu Glu Phe Arg Lys Leu Ala Gly Arg Ala Leu
   1               5                   10                  15
   Gly Lys Leu His Arg Leu Leu Glu Lys Arg Gln Glu Gly Ala Glu Thr
               20                  25                  30
   Leu Glu Leu Ser Ala Asp Gly Arg Pro Val Thr Thr Gln Thr Arg Asp
           35                  40                  45
   Pro Pro Val Val Asp Cys Thr Cys Phe Gly Leu Pro Arg Arg Tyr Ile
       50                  55                  60
   Ile Ala Ile Met Ser Gly Leu Gly Phe Cys Ile Ser Phe Gly Ile Arg
   65                  70                  75                  80
   Cys Asn Leu Gly Val Ala Ile Val Ser Met Val Asn Asn Ser Thr Thr
                   85                  90                  95
   His Arg Gly Gly His Val Val Val Gln Lys Ala Gln Phe Ser Trp Asp
               100                 105                 110
   Pro Glu Thr Val Gly Leu Ile His Gly Ser Phe Phe Trp Gly Tyr Ile
           115                 120                 125
   Val Thr Gln Ile Pro Gly Gly Phe Ile Cys Gln Lys Phe Ala Ala Asn
       130                 135                 140
   Arg Val Phe Gly Phe Ala Ile Val Ala Thr Ser Thr Leu Asn Met Leu
   145                 150                 155                 160
   Ile Pro Ser Ala Ala Arg Val His Tyr Gly Cys Val Ile Phe Val Arg
                   165                 170                 175
   Ile Leu Gln Gly Leu Val Glu Gly Val Thr Tyr Pro Ala Cys His Gly
               180                 185                 190
   Ile Trp Ser Lys Trp Ala Pro Pro Leu Glu Arg Ser Arg Leu Ala Thr
           195                 200                 205
   Thr Ala Phe Cys Gly Ser Tyr Ala Gly Ala Val Val Ala Met Pro Leu
       210                 215                 220
   Ala Gly Val Leu Val Gln Tyr Ser Gly Trp Ser Ser Val Phe Tyr Val
   225                 230                 235                 240
   Tyr Gly Ser Phe Gly Ile Phe Trp Tyr Leu Phe Trp Leu Leu Val Ser
   245 250 255
   Tyr Glu Ser Pro Ala Leu His Pro Ser Ile Ser Glu Glu Glu Arg Lys
   260 265 270
   Tyr Ile Glu Asp Ala Ile Gly Glu Ser Ala Lys Leu Met Asn Pro Leu
   275 280 285
   Thr Lys Phe Ser Thr Pro Trp Arg Arg Phe Phe Thr Ser Met Pro Val
   290 295 300
   Tyr Ala Ile Ile Val Ala Asn Phe Cys Arg Ser Trp Thr Phe Tyr Leu
   305 310 315 320
   Leu Leu Ile Ser Gln Pro Asp Tyr Phe Glu Glu Val Phe Gly Phe Glu
   325 330 335
   Ile Ser Lys Val Gly Leu Val Ser Ala Leu Pro His Leu Val Met Thr
   340 345 350
   Ile Ile Val Pro Ile Gly Gly Gln Ile Ala Asp Phe Leu Arg Ser Arg
   355 360 365
   Arg Ile Met Ser Thr Thr Asn Val Arg Lys Leu Met Asn Cys Gly Gly
   370 375 380
   Phe Gly Met Glu Ala Thr Leu Leu Leu Val Val Gly Tyr Ser His Ser
   385 390 395 400
   Lys Gly Val Ala Ile Ser Phe Leu Val Leu Ala Val Gly Phe Ser Gly
   405 410 415
   Phe Ala Ile Ser Gly Phe Asn Val Asn His Leu Asp Ile Ala Pro Arg
   420 425 430
   Tyr Ala Ser Ile Leu Met Gly Ile Ser Asn Gly Val Gly Thr Leu Ser
   435 440 445
   Gly Met Val Cys Pro Ile Ile Val Gly Ala Met Thr Lys His Lys Thr
   450 455 460
   Arg Glu Glu Trp Gln Tyr Val Phe Leu Ile Ala Ser Leu Val His Tyr
   465 470 475 480
   Gly Gly Val Ile Phe Tyr Gly Val Phe Ala Ser Gly Glu Lys Gln Pro
   485 490 495
   Trp Ala Glu Pro Glu Glu Met Ser Glu Glu Lys Cys Gly Phe Val Gly
   500 505 510
   His Asp Gln Leu Ala Gly Ser Asp Asp Ser Glu Met Glu Asp Glu Ala
   515 520 525
   Glu Pro Pro Gly Ala Pro Pro Ala Pro Pro Pro Ser Tyr Gly Ala Thr
   530 535 540
   His Ser Thr Phe Gln Pro Pro Arg Pro Pro Pro Pro Val Arg Asp Tyr
   545 550 555 560
<210> 5
   <211> 2024
   <212> DNA
   <213> Rattus Norvegicus
<400> 5
   gaattcggca cgagcggagc tgcggggccg ggccgggccg gggcggaccc cgggatcccg 60
   gacgcggccg cccgggcccg cgggcggggg gattggcagg ggacccgcgt gggcacagcc 120
   accatggagt tccggcagga ggagtttcgg aagctggcgg ggcgcgccct ggggaggctg 180
   caccggttac tggagaagcg gcaggaaggc gcggagacat tggagctgag cgccgacggg 240
   cgcccagtga ccacacacac gcgggacccg ccggtggtgg actgcacttg ctttggcctc 300
   cctcgccgct acatcatcgc gatcatgagc ggtctgggtt tctgcatcag ctttggcatc 360
   cgctgcaacc tgggcgtggc catcgtatcc atggtcaaca acagtacaac ccaccgtggg 420
   ggccacgtgg tggtgcagaa agcccagttc aactgggatc cagagactgt cggcctcata 480
   catggctcct ttttctgggg gtacattgtc actcagattc ctggaggatt tatctgccaa 540
   aaattcgcag ccaacagggt ctttggcttt gccattgtgg ctacctccac cctaaatatg 600
   ttgatccctt cagcagcccg tgttcactat ggctgtgtca tcttcgtgag gatccttcag 660
   ggattggtgg agggggtcac ataccctgct tgccatggca tctggagcaa atgggcccct 720
   cccttagaac ggagtcggct ggcgacgaca gccttttgcg gttcctatgc cggggcagtg 780
   gttgccatgc ctctggctgg ggtcctggta cagtattcag gatggagttc tgtcttctat 840
   gtctatggca gcttcgggat cttttggtac ctgttctggt tgcttgtctc ctacgagtca 900
   cctgcactac accccagcat ctccgaggag gagcgcaaat acattgagga tgccatcgga 960
   gaaagcgcca agctcatgaa ccctgttacg aagtttaaca caccctggag gcgcttcttt 1020
   acctccatgc cggtctatgc catcattgtc gccaactttt gccgcagctg gactttctac 1080
   ctgctcctca tctcccagcc cgcctacttt gaagaagtgt tcggctttga gatcagcaag 1140
   gtgggactgg tgtcggcact gcctcacctt gtcatgacta tcatcgtacc catcggaggc 1200
   cagatcgccg acttcctgcg cagtcgtcat ataatgtcca cgaccaatgt gcgaaagctg 1260
   atgaactgcg ggggtttcgg gatggaagct acgctgctgc tggtggtcgg atactcacac 1320
   tccaagggcg tggccatctc cttcctggtc ctggctgtgg gcttcagtgg ctttgctatc 1380
   tctgggttta acgtgaacca cttggacatc gcccctcgat atgccagcat cttgatgggc 1440
   atttccaatg gcgtgggcac actgtctggg atggtgtgcc ccatcatcgt gggtgcaatg 1500
   accaagcaca agacgcggga ggagtggcag tacgtgttcc tcatagcctc cctggtgcac 1560
   tatggaggtg tcatcttcta tggggtcttt gcttcgggag agaaacagcc gtgggcagag 1620
   ccggaggaga tgagcgagga gaagtgtggc tttgttggcc acgaccagct ggctggcagt 1680
   gacgaaagtg aaatggaaga cgaggttgag cccccggggg caccccccgc acctccgcct 1740
   tcctacgggg ccacacacag cacagttcag cctccaaggc ccccaccccc tgtccgggac 1800
   tactgaccac gtgcctccca ctggtgggca gtttccagga cctccactcg atacacctct 1860
   agcctaaacg gcagtgtcga ggaaccccac tcctctcctg cctcaggctt aagatgcaag 1920
   tcttcccttg tgcccagtgc tgtccgacca gccctctctc cttctcaact gcctcttgca 1980
   ggggtgaagc tgcacactag cagtttcaag ctcgtgccga attc                  2024
<210> 6
   <211> 560
   <212> PRT
   <213> Rattus Norvegicus
<400> 6
   Met Glu Phe Arg Gln Glu Glu Phe Arg Lys Leu Ala Gly Arg Ala Leu
   1 5 10 15
   Gly Arg Leu His Arg Leu Leu Glu Lys Arg Gln Glu Gly Ala Glu Thr
   20 25 30
   Leu Glu Leu Ser Ala Asp Gly Arg Pro Val Thr Thr His Thr Arg Asp
   35 40 45
   Pro Pro Val Val Asp Cys Thr Cys Phe Gly Leu Pro Arg Arg Tyr Ile
   50 55 60
   Ile Ala Ile Met Ser Gly Leu Gly Phe Cys Ile Ser Phe Gly Ile Arg
   65 70 75 80
   Cys Asn Leu Gly Val Ala Ile Val Ser Met Val Asn Asn Ser Thr Thr
   85 90 95
   His Arg Gly Gly His Val Val Val Gln Lys Ala Gln Phe Asn Trp Asp
   100 105 110
   Pro Glu Thr Val Gly Leu Ile His Gly Ser Phe Phe Trp Gly Tyr Ile
   115 120 125
   Val Thr Gln Ile Pro Gly Gly Phe Ile Cys Gln Lys Phe Ala Ala Asn
   130 135 140
   Arg Val Phe Gly Phe Ala Ile Val Ala Thr Ser Thr Leu Asn Met Leu
   145 150 155 160
   Ile Pro Ser Ala Ala Arg Val His Tyr Gly Cys Val Ile Phe Val Arg
   165 170 175
   Ile Leu Gln Gly Leu Val Glu Gly Val Thr Tyr Pro Ala Cys His Gly
   180 185 190
   Ile Trp Ser Lys Trp Ala Pro Pro Leu Glu Arg Ser Arg Leu Ala Thr
   195 200 205
   Thr Ala Phe Cys Gly Ser Tyr Ala Gly Ala Val Val Ala Met Pro Leu
   210 215 220
   Ala Gly Val Leu Val Gln Tyr Ser Gly Trp Ser Ser Val Phe Tyr Val
   225 230 235 240
   Tyr Gly Ser Phe Gly Ile Phe Trp Tyr Leu Phe Trp Leu Leu Val Ser
   245 250 255
   Tyr Glu Ser Pro Ala Leu His Pro Ser Ile Ser Glu Glu Glu Arg Lys
   260 265 270
   Tyr Ile Glu Asp Ala Ile Gly Glu Ser Ala Lys Leu Met Asn Pro Val
   275 280 285
   Thr Lys Phe Asn Thr Pro Trp Arg Arg Phe Phe Thr Ser Met Pro Val
   290 295 300
   Tyr Ala Ile Ile Val Ala Asn Phe Cys Arg Ser Trp Thr Phe Tyr Leu
   305 310 315 320
   Leu Leu Ile Ser Gln Pro Ala Tyr Phe Glu Glu Val Phe Gly Phe Glu
   325 330 335
   Ile Ser Lys Val Gly Leu Val Ser Ala Leu Pro His Leu Val Met Thr
   340 345 350
   Ile Ile Val Pro Ile Gly Gly Gln Ile Ala Asp Phe Leu Arg Ser Arg
   355 360 365
   His Ile Met Ser Thr Thr Asn Val Arg Lys Leu Met Asn Cys Gly Gly
   370 375 380
   Phe Gly Met Glu Ala Thr Leu Leu Leu Val Val Gly Tyr Ser His Ser
   385 390 395 400
   Lys Gly Val Ala Ile Ser Phe Leu Val Leu Ala Val Gly Phe Ser Gly
   405 410 415
   Phe Ala Ile Ser Gly Phe Asn Val Asn His Leu Asp Ile Ala Pro Arg
   420 425 430
   Tyr Ala Ser Ile Leu Met Gly Ile Ser Asn Gly Val Gly Thr Leu Ser
   435 440 445
   Gly Met Val Cys Pro Ile Ile Val Gly Ala Met Thr Lys His Lys Thr
   450 455 460
   Arg Glu Glu Trp Gln Tyr Val Phe Leu Ile Ala Ser Leu Val His Tyr
   465 470 475 480
   Gly Gly Val Ile Phe Tyr Gly Val Phe Ala Ser Gly Glu Lys Gln Pro
   485 490 495
   Trp Ala Glu Pro Glu Glu Met Ser Glu Glu Lys Cys Gly Phe Val Gly
   500 505 510
   His Asp Gln Leu Ala Gly Ser Asp Glu Ser Glu Met Glu Asp Glu Val
   515 520 525
   Glu Pro Pro Gly Ala Pro Pro Ala Pro Pro Pro Ser Tyr Gly Ala Thr
   530 535 540
   His Ser Thr Val Gln Pro Pro Arg Pro Pro Pro Pro Val Arg Asp Tyr
   545 550 555 560
<210> 7
   <211> 3946
   <212> DNA
   <213> Homo Sapiens
<400> 7
   cgtttaaaag ccatcagatt tgagagcaat aagtcttcaa aaccgggaat ttacattgtt 60
   tttcagctga ccgacttcca ggaaaaggac tcaaccgcat ctacccaaat accgtggcac 120
   tgcttgcgct ctttgccacc ggatactccc cttccaatga gactttctga ttgtgtctac 180
   caactctcct attaggaaac ccgtgggttg catgcagcta ttctgttgta ttctcattct 240
   cactctccct cccttctctc actctcactc ttgctggagg cgagccacta ccattctgct 300
   gagaaggaaa agcccgcaac tactttaaga gattaagaca atatgcgcaa tcctcgcctt 360
   tcctagcaat cactatttaa atctggcaag aactgacaac agtctttgca agaatggaat 420
   ccgtaaaaca aaggattttg gccccaggaa aagaggggct aaagaatttt gctggaaaat 480
   cactcggcca gatctacagg gtgctggaga agaagcaaga caccggggag acaatcgagc 540
   tgacggagga tgggaagccc ctagaggtgc ccgagaggaa ggcgccgctg tgcgactgca 600
   cgtgcttcgg cctgccccgc cgctacatta tcgccatcat gagcggcctg ggcttctgca 660
   tctccttcgg tatccgctgc aacctgggcg tggccattgt ggacatggtc aacaacagca 720
   ccatccaccg cgggggcaag gtcatcaagg agaaagccaa attcaactgg gacccggaaa 780
   ccgtggggat gatccacggt tccttctttt ggggctacat catcactcag attccgggag 840
   gctacatcgc gtctcggctg gcagccaaca gggttttcgg agctgccata cttcttacct 900
   ctaccctaaa tatgctaatt ccatcagcag ccagagtgca ttatggatgt gtcatctttg 960
   tcagaatact gcagggactt gttgagggtg tgacctaccc agcatgtcat gggatatgga 1020
   gcaaatgggc cccacctcta gagaggagta gactggcaac cacctccttt tgtggttcct 1080
   atgccggagc tgtgattgca atgcctttag ctggcattct tgtgcagtac actggctggt 1140
   cttcagtgtt ttatgtctac ggaagctttg gaatggtctg gtacatgttt tggcttttgg 1200
   tgtcttatga aagtcctgca aagcatccta ctattacaga tgaagaacgt aggtacatag 1260
   aagaaagcat tggagagagt gcaaatcttt taggtgcaat ggaaaaattc aagactccat 1320
   ggaggaagtt ttttacatcc atgccagtct atgcaataat tgttgcaaac ttctgcagaa 1380
   gctggacttt ttatttattg cttattagtc agccagcata ttttgaggaa gtctttggat 1440
   ttgaaattag caaggttggt atgctatctg ctgtgccaca cttagtaatg acaattattg 1500
   tgcctattgg gggacaaatt gcagattttc taagaagcaa gcagattctt tcaactacga 1560
   cagtgagaaa gatcatgaat tgtggtggtt ttggcatgga agccacactg ctcctggtcg 1620
   ttggctattc tcatactaga ggggtagcaa tctcattctt ggtacttgca gtgggattca 1680
   gtggatttgc tatatctggt ttcaatgtta accacttgga tatcgctcca agatatgcca 1740
   gtatcttaat gggcatttcg aatggtgttg gcacattgtc aggaatggtt tgtcctatca 1800
   ttgttggtgc aatgacaaag aataagtcac gtgaagagtg gcagtatgtc ttcctgatcg 1860
   ctgccctagt ccactatggt ggagttatat tttatgcaat atttgcctca ggagagaaac 1920
   aaccctgggc agacccggag gaaacaagtg aagaaaaatg tggatttatt catgaagatg 1980
   aactcgatga agaaacaggg gacattactc aaaattatat aaattatggt accaccaagt 2040
   cttatggtgc cacaacacag gccaatggag gttggcctag tggttgggaa aagaaagagg 2100
   aatttgtaca aggagaagta caagactcac atagctataa ggaccgagtt gattattcat 2160
   aacaaaacta attactggat ttatttttag tgtttgtgat taaattcatt gtgattgcac 2220
   aaaaatttta aaaacacgtg atgtaaactt gcaagcatat caaccaggca agtcttgctg 2280
   taaaaatgaa aacaaaacaa acccatgagg ttaccatcaa gtgcaatctg taaaattgtg 2340
   aagttccatc atttccattc aagtcatcca ttcttgcatt tgtgacttaa aggttgactg 2400
   gtcaaaattg tagaaacaag tagttaccca ttggattcat atgagctaaa actcatcact 2460
   atttactaaa gcacaacatc tcatcctaca aaagttaaga agccaaagct acttgatcat 2520
   gcaaaatgca cttatatatt tgttacactg tattgcaaga tagcacacag aagttggctg 2580
   cgtcaagtag aggcgacatt tattaagtga aaatcatgga gttgggatat ctctcaatta 2640
   aagaaataca ttgtgaacta tcagctacaa agttgtactg aataactatt agaattgcat 2700
   aatgtgagat attttgttag tcctcaaaag gaatatcttg cagtgttttc tatgaaatgc 2760
   ttgggcacaa acacttattt ctgtgaaaga gaacatgtaa gttgaggggt atgcttcatg 2820
   ttcttccatc catttaccta atagtatgaa acagttcaca tttcaataaa atcaaacttt 2880
   tcatgtagcg tatcacataa cttttttgca aaaaatataa aaagaaataa acttcaatgt 2940
   attttttatt acaactttgt actggttgta acttgcatta gaaaaaaaaa agagatatat 3000
   aaaccacaaa gaatctaata agaaatttat tatggagata tagcccttaa aatgcaatat 3060
   taagaacaaa gaaatagaaa atggtttaga tatctttctt ccttcataat taaatactat 3120
   atgaaacttg tgccacagag ctatatgtaa tatgaaaaga ttaacttcat agagatattg 3180
   taagtaggta attttattat ttaaagtcct attaagaaat atttgtctta aatatatagg 3240
   acaatacatt atattaaaat ggtctctctc tatatatatc tgtatatctt atacatgtcc 3300
   atacacagaa acataataaa caatcttcac acgaaaccaa aaatagcata cacctaatgt 3360
   tgggttaggg aattgcaatt tctactttca tagagtcata gaattttagg tggggaagag 3420
   gcattttgct tgtcatttct taatataact caacaagaat tgcaacattt gtgtaccaag 3480
   caataagtgc aatgcataaa atttcctgtc tgtatattac cttcattttg cttgtagtag 3540
   ctgtttgggt ggttggaata attttatttt tcttttaaaa aagctaacat cagacccctt 3600
   tataatgtcc taaaattatg ataatacatt tcccaattca actcaaaata ttattggtgt 3660
   attttgtcta ttctggatat ttgatctgtt taatgtactg tgctagtgac tggaggccct 3720
   gctactgcaa atataaaacc taaagtttgt ttaaaaaaat gcaaatcatt ctttacctta 3780
   agaaaaaaaa aatacccttt gctttgtgcc tcaaagtgat gtaatgtgat cacagctttt 3840
   gttgtgttga atgaaaatat gtggactgtc attttgttgc agcaaaaaag tgttaataaa 3900
   atgctctatt tatccttttt taaaaaaaaa aaaaaaaaaa aaaaaa                3946
<210> 8
   <211> 582
   <212> PRT
   <213> Homo Sapiens
<400> 8
   Met Glu Ser Val Lys Gln Arg Ile Leu Ala Pro Gly Lys Glu Gly Leu
   1 5 10 15
   Lys Asn Phe Ala Gly Lys Ser Leu Gly Gln Ile Tyr Arg Val Leu Glu
   20 25 30
   Lys Lys Gln Asp Thr Gly Glu Thr Ile Glu Leu Thr Glu Asp Gly Lys
   35 40 45
   Pro Leu Glu Val Pro Glu Arg Lys Ala Pro Leu Cys Asp Cys Thr Cys
   50 55 60
   Phe Gly Leu Pro Arg Arg Tyr Ile Ile Ala Ile Met Ser Gly Leu Gly
   65 70 75 80
   Phe Cys Ile Ser Phe Gly Ile Arg Cys Asn Leu Gly Val Ala Ile Val
   85 90 95
   Asp Met Val Asn Asn Ser Thr Ile His Arg Gly Gly Lys Val Ile Lys
   100 105 110
   Glu Lys Ala Lys Phe Asn Trp Asp Pro Glu Thr Val Gly Met Ile His
   115 120 125
   Gly Ser Phe Phe Trp Gly Tyr Ile Ile Thr Gln Ile Pro Gly Gly Tyr
   130 135 140
   Ile Ala Ser Arg Leu Ala Ala Asn Arg Val Phe Gly Ala Ala Ile Leu
   145 150 155 160
   Leu Thr Ser Thr Leu Asn Met Leu Ile Pro Ser Ala Ala Arg Val His
   165 170 175
   Tyr Gly Cys Val Ile Phe Val Arg Ile Leu Gln Gly Leu Val Glu Gly
   180 185 190
   Val Thr Tyr Pro Ala Cys His Gly Ile Trp Ser Lys Trp Ala Pro Pro
   195 200 205
   Leu Glu Arg Ser Arg Leu Ala Thr Thr Ser Phe Cys Gly Ser Tyr Ala
   210 215 220
   Gly Ala Val Ile Ala Met Pro Leu Ala Gly Ile Leu Val Gln Tyr Thr
   225 230 235 240
   Gly Trp Ser Ser Val Phe Tyr Val Tyr Gly Ser Phe Gly Met Val Trp
   245 250 255
   Tyr Met Phe Trp Leu Leu Val Ser Tyr Glu Ser Pro Ala Lys His Pro
   260 265 270
   Thr Ile Thr Asp Glu Glu Arg Arg Tyr Ile Glu Glu Ser Ile Gly Glu
   275 280 285
   Ser Ala Asn Leu Leu Gly Ala Met Glu Lys Phe Lys Thr Pro Trp Arg
   290 295 300
   Lys Phe Phe Thr Ser Met Pro Val Tyr Ala Ile Ile Val Ala Asn Phe
   305 310 315 320
   Cys Arg Ser Trp Thr Phe Tyr Leu Leu Leu Ile Ser Gln Pro Ala Tyr
   325 330 335
   Phe Glu Glu Val Phe Gly Phe Glu Ile Ser Lys Val Gly Met Leu Ser
   340 345 350
   Ala Val Pro His Leu Val Met Thr Ile Ile Val Pro Ile Gly Gly Gln
   355 360 365
   Ile Ala Asp Phe Leu Arg Ser Lys Gln Ile Leu Ser Thr Thr Thr Val
   370 375 380
   Arg Lys Ile Met Asn Cys Gly Gly Phe Gly Met Glu Ala Thr Leu Leu
   385 390 395 400
   Leu Val Val Gly Tyr Ser His Thr Arg Gly Val Ala Ile Ser Phe Leu
   405 410 415
   Val Leu Ala Val Gly Phe Ser Gly Phe Ala Ile Ser Gly Phe Asn Val
   420 425 430
   Asn His Leu Asp Ile Ala Pro Arg Tyr Ala Ser Ile Leu Met Gly Ile
   435 440 445
   Ser Asn Gly Val Gly Thr Leu Ser Gly Met Val Cys Pro Ile Ile Val
   450 455 460
   Gly Ala Met Thr Lys Asn Lys Ser Arg Glu Glu Trp Gln Tyr Val Phe
   465 470 475 480
   Leu Ile Ala Ala Leu Val His Tyr Gly Gly Val Ile Phe Tyr Ala Ile
   485 490 495
   Phe Ala Ser Gly Glu Lys Gln Pro Trp Ala Asp Pro Glu Glu Thr Ser
   500 505 510
   Glu Glu Lys Cys Gly Phe Ile His Glu Asp Glu Leu Asp Glu Glu Thr
   515 520 525
   Gly Asp Ile Thr Gln Asn Tyr Ile Asn Tyr Gly Thr Thr Lys Ser Tyr
   530 535 540
   Gly Ala Thr Thr Gln Ala Asn Gly Gly Trp Pro Ser Gly Trp Glu Lys
   545 550 555 560
   Lys Glu Glu Phe Val Gln Gly Glu Val Gln Asp Ser His Ser Tyr Lys
   565 570 575
   Asp Arg Val Asp Tyr Ser
   580
<210> 9
   <211> 3982
   <212> DNA
   <213> Rattus Norvegicus
<400> 9
   agacagtaag gttcttttgc ttttttccct tacacaagga ttcgatgacg tttttggtca 60
   atctgattaa aagacagcgg atttggttgc gttaagactt caaaaccggg aatttacgtt 120
   gtttttcggt gaggtgactt ccagaacggg gactcatcag cacccgccca aataccacgg 180
   cactgcgcgc gccctcggcc accggatcct ccccttccaa tgagactttg tgactgtgtg 240
   taccaattct cctattagga aacccgtggg ctgaatgcag ctattccgtt gtactctctt 300
   tctcgctctc cctcccctct ccaactcaca gccttgctga aaagctcatc tctgctgaga 360
   agaaaacgtt ctaccttaac ctattaagac tatgcgcaga actcgccttt catagccatc 420
   acaatttaaa tctggtaagg ctggacacga gtctttacaa gaatggagtc ggtaaaacaa 480
   aggattttgg ccccggggaa agaggggata aagaattttg ctggaaaatc cctcggacag 540
   atctacaggg tgctggagaa gaagcaggat aaccgagaga ccatcgagct gacagaggac 600
   ggcaagcccc tggaggtgcc tgagaagaag gctccgctat gcgactgtac gtgcttcggc 660
   ctgccgcgcc gctacatcat agccatcatg agcggcctcg gcttctgcat ctcctttggt 720
   atccgctgta acctgggtgt ggccattgtg gacatggtca acaacagcac catccaccgg 780
   ggtggcaaag ttatcaagga gaaagccaag tttaactggg accccgagac tgtggggatg 840
   attcacgggt cgttcttctg gggctatatc atcacgcaga ttccgggcgg atacatcgca 900
   tcgcgactgg ctgctaaccg ggtctttggg gctgccatac tgcttacctc taccctcaat 960
   atgctgatcc catctgcagc cagagtgcat tatggatgcg tcatctttgt tagaatattg 1020
   caaggacttg tggagggcgt cacctaccca gcctgtcacg ggatatggag caagtgggcc 1080
   cctcctttgg agaggagtag gttggctacc acctccttct gtggttccta tgctggagca 1140
   gtcattgcaa tgcccctagc tggtatcctg gtgcagtaca ctggatggtc ttcagtattt 1200
   tacgtatatg gaagctttgg tatggtctgg tatatgttct ggcttctggt gtcttacgag 1260
   agccccgcaa agcatccaac cataacagac gaagaacgta ggtacataga agagagcatc 1320
   ggggagagcg caaatctgtt aggagcaatg gagaaattca agaccccatg gaggaagttt 1380
   ttcacatcca tgcccgtcta tgcgataatt gttgcaaact tctgcaggag ttggactttt 1440
   tatttactgc tcatcagtca accagcttat ttcgaggagg tttttggatt tgaaatcagc 1500
   aaggttggca tgttgtctgc ggtcccacac ctggtcatga caatcattgt gcctatcggg 1560
   gggcaaattg cagactttct aaggagcaag caaattcttt caacaactac agtgcgaaag 1620
   atcatgaact gcgggggttt tggcatggaa gccacactgc ttctggttgt tggctactct 1680
   catactagag ggtggccatc ctccttcttg gtgcttgcag tgggattcag tggatttgct 1740
   atctctggtt tcaatgtgaa ccacttggat attgccccga gatatgccag tatcttaatg 1800
   ggcatttcaa atggtgttgg cacgctgtcg ggaatggtct gcccgatcat tgttggtgca 1860
   atgacgaaga acaagtcccg tgaagaatgg cagtatgtct tcctcatcgc tgcactggtc 1920
   cactatggtg gagtcatatt ttatgcacta tttgcctcag gagagaagca accttgggca 1980
   gaccctgagg aaacaagcga agaaaagtgt ggcttcattc atgaagatga actggatgaa 2040
   gaaacggggg acatcactca gaattacata aattacggta ccaccaaatc ctacggcgcc 2100
   acctcacagg agaacggagg ctggcctaac ggctgggaga aaaaggaaga atttgtgcaa 2160
   gaaagtgcgc aagacgcgta ctcctataag gaccgagatg attattcata acgaagctag 2220
   ttgctggatt cctttgtagt gtttgtgatt aaattaattg tgattgcaca aaatcatttt 2280
   aagaaatgtg gtgtaaacat gtaaacacat caaccaagca agtcttgctg ttcaaaaaat 2340
   aataataata tgaattcaaa acagaccgtg agagtcccat caagtgcaat ctgtggcggc 2400
   agtcacgtga cgccatttcc attcaggcca ttcgtccttt tcgtttgtga tttaaaggtt 2460
   tcctgtagaa ataagtaggt attcgttgga tccatcacca cgttagagag tacaactaca 2520
   acagttggca catgtcatcc tacggaagtt aggaagccaa agctactgga ttatgtgaac 2580
   tgcatttatt tatttattac actggactgc aaaatatccc agggaaatcc tgtctagaga 2640
   catagtagaa ctggaaagat ggctagattg ggtactgacg ataatcattg tgtgtatatc 2700
   atggagtggc tatatctttt aattggagaa ctatattgta tagctagcaa aattgtactg 2760
   aattattact aggagtgcac agtgtgtgat attttgtgat cttccaaaag cttatcttgc 2820
   agtgttttgt gaaacgcttg ggcacaaaca cttattttta tgaacaagag cttgtaaagg 2880
   gaggagtatg ctccatgctc tcccattcac tacctgacag tatcaaacct tcacatttca 2940
   atgaaatcca acgtccatgt aacatatcac atgacttttt ttgcaaaaaa gaatataaga 3000
   agaaatagac ttcaatgtat tttttattac aactttgtac tggttgtaac ttgcattagg 3060
   aaaaatgatt aatatatgta taatcgtaaa gaatctaata aaaatttact atgaagatat 3120
   agcccttaaa atgcaatatt aacaacaaaa atatatagaa aatttagata atcttccttg 3180
   ataactagag actatatgga actcacacca caaagctata tataatatga aaagataaac 3240
   aatagagatt gtatatgtag acgattttat gacctaatgt cccatttaag aggtatttgt 3300
   cttgagtata tagtacaaag tatattaaaa ttatatctac atccctgtat atcttataca 3360
   tatccactca cacaaacata acaaatactt ttcacacaga accaaaaaca agcatacacc 3420
   taatgttggg tttggggatt gcaatttcta ctttcataga gtcatagaat tttagatggg 3480
   aaaaaaaaag gcattttgct cgtcatttct taatataatt aattcaacag gaactgcaac 3540
   atttgtgtac caagcaataa gtgcgaagca taaacctgct gtgtgtaaac tatccccata 3600
   ctgcttgtgg tagcactgat ttctttcttt taaagaactt aacatcggag ctctttacaa 3660
   tgttttgcgc tgataagaat gcacatccca atttaacgca aagtgtcacc tggtgtgttt 3720
   acctgtctgt tttgggtatt tggtctgttt ggtgtcctgt gctcttgact ggaggccctg 3780
   ctactgcgaa tataaaacgt gaagtttgtt tctaaatgca aaccactcct gaccttaaga 3840
   aactaaagtc cctctctgct ttgtgtctcc aagtactatc atgtgaccat aacccttgct 3900
   gtgctgagta aaaagatgtg aactgtcatt ttgttgctgc gaagcaagtg ttaataaaat 3960
   gttctattta aaaaaaaaaa aa                                          3982
<210> 10
   <211> 582
   <212> PRT
   <213> Rattus Norvegicus
<400> 10
   Met Glu Ser Val Lys Gln Arg Ile Leu Ala Pro Gly Lys Glu Gly Ile
   1               5                   10                  15
   Lys Asn Phe Ala Gly Lys Ser Leu Gly Gln Ile Tyr Arg Val Leu Glu
               20                  25                  30
   Lys Lys Gln Asp Asn Arg Glu Thr Ile Glu Leu Thr Glu Asp Gly Lys
           35                  40                  45
   Pro Leu Glu Val Pro Glu Lys Lys Ala Pro Leu Cys Asp Cys Thr Cys
       50                  55                  60
   Phe Gly Leu Pro Arg Arg Tyr Ile Ile Ala Ile Met Ser Gly Leu Gly
   65                  70                  75                  80
   Phe Cys Ile Ser Phe Gly Ile Arg Cys Asn Leu Gly Val Ala Ile Val
                   85                  90                  95
   Asp Met Val Asn Asn Ser Thr Ile His Arg Gly Gly Lys Val Ile Lys
               100                 105                 110
   Glu Lys Ala Lys Phe Asn Trp Asp Pro Glu Thr Val Gly Met Ile His
           115                 120                 125
   Gly Ser Phe Phe Trp Gly Tyr Ile Ile Thr Gln Ile Pro Gly Gly Tyr
       130                 135                 140
   Ile Ala Ser Arg Leu Ala Ala Asn Arg Val Phe Gly Ala Ala Ile Leu
   145                 150                 155                 160
   Leu Thr Ser Thr Leu Asn Met Leu Ile Pro Ser Ala Ala Arg Val His
   165 170 175
   Tyr Gly Cys Val Ile Phe Val Arg Ile Leu Gln Gly Leu Val Glu Gly
   180 185 190
   Val Thr Tyr Pro Ala Cys His Gly Ile Trp Ser Lys Trp Ala Pro Pro
   195 200 205
   Leu Glu Arg Ser Arg Leu Ala Thr Thr Ser Phe Cys Gly Ser Tyr Ala
   210 215 220
   Gly Ala Val Ile Ala Met Pro Leu Ala Gly Ile Leu Val Gln Tyr Thr
   225 230 235 240
   Gly Trp Ser Ser Val Phe Tyr Val Tyr Gly Ser Phe Gly Met Val Trp
   245 250 255
   Tyr Met Phe Trp Leu Leu Val Ser Tyr Glu Ser Pro Ala Lys His Pro
   260 265 270
   Thr Ile Thr Asp Glu Glu Arg Arg Tyr Ile Glu Glu Ser Ile Gly Glu
   275 280 285
   Ser Ala Asn Leu Leu Gly Ala Met Glu Lys Phe Lys Thr Pro Trp Arg
   290 295 300
   Lys Phe Phe Thr Ser Met Pro Val Tyr Ala Ile Ile Val Ala Asn Phe
   305 310 315 320
   Cys Arg Ser Trp Thr Phe Tyr Leu Leu Leu Ile Ser Gln Pro Ala Tyr
   325 330 335
   Phe Glu Glu Val Phe Gly Phe Glu Ile Ser Lys Val Gly Met Leu Ser
   340 345 350
   Ala Val Pro His Leu Val Met Thr Ile Ile Val Pro Ile Gly Gly Gln
   355 360 365
   Ile Ala Asp Phe Leu Arg Ser Lys Gln Ile Leu Ser Thr Thr Thr Val
   370 375 380
   Arg Lys Ile Met Asn Cys Gly Gly Phe Gly Met Glu Ala Thr Leu Leu
   385 390 395 400
   Leu Val Val Gly Tyr Ser His Thr Arg Gly Val Ala Ile Ser Phe Leu
   405 410 415
   Val Leu Ala Val Gly Phe Ser Gly Phe Ala Ile Ser Gly Phe Asn Val
   420 425 430
   Asn His Leu Asp Ile Ala Pro Arg Tyr Ala Ser Ile Leu Met Gly Ile
   435 440 445
   Ser Asn Gly Val Gly Thr Leu Ser Gly Met Val Cys Pro Ile Ile Val
   450 455 460
   Gly Ala Met Thr Lys Asn Lys Ser Arg Glu Glu Trp Gln Tyr Val Phe
   465 470 475 480
   Leu Ile Ala Ala Leu Val His Tyr Gly Gly Val Ile Phe Tyr Ala Leu
   485 490 495
   Phe Ala Ser Gly Glu Lys Gln Pro Trp Ala Asp Pro Glu Glu Thr Ser
   500 505 510
   Glu Glu Lys Cys Gly Phe Ile His Glu Asp Glu Leu Asp Glu Glu Thr
   515 520 525
   Gly Asp Ile Thr Gln Asn Tyr Ile Asn Tyr Gly Thr Thr Lys Ser Tyr
   530 535 540
   Gly Ala Thr Ser Gln Glu Asn Gly Gly Trp Pro Asn Gly Trp Glu Lys
   545 550 555 560
   Lys Glu Glu Phe Val Gln Glu Ser Ala Gln Asp Ala Tyr Ser Tyr Lys
   565 570 575
   Asp Arg Asp Asp Tyr Ser
   580
<210> 11
   <211> 2836
   <212> DNA
   <213> Mus musculus
<400> 11
   cggccgcccg ggcccgcggg cggggggatt ggcaggggac ccgcgtgggc acagccacca 60
   tggagttccg gcaggaggag tttcggaagc tggcggggcg cgccctgggg aggctgcacc 120
   ggttactgga gaagcggcag gaaggcgcgg agacactgga gctgagtgcc gacgggcggc 180
   cagtgaccac gcacactcgg gacccgcctg tggtggactg cacctgcttt ggcctccctc 240
   gtcgctacat catcgccatc atgagcggtc tgggtttctg tatcagcttt ggcatccgct 300
   gcaacctggg cgtggccatc gtgtccatgg tcaacaacag cacaacccac cgtgggggcc 360
   acgtggtggt gcagaaagcc cagttcaact gggatccaga gactgtcggc ctcatacatg 420
   gctccttttt ctggggctac attgtcactc agattcctgg aggatttatc tgccaaaaat 480
   tcgcagccaa cagggtcttt ggctttgcca ttgtggctac ctccacccta aacatgttga 540
   tcccttcagc agcccgcgtt cactatggct gtgtcatctt cgtgaggatc cttcagggat 600
   tggtggaggg ggtcacatac cctgcttgcc atggcatctg gagcaaatgg gcccctccct 660
   tagaacggag tcggctggca acgacagcct tttgcggttc ctatgctggg gcggtggttg 720
   ccatgccctt ggctggggtc cttgtgcagt attcaggatg gagttctgtc ttctatgtct 780
   atggcagctt cgggatcttt tggtacctgt tctggttgct tgtctcctat gagtcaccgg 840
   cactgcaccc cagcatctct gaggaggagc gcaaatacat tgaggatgcc atcggggaga 900
   gcgccaagct catgaaccct gttacgaagt ttaacacacc ctggaggcgc ttctttacgt 960
   ccatgcccgt ctatgccatc atcgttgcga acttttgccg cagctggacc ttctacctgc 1020
   tcctcatctc tcagcccgcc tactttgaag aagtgttcgg ctttgagatc agcaaggtgg 1080
   ggctggtgtc ggcgctgcct caccttgtca tgaccatcat cgtacccatt ggaggccaga 1140
   tcgctgactt tttgcgcagt cgtcacataa tgtccactac caacgtgcga aagctcatga 1200
   actgcggggg tttcgggatg gaagccacgc tgctgctggt ggtcggatac tcgcactcca 1260
   agggcgtggc catctccttc ctggtcctgg ctgtgggctt cagtggcttt gccatctctg 1320
   ggtttaacgt gaaccacttg gacatcgccc ctcgctatgc cagcatcttg atgggcattt 1380
   ccaatggcgt gggcacactg tctgggatgg tgtgccccat catcgtgggt gcaatgacca 1440
   agcacaagac gcgggaggag tggcagtacg tgttcctcat agcctccctg gtgcactacg 1500
   gcggtgtcat cttctatggg gtctttgctt cgggagagaa gcagccgtgg gcagagccgg 1560
   aggagatgag cgaggagaag tgtggctttg ttggccacga ccagctggct ggcagtgacg 1620
   aaagtgaaat ggaggacgag gctgagcccc caggggcgcc ccccgcgccg cctccgtcct 1680
   acggggccac acacagcaca gtgcagcctc cgaggccccc gccccctgtc cgggactact 1740
   gaccacgggc ctcccactgt ggggcagttt ccaggacttc cactccatac acctctagcc 1800
   tgagcggcag tgtcgaggaa ccccactcct cccctgcctc aggcttaaga tgcaagtcct 1860
   cccttgttcc cagtgctgtc cgaccagccc tctttccctc tcaactgcct cctgcggggg 1920
   gtgaagctgc acactagcag tttcaaggat acccagactc ccctgaaagt cgttctccgc 1980
   ttgtttctgc ctgtgtgggc tcaaatctcc cctttgaggg ctttatttgg agggacagtt 2040
   caacctcttc ctctcttgtg gttttgaggt ttcacccctt cccccaagac cccagggatt 2100
   ctcaggctac cccgagatta ttcaggtggt cccctactca gaagacttca tggtcgtcct 2160
   ctattagttt caaggctcgc ctaaccaatt ctacattttt ccaagctggt ttaacctaac 2220
   caccaatgcc gccgttccca ggactgattc tcaccagcgt ttctgaggga aaatggcggt 2280
   ttcaagtccc cccacccccc ttttcttccc tcgtcccctc accagcacac tttgccgggc 2340
   cttgacctta gcttagtaca atcattgtcc agggaaatgg ccaaaatggc tctgctcacc 2400
   ccgtgctctt tttctgactc agttttcagg tctcagtagt ggctgcccaa agctattaat 2460
   tcagcggctc gaggccacct cttcctcccc gtggtggttt caggatcccc ctcgcccccc 2520
   cccccccaaa tccttgcact ttattctcct gggtggttcc aggccgccct cggtttctca 2580
   gtggccattt gttgtgtgtc cctcaggggc taaatgattc caaatctggg gtgcttcccc 2640
   tcatagacac ccctctctca acgtagaaat ctgggtgggg gtgaggtgtg tgagagaagt 2700
   tacagaatcc caggaaaggg agcggggctg ggaggagagg gttgttcctg gggcagggtc 2760
   gtgtcttggt gtctgtctct gtgacgtaaa tcctgccctg ccccacccca ctcccaataa 2820
   acgctctggt gtacgg                                                 2836
<210> 12
   <211> 560
   <212> PRT
   <213> Mus musculus
<400> 12
   Met Glu Phe Arg Gln Glu Glu Phe Arg Lys Leu Ala Gly Arg Ala Leu
   1               5                   10                  15
   Gly Arg Leu His Arg Leu Leu Glu Lys Arg Gln Glu Gly Ala Glu Thr
               20                  25                  30
   Leu Glu Leu Ser Ala Asp Gly Arg Pro Val Thr Thr His Thr Arg Asp
           35                  40                  45
   Pro Pro Val Val Asp Cys Thr Cys Phe Gly Leu Pro Arg Arg Tyr Ile
       50                  55                  60
   Ile Ala Ile Met Ser Gly Leu Gly Phe Cys Ile Ser Phe Gly Ile Arg
   65                  70                  75                  80
   Cys Asn Leu Gly Val Ala Ile Val Ser Met Val Asn Asn Ser Thr Thr
                   85                  90                  95
   His Arg Gly Gly His Val Val Val Gln Lys Ala Gln Phe Asn Trp Asp
               100                 105                 110
   Pro Glu Thr Val Gly Leu Ile His Gly Ser Phe Phe Trp Gly Tyr Ile
           115                 120                 125
   Val Thr Gln Ile Pro Gly Gly Phe Ile Cys Gln Lys Phe Ala Ala Asn
       130                 135                 140
   Arg Val Phe Gly Phe Ala Ile Val Ala Thr Ser Thr Leu Asn Met Leu
   145                 150                 155                 160
   Ile Pro Ser Ala Ala Arg Val His Tyr Gly Cys Val Ile Phe Val Arg
                   165                 170                 175
   Ile Leu Gln Gly Leu Val Glu Gly Val Thr Tyr Pro Ala Cys His Gly
               180                 185                 190
   Ile Trp Ser Lys Trp Ala Pro Pro Leu Glu Arg Ser Arg Leu Ala Thr
           195                 200                 205
   Thr Ala Phe Cys Gly Ser Tyr Ala Gly Ala Val Val Ala Met Pro Leu
       210                 215                 220
   Ala Gly Val Leu Val Gln Tyr Ser Gly Trp Ser Ser Val Phe Tyr Val
   225                 230                 235                 240
   Tyr Gly Ser Phe Gly Ile Phe Trp Tyr Leu Phe Trp Leu Leu Val Ser
                   245                 250                 255
   Tyr Glu Ser Pro Ala Leu His Pro Ser Ile Ser Glu Glu Glu Arg Lys
   260 265 270
   Tyr Ile Glu Asp Ala Ile Gly Glu Ser Ala Lys Leu Met Asn Pro Val
   275 280 285
   Thr Lys Phe Asn Thr Pro Trp Arg Arg Phe Phe Thr Ser Met Pro Val
   290 295 300
   Tyr Ala Ile Ile Val Ala Asn Phe Cys Arg Ser Trp Thr Phe Tyr Leu
   305 310 315 320
   Leu Leu Ile Ser Gln Pro Ala Tyr Phe Glu Glu Val Phe Gly Phe Glu
   325 330 335
   Ile Ser Lys Val Gly Leu Val Ser Ala Leu Pro His Leu Val Met Thr
   340 345 350
   Ile Ile Val Pro Ile Gly Gly Gln Ile Ala Asp Phe Leu Arg Ser Arg
   355 360 365
   His Ile Met Ser Thr Thr Asn Val Arg Lys Leu Met Asn Cys Gly Gly
   370 375 380
   Phe Gly Met Glu Ala Thr Leu Leu Leu Val Val Gly Tyr Ser His Ser
   385 390 395 400
   Lys Gly Val Ala Ile Ser Phe Leu Val Leu Ala Val Gly Phe Ser Gly
   405 410 415
   Phe Ala Ile Ser Gly Phe Asn Val Asn His Leu Asp Ile Ala Pro Arg
   420 425 430
   Tyr Ala Ser Ile Leu Met Gly Ile Ser Asn Gly Val Gly Thr Leu Ser
   435 440 445
   Gly Met Val Cys Pro Ile Ile Val Gly Ala Met Thr Lys His Lys Thr
   450 455 460
   Arg Glu Glu Trp Gln Tyr Val Phe Leu Ile Ala Ser Leu Val His Tyr
   465 470 475 480
   Gly Gly Val Ile Phe Tyr Gly Val Phe Ala Ser Gly Glu Lys Gln Pro
   485 490 495
   Trp Ala Glu Pro Glu Glu Met Ser Glu Glu Lys Cys Gly Phe Val Gly
   500 505 510
   His Asp Gln Leu Ala Gly Ser Asp Glu Ser Glu Met Glu Asp Glu Ala
   515 520 525
   Glu Pro Pro Gly Ala Pro Pro Ala Pro Pro Pro Ser Tyr Gly Ala Thr
   530 535 540
   His Ser Thr Val Gln Pro Pro Arg Pro Pro Pro Pro Val Arg Asp Tyr
   545 550 555 560
<210> 13
   <211> 2528
   <212> DNA
   <213> Mus musculus
<400> 13
   ggcacgaggc tcagtcttaa ttccactctg ccactctgcc gcagagcaca attacgccgg 60
   cgcgatggga ggagaccatg ttaaggcagg aagctaacag cagccgctca cctgaggcct 120
   aggaagctcc caagggttct gagagctatg agctctgatc agcaaagtca ccattttaga 180
   cagtagggtt cttttgcttt tttccttaca caagggttcg atgacgtttc tggtcaatct 240
   gattaaaaga cagcggattt gattgcgata agacttcaaa accgggaatt tacgttgttt 300
   ttcggtgagg tgacttccag aacagggact catcagcacc cgcccaaata ccacggcact 360
   gcgcgcgccc tcggccaccg gatcctcccc ttccaatgag actttgtgac tgtgtgtacc 420
   aattctccta ttaggaaacc cgtgggctgc atgcagctat tctgttgtac tctctttctc 480
   gccctccctc ccctctccaa ctcacagcct tgctggaaag ctcacctctg ctgagaagaa 540
   aaagctctac cttaaccaac taagactatg cgcagaatcc gtctttcata gccacaacaa 600
   tttaaatctg gtaaggctgg acaccagtct ttacaagaat ggagtcggta aaacaaagga 660
   ttttggcccc ggggaaagag gggataaaga attttgctgg aaaatccctc ggacagatct 720
   acagggtgct ggagaagaag caggacaacc gagagaccat cgagctgaca gaggacggta 780
   agcccctgga ggtgcctgag aagaaggctc cgctatgcga ctgcacgtgc ttcggcctgc 840
   cgcgccgcta catcatagcc atcatgagcg gcctcggctt ctgcatatcc ttcggcatcc 900
   gctgtaacct gggcgtggcc atcgtggaca tggtcaacaa cagcactatc caccgcggag 960
   gcaaagttat caaggagaaa gccaaattta actgggaccc cgagaccgtg gggatgatcc 1020
   acggatcgtt cttctggggc tatatcatca cccagattcc aggaggatat atcgcatcgc 1080
   ggctggctgc taaccgggtc tttggggctg cgatactgct cacctctacc ctcaatatgc 1140
   tgatcccatc tgcagccaga gtgcattatg gatgtgtcat ctttgttagg atattgcaag 1200
   gacttgtgga gggtgtcacc tacccagcct gtcatgggat atggagcaag tgggcccctc 1260
   ccttggagag gagtaggttg gctacaacct ccttttgtgg ttcctatgct ggagcagtca 1320
   ttgcaatgcc cttagctggt atccttgtgc agtacactgg atggtcgtca gtattttatg 1380
   tgtatggaag ctttggcatg gtctggtaca tgttctggct tctggtgtct tatgagagcc 1440
   ctgcaaagca tcctaccatt acagatgaag aacgtaggta catagaggag agcattggag 1500
   agagcgcaaa tctgctaggt gcaatggaaa aatttaagac cccatggagg aagtttttca 1560
   catccatgcc cgtctacgcg ataattgttg ccaacttctg caggagctgg actttttatt 1620
   tactgctcat cagtcagcca gcttattttg aggaggtttt tggatttgaa atcagcaagg 1680
   ttggcatgtt gtctgcagtc cctcaccttg tcatgacaat cattgtgcct atcggggggc 1740
   aaattgcaga tttcctaagg agcaagcaaa ttctctcaac aactacagtg agaaagatca 1800
   tgaattgtgg gggttttggc atggaagcca cgctgcttct ggttgttggc tactctcata 1860
   ctagaggggt ggccatctcc ttcttggtgc ttgcagtagg attcagtgga tttgctatct 1920
   ctggtttcaa tgttaatcac ttggatattg ctccaagata tgccagtatc ttaatgggca 1980
   tttcaaatgg cgttggcacg ctgtcgggga tggtttgccc tatcattgtt ggtgcaatga 2040
   caaagaataa gtcccgtgaa gaatggcagt atgtcttcct cattgctgca ctcgtccact 2100
   atggtggagt catattttat gcactatttg cctcaggaga gaaacaacct tgggcagacc 2160
   ctgaggaaac aagcgaagaa aaatgtggct tcattcacga agatgaactg gatgaagaaa 2220
   cgggggacat cactcagaat tacataaatt acggtaccac caaatcttac ggtgctacct 2280
   cacaggagaa tggaggctgg cctaacggct gggagaaaaa ggaagaattt gtgcaagaag 2340
   gtgcgcaaga cgcgtacacc tataaggacc gagatgatta ttcataacga tgctagttgc 2400
   tggattcatt tgtagtgttt gtgaatcaat taattgtgat tgcacaaaaa taattttaaa 2460
   aatgtggtgt gaacatgtaa acatatcaac caagcaagtc ttgctgttca aaaaaaaaaa 2520
   aaaaaaaa                                                          2528
<210> 14
   <211> 582
   <212> PRT
   <213> Mus musculus
<400> 14
   Met Glu Ser Val Lys Gln Arg Ile Leu Ala Pro Gly Lys Glu Gly Ile
    1               5                  10                  15
   Lys Asn Phe Ala Gly Lys Ser Leu Gly Gln Ile Tyr Arg Val Leu Glu
               20                  25                  30
   Lys Lys Gln Asp Asn Arg Glu Thr Ile Glu Leu Thr Glu Asp Gly Lys
           35                  40                  45
   Pro Leu Glu Val Pro Glu Lys Lys Ala Pro Leu Cys Asp Cys Thr Cys
       50                  55                  60
   Phe Gly Leu Pro Arg Arg Tyr Ile Ile Ala Ile Met Ser Gly Leu Gly
   65                  70                  75                  80
   Phe Cys Ile Ser Phe Gly Ile Arg Cys Asn Leu Gly Val Ala Ile Val
                   85                  90                  95
   Asp Met Val Asn Asn Ser Thr Ile His Arg Gly Gly Lys Val Ile Lys
               100                 105                 110
   Glu Lys Ala Lys Phe Asn Trp Asp Pro Glu Thr Val Gly Met Ile His
           115                 120                 125
   Gly Ser Phe Phe Trp Gly Tyr Ile Ile Thr Gln Ile Pro Gly Gly Tyr
   130 135 140
   Ile Ala Ser Arg Leu Ala Ala Asn Arg Val Phe Gly Ala Ala Ile Leu
   145 150 155 160
   Leu Thr Ser Thr Leu Asn Met Leu Ile Pro Ser Ala Ala Arg Val His
   165 170 175
   Tyr Gly Cys Val Ile Phe Val Arg Ile Leu Gln Gly Leu Val Glu Gly
   180 185 190
   Val Thr Tyr Pro Ala Cys His Gly Ile Trp Ser Lys Trp Ala Pro Pro
   195 200 205
   Leu Glu Arg Ser Arg Leu Ala Thr Thr Ser Phe Cys Gly Ser Tyr Ala
   210 215 220
   Gly Ala Val Ile Ala Met Pro Leu Ala Gly Ile Leu Val Gln Tyr Thr
   225 230 235 240
   Gly Trp Ser Ser Val Phe Tyr Val Tyr Gly Ser Phe Gly Met Val Trp
   245 250 255
   Tyr Met Phe Trp Leu Leu Val Ser Tyr Glu Ser Pro Ala Lys His Pro
   260 265 270
   Thr Ile Thr Asp Glu Glu Arg Arg Tyr Ile Glu Glu Ser Ile Gly Glu
   275 280 285
   Ser Ala Asn Leu Leu Gly Ala Met Glu Lys Phe Lys Thr Pro Trp Arg
   290 295 300
   Lys Phe Phe Thr Ser Met Pro Val Tyr Ala Ile Ile Val Ala Asn Phe
   305 310 315 320
   Cys Arg Ser Trp Thr Phe Tyr Leu Leu Leu Ile Ser Gln Pro Ala Tyr
   325 330 335
   Phe Glu Glu Val Phe Gly Phe Glu Ile Ser Lys Val Gly Met Leu Ser
   340 345 350
   Ala Val Pro His Leu Val Met Thr Ile Ile Val Pro Ile Gly Gly Gln
   355 360 365
   Ile Ala Asp Phe Leu Arg Ser Lys Gln Ile Leu Ser Thr Thr Thr Val
   370 375 380
   Arg Lys Ile Met Asn Cys Gly Gly Phe Gly Met Glu Ala Thr Leu Leu
   385                 390                 395                 400
   Leu Val Val Gly Tyr Ser His Thr Arg Gly Val Ala Ile Ser Phe Leu
                   405                 410                 415
   Val Leu Ala Val Gly Phe Ser Gly Phe Ala Ile Ser Gly Phe Asn Val
               420                 425                 430
   Asn His Leu Asp Ile Ala Pro Arg Tyr Ala Ser Ile Leu Met Gly Ile
           435                 440                 445
   Ser Asn Gly Val Gly Thr Leu Ser Gly Met Val Cys Pro Ile Ile Val
       450                 455                 460
   Gly Ala Met Thr Lys Asn Lys Ser Arg Glu Glu Trp Gln Tyr Val Phe
   465                 470                 475                 480
   Leu Ile Ala Ala Leu Val His Tyr Gly Gly Val Ile Phe Tyr Ala Leu
                   485                 490                 495
   Phe Ala Ser Gly Glu Lys Gln Pro Trp Ala Asp Pro Glu Glu Thr Ser
               500                 505                 510
   Glu Glu Lys Cys Gly Phe Ile His Glu Asp Glu Leu Asp Glu Glu Thr
           515                 520                 525
   Gly Asp Ile Thr Gln Asn Tyr Ile Asn Tyr Gly Thr Thr Lys Ser Tyr
       530                 535                 540
   Gly Ala Thr Ser Gln Glu Asn Gly Gly Trp Pro Asn Gly Trp Glu Lys
   545                 550                 555                 560
   Lys Glu Glu Phe Val Gln Glu Gly Ala Gln Asp Ala Tyr Thr Tyr Lys
                   565                 570                 575
   Asp Arg Asp Asp Tyr Ser
               580

## Beschreibung

Die Erfindung betrifft ein Verfahren zur Auffindung pharmazeutisch relevanter Substanzen unter Verwendung von BNPI bzw. davon abgeleiteter Biomoleküle.

Das Auffinden der Angriffsorte pharmazeutischer Wirkstoffe, der sogenannten "Targets" ist eine der wichtigsten Aufgaben moderner Pharmaforschung. Über die Affinitäten an diesen Targets oder auch über die durch eine Wechselwirkung mit diesen Targets ausgelösten physiologischen Effekte lassen sich über sogenannte "Screeningverfahren" aus der Vielzahl von bekannter Substanzen, beispielsweise aus den Substanzbibliotheken der pharmazeutischen Forschung, interessante Substanzen oder Substanzklassen herausfiltern, die in den mit diesem Target assoziierten Indikationen mit hoher Wahrscheinlichkeit wirksam sind. Zu den wichtigsten Vertretern dieser Targets gehören Proteine, im allgemeinen Rezeptoren, insbesondere G-Protein gekoppelte Rezeptoren, und Transportproteine. Die Auffindung dieser Targets gestaltet sich aber teilweise sehr schwierig, da die potentielle Auswahl sehr groß ist. Zur Orientierung und Identifizierung dienen zum einen Erkenntnisse über die (physiologische) Funktion mit der (potentiellen) Position in Signalkaskaden und Stoffwechselwegen zum anderen aber auch Lokalisation und Expressionsgrad in den verschiedenen Geweben. Im Rahmen dieser Erfindung wurde dabei ein besonderes Augenmerk auf das zentrale Nervensystem gerichtet, wo es nicht nur auf eine generelle Lokalisation sondern auf eine sehr spezifische und präzise Verteilung in den verschiedenen Regionen ankommt.

Verfahren zum Auffinden von Targets werden im Stand der Technik zwar diskutiert, jedoch beschreibt kein Stand der Technik einen Zusammenhang zwischen BNPI, davon abgeleitetetn Wirkstoffen und bestimmten Indikationen. So offenbart z.B. WO 01/64835 A aufgrund von Sequenzierungsprojekten erhaltene Nukleinsäure- und Aminosäuresequenzen sowie in allgemeiner und unbestimmter Form ein Verfahren zur Identifizierung von Wirkstoffen, die an die offenbarten Polypeptide binden. Das in WO 01/64835 A gezeigte Screeningverfahren verwendet potentiell jede der über 27.000 genannten Sequenzen, setzt diese jedoch in keinen Bezug zu einer bestimmten Indikation oder beschreibt über solche Screeningverfahren identifizierte Wirkstoffe.

Die Druckschrift US-A-5 985 604 offenbart die Nukleinsäure- und Aminosäuresequenz des "human sodium-dependent phosphate cotransporters" NAPTR. Weiterhin beschreibt US-A-5 985 604 Verfahren zur Herstellung dieses Polypeptids sowie zur Detektion des Polypeptids aus einer Probe. US-A-5 985 604 beschreibt jedoch keine Verfahren zur Identifizierung von Wirkstoffen, die mit NAPTR eine Bindung eingehen oder beschreibt einen Zusammenhang mit bestimmten Indikationen.

WO 01/57190 A beschreibt das aus adulter Zervix isolierte "human epidermal protein-1" sowie Verfahren zur Detektion von Polynukleotiden bzw. Polypeptiden von humanem "epidermal protein-1" aus einer Probe. WO 01/57190 A beschreibt nicht BNPI und stellt keinen Zusammenhang zwischen BNPI und bestimmten Indikationen her.

Aufgabe der Erfindung war daher die Auffindung und Identifizierung eines oder mehrerer derartiger Targets, insbesondere mit zentralnervöser Lokalisation und Wirksamkeit, und die Entwicklung eines entsprechenden Screeningverfahrens. Die Erfindung betrifft daher ein Verfahren zur Auffindung pharmazeutisch relevanter Substanzen mit Wirksamkeit in den Indikationen oder zur Behandlung von amyotropher Lateralsklerose.

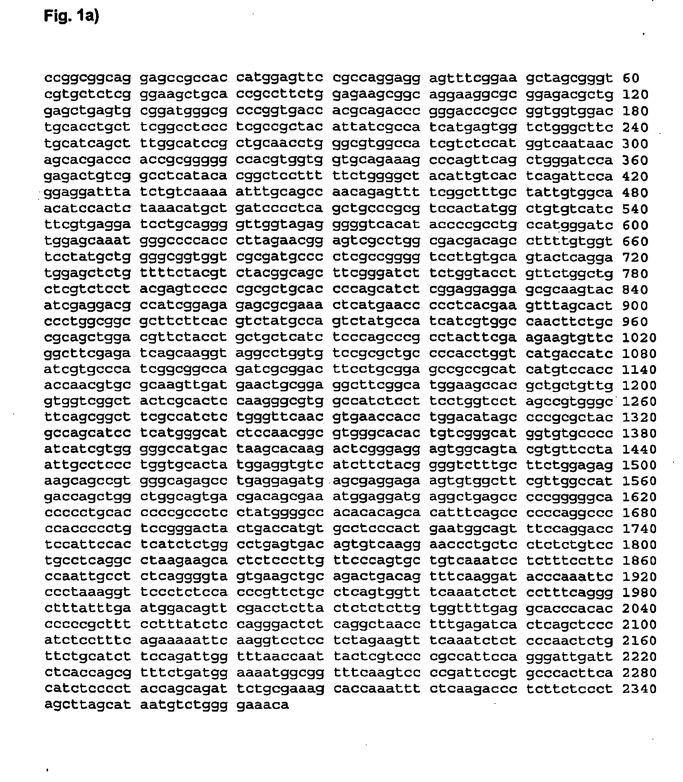

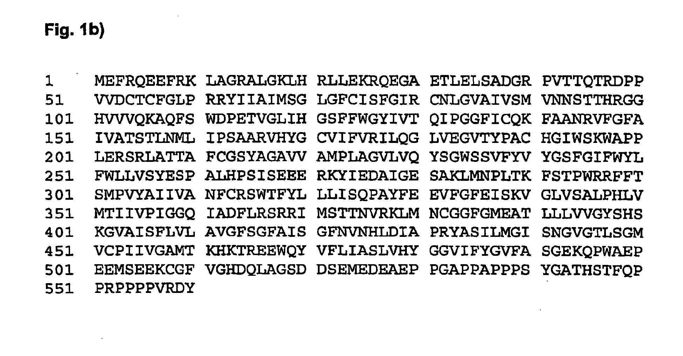

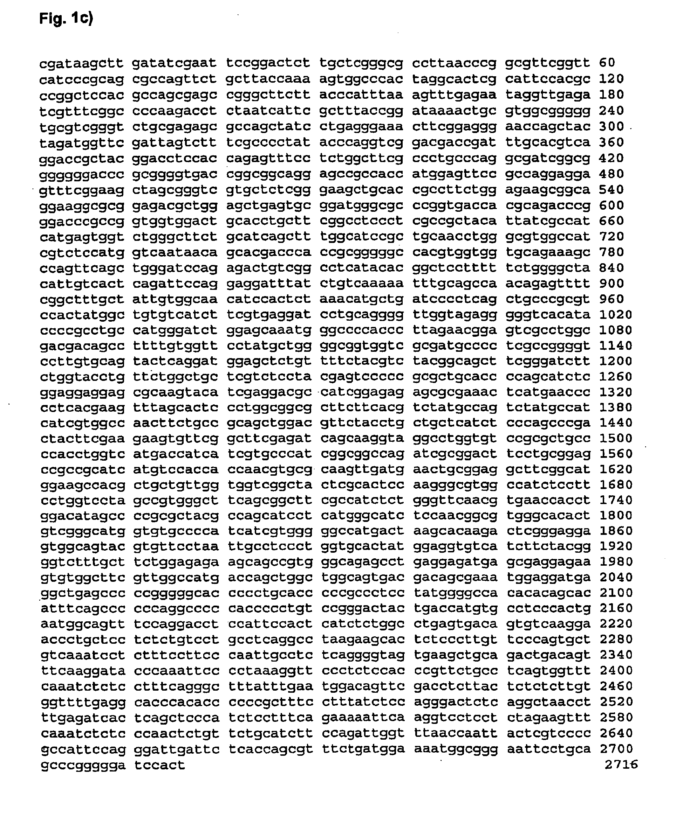

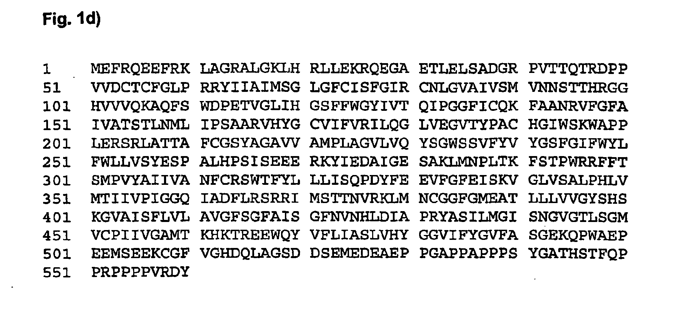

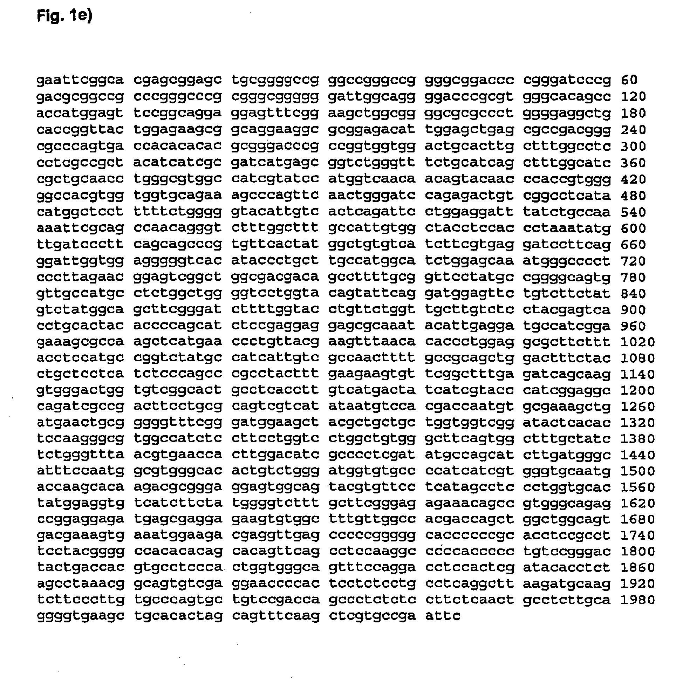

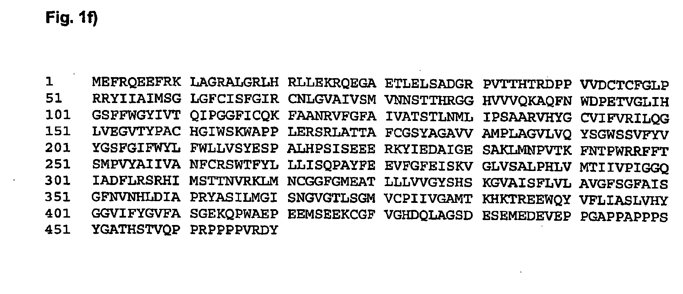

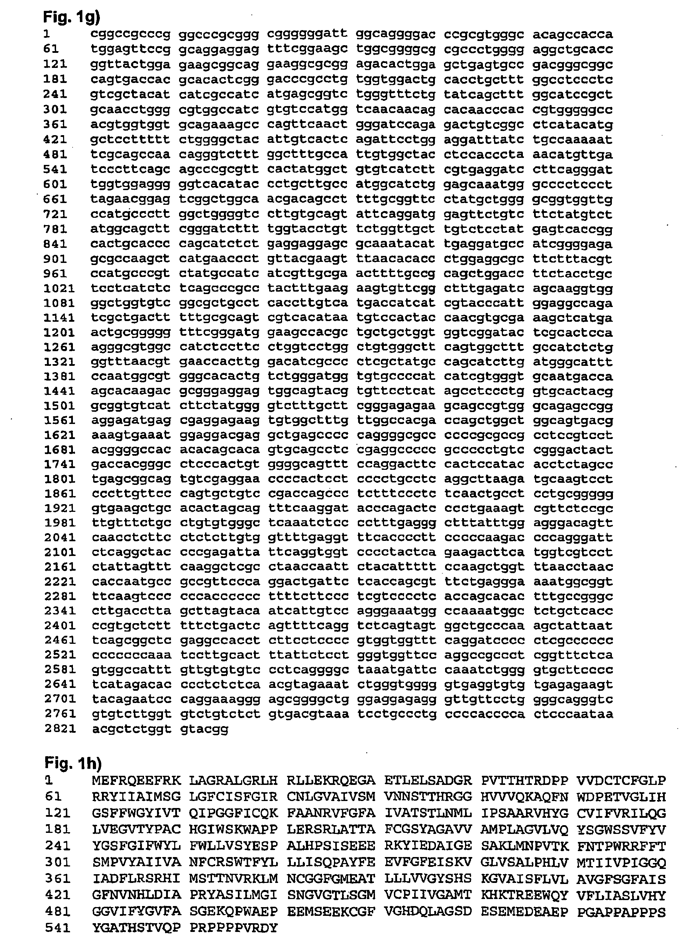

Erkrankungen des spinalen Motoneurons, Muskelatrophien, Muskeldystrophien, oder multipler Sklerose, mit folgenden Verfahrensschritten:
(a) Inkubation einer zu testenden Substanz unter geeigneten Bedingungen mit mindestens einem Biomolekül aus Gruppe I ausgewählt aus:
   dem Protein BNPI und/oder einem Protein gemäß einer der Abbildungen 1b), 1d), 1f), 1h) und/oder einem zu einem dieser vorgenannten Proteine zu mindestens 90 % ähnlichen Protein und/oder einem Protein, für das ein Polynukleotid gemäß einer der Abbildungen 1a), 1c), 1e), 1g) oder ein dazu zu mindestens 90 % ähnliches Polynukleotid kodiert, und/oder einem Protein, das durch eine Nukleinsäure kodiert wird, die unter stringenten Bedingungen an ein Polynukleotid gemäß einer der Abbildungen 1a), 1c), 1e), 1g) oder deren Antisense Polynukleotide bindet,
   und/oder einer Zelle und/oder einer Präparation aus einer solchen Zelle, die mindestens eines der vorgenannten Proteine bzw. Biomoleküle aus Gruppe I, synthetisiert hat,
(b) Messung der Bindung der Testsubstanz an dem oder den gegebenenfalls von einer Zelle synthetisierten Biomolekül/en aus Gruppe I oder an einer Zelle und/oder einer Präparation aus einer solchen Zelle, die mindestens eines der vorgenannten Biomoleküle aus Gruppe 1 synthetisiert hat, oder Messung mindestens eines der durch die Bindung der Testsubstanz an dem oder den gegebenenfalls von einer Zelle synthetisierten Biomolekül/en aus Gruppe I oder an einer Zelle und/oder einer Präparation aus einer solchen Zelle, die mindestens eines der vorgenannten Biomoleküle aus Gruppe 1 synthetisiert hat, veränderten funktionellen Parameter
   über Messung der Regulation, Hemmung und/oder Aktivierung von Rezeptoren, lonenkanälen und/oder Enzymen, insbesondere über Messung der Veränderung der Genexpression, des lonenmilieus, des pH oder des Membranpotentials, über Veränderung der Enzymaktivität oder der Konzentration der 2^{nd} messenger, oder
   über Messung der Bindung über die Verdrängung eines bekannten markierten Liganden des Biomoleküls und/oder Proteins und/oder über die daran gebundene Aktivität einer markierten Testsubstanz.

Dieses neue Screeningverfahren basiert darauf, daß hier eine potentielle medizinische Wirksamkeit einer Substanz Ober ihre Wechselwirkung mit mindestens einer physiologisch relevanten Protein- oder Peptidstruktur, einem Target, BNPI oder verwandten Strukturen, aufgefunden werden kann. BNPI wird in der Literatur und zum Teil auch dieser Erfindung als VGLUT1 bezeichnet. Diese Begriffe sind daher insbesondere im Rahmen dieser Erfindung als völlig gleichbedeutend anzusehen. BNPI bzw. die davon abgeleiteten hier aufgezählten Proteine bzw. Peptide oder für diese kodierenden Nukleinsäuren wurden im Rahmen dieser Erfindung als interessante Targets identifiziert. Dabei zeigte BNPI eine Lokalisation in den unterschiedlichsten ZNS-Bereichen, aber überraschenderweise - trotz teilweise engster Nachbarschaft - auch eine stets streng getrennte Lokalisation, wobei gerade diese strenge Trennung deutlich darauf hindeutet, daß über BNPI wichtige physiologische Prozeße gesteuert werden. BNPI (VGLUT1) wird vor allem von einer Population großer DRG-Neurone stark exprimiert, die Substanz P negativ sind. Das verwandte DNPI (VGLUT2) hingegen, bzw. dessen mRNA findet sich vor allem in den mittelgroßen und kleinen Substanz-P-positiven Neuronen. Die Lokalisation von VGLUT1 (BNPI) ist völlig unabhängig von der Lokalisation des verwandten Transporters VGLUT3. Da BNPI in therapeutisch sehr interessanten Bereichen des ZNS lokalisiert sind und daher für eine entsprechende Vielzahl von Indikationen von Interesse sind, ist BNPI entsprechend ein wichtiges Target, mit dem Screeningverfahren auf pharmakologisch wirksame Verbindungen durchgeführt werden können. Folglich ist auch bevorzugt, wenn in einem Screeningverfahren BNPI bzw. jeweils eines der davon abgeleiteten hier aufgezählten Biomoleküle wie Proteine bzw. Peptide oder eine für diese kodierende Nukleinsäure eingesetzt werden oder das Ergebniss zweier getrennter Screeningverfahren mit BNPI bzw. einem der davon abgeleiteten hier aufgezählten Biomoleküle wie Protein bzw. Peptid oder einer für diese kodierenden Nukleinsäure durchgeführt werden und in beiden Fällen durch differentiellen Abgleich der Daten optimiert pharmakologisch wirksame Substanzen identifiziert werden, die dann hochspezifisch sind.

Dabei beziehen sich die Begriffe pharmazeutisch relevant oder pharmakologisch wirksam auf einen potentiell heilenden oder lindemden Einfluß der Substanz auf bestimmte Krankheitsbilder. Der Begriff Substanz umfaßt jede als Arzneimittel-Wirkstoff geeignete Verbindung, insbesondere also niedermolekulare Wirkstoffe, aber auch andere wie Nukleinsäuren, Fette, Zucker, Peptide oder Proteine wie Antikörper.

Die Inkubation unter geeigneten Bedingungen ist hier so zu verstehen, daß die zu untersuchende Substanz mit dem Biomolekül oder der Zelle oder der entsprechenden Präparation in einem wässrigen Medium eine definierte Zeit vor der Messung reagieren kann. Dabei kann das wässrige Medium temperiert werden, beispielsweise zwischen 4°C und 40°C, vorzugsweise bei Raumtemperatur oder bei 37°C. Die Inkubationszeit kann zwischen wenigen Sekunden und mehreren Stunden variiert werden, je nach der Wechselwirkung der Substanz mit dem Biomolekül oder Protein. Bevorzugt sind aber Zeiten zwischen 1 min und 60 min. Das wäßrige Medium kann geeignete Salze und/oder Puffersysteme enthalten, so daß bei der Inkubation beispielsweise ein pH zwischen 6 und 8, vorzugsweise pH 7,0 - 7,5 im Medium herrscht. Dem Medium können weiter geeignete Substanzen, wie Coenzyme, Nährstoffe etc. beigefügt werden. Die geeigneten Bedingungen können vom Fachmann in Abhängigkeit von der zu untersuchenden Wechselwirkung der Substanz mit dem Protein aufgrund seiner Erfahrung, der Literatur oder weniger, einfacher Vorversuche leicht festgelegt werden, um im Verfahren einen möglichst deutlichen Meßwert zu erhalten.

Eine Zelle, die ein bestimmtes Protein bzw. Biomolekül synthetisiert hat, ist eine Zelle, die dieses Protein bereits endogen exprimiert hat oder eine solche, die gentechnisch verändert wurden, so daß sie dieses Protein bzw. Biomolekül exprimiert und entsprechend vor Beginn des erfindungsgemäßen Verfahrens das Protein enthält. Die Zellen können Zellen aus evt. immortalisierten Zellinien sein oder native aus Geweben stammende und aus diesen isolierte Zellen sein, wobei der Zellverband meist aufgelöst ist. Die Präparation aus diesen Zellen umfaßt insbesondere Homogenate aus den Zellen, das Cytosol, eine Membranfraktion der Zellen mit Membranfragmenten, eine Suspension isolierter Zellorganellen etc.

Aus welcher Spezies diese Proteine Biomoleküle stammen, ist für die Funktion des Verfahrens unerheblich, es ist aber bevorzugt, die humane, Maus- oder Ratten-Variante zu verwenden. BNPI ist in Hinblick auf die kodierende DNA- und die Aminosäure-Sequenz bekannt und auch in seiner generellen Funktion beschrieben. BNPI, der "brain Na+ dependent inorganic phosphate cotransporter" ist in der WO 96/34288 beschrieben. Neben der Funktion als nairiumabhängigen Phosphattransporter wurde für BNPI auch eine Funktion als vesikulärer Glutamat-Transporter beschrieben und BNPI als VGlutT1 bezeichnet (Bellocchio et al. (2000), Science 189:957-960; Takamori et al. (2000), Nature 407; 189-194).

Der Maßstab, über den das Verfahren die Auffindung interessanter Substanzen erlaubt, ist entweder die Bindung an das Biomolekül, das Protein, die durch Verdrängung eines bekannten Liganden oder das Ausmaß gebundener Substanz nachgewiesen werden kann, oder die Veränderung eines funktionellen Parameters durch die Wechselwirkung der Substanz mit dem Protein bzw. Biomolekül. Diese Wechselwirkung kann insbesondere in einer Regulation, Hemmung und/oder Aktivierung von Rezeptoren, lonenkanälen und/oder Enzymen liegen und veränderte funktionelle Parameter können beispielsweise die Genexpression, das Ionenmilieu der pH oder das Membranpotential bzw. die Veränderung der Enzymaktivität oder der Konzentration der 2^{nd} messenger sein.

Zur Erläuterung der Erfindung werden im folgenden neben den im allgemeinen Text zu Begriffen gegebenen Erklärungen weitere Definitionen angegeben, um klarzustellen, wie bestimmte, insbesondere in den Ansprüchen verwendete Begriffe im Sinne dieser Erfindung zu verstehen und auszulegen sind.
- Substanz: Damit ist eine chemische Verbindung gemeint. Hier handelt es sich im engeren Sinne um Verbindungen, die potentiell eine Wirkung im Körper entfalten können, niedermolekulare Wirkstoffe, Nukleinsäuren, Fette, Zucker, Peptide oder Proteine, insbesondere hier niedermolekulare Wirkstoffe.
- pharmazeutisch relevante Substanz: Im Sinne der Erfindung ist eine pharmazeutisch relevante Substanz eine Substanz, die über die Bindung an die Biomoleküle der Gruppen I bis III in wenigstens einer der genannten Indikationen wirksam sein könnte und theoretisch das Potential besitzt, physiologisch die Symptome direkt oder indirekt zu beeinflußen, insbesondere so erscheint, als könne sie therapeutisch, beispielsweise in einem Arzneimittel, eingesetzt werden.
- schmerzregulierend: Im Sinne der Erfindung heißt schmerzregulierend, daß die Substanz die Wahrnehmung von Schmerz direkt oder indirekt beeinflußt, insbesondere natürlich analgetisch wirkt.
- Schmerz: Im Sinne der Erfindung bedeutet Schmerz insbesondere ein Schmerzempfinden, präziser akkuter, chronischer, neuropathischer und entzündlicher Schmerz inclusive Migräne, insbesondere ist der Schmerz zugehörig zu folgenden Arten:
   chronischem Schmerz, insbesondere muskuloskelettalem Schmerz; neuropathischem Schmerz, insbesondere allodynischem Schmerz, mechanischer Hyperalgesie oder diabetischer Neuropathie; viszeralem Schmerz, cerebralem Schmerz, peripherem Schmerz oder entzündungsbedingtem Schmerz, insbesondere peripherem Entzündungsschmerz; sowie Migräne, Cluster-Kopfschmerz oder den Schmerz bei Trigeminus Neuralgie.
- Inkubation: Unter Inkubation ist das Einbringen und Belassen eines biologischen Untersuchungsobjektes, beispielsweise einer Zelle oder eines Proteins, in einem temperierten Medium wie in einem Brutschrank oder auf einem Wasserbad zu verstehen. Dabei heiß hier unter geeigneten Bedingungen eine Inkubation unter physiologischen Bedingungen (z.B. 37°C pH7,2) oder bei den Bedingungen, bei denen eine optimale Messung im Verfahren möglich wird.
- Zelle: Die Zelle ist ein sich selbst regulierendes, offenes, mit seiner Umgebung durch permanenten Stoffaustausch in einem Fließgleichgewicht stehendes System mit eigenem Stoffwechsel, und Vermehrungsfähigkeit. Die Zelle kann separat kultiviert oder Teil eines Gewebes, insbesondere aus einem Organ, sein, und dort vereinzelt oder noch im Zellverband vorliegen.
- Präparation aus einer Zelle: Darunter versteht man Präparate, die mittels chemischer, biologischer, mechanischer oder physikalischer Methoden unter Änderung der Zellstruktur hergestellt werden, beispielsweise Membranfragmente, isolierte Zellkompartimente, isoliertes Cytosol, oder aus Gewebe gewonnenes Homogenat.
- Peptid: Verbindung aus über peptidische Bindungen zu Ketten verknüpften Aminosäuren. Ein Oligopeptid besteht aus zwischen 2 und 9 Aminosäuren, ein Polypeptid aus zwischen 10 und 100 Aminosäuren.
- Protein: Verbindung aus über peptidische Bindungen zu Ketten verknüpften mehr als 100 Aminosäuren u.U. mit einer definierten Raumstruktur.
- PIM1-Kinase: PIM3-Kinase: Ein Proto-Oncogen und eine Serin-Threonin-Kinase.
- Polynukleotid: Das zugrundeliegende Nukleotid ist ein grundsätzlich aus Nucleinbase, Pentose und Phosphorsäure bestehender Grundbaustein der Nucleinsäuren. Diese entspricht einem hochmolekularen Polynucleotid aus mehreren Nukleotiden, die über Phosphorsäure-Pentose-Veresterung miteinander verknüpft sind. Unter diese Erfindung fallen aber auch modifizierte Polynukleotide, die zwar die Basenabfolge beibehalten, aber über ein modifiziertes Rückrat statt der Phosphorsäure-Pentose verfügen.
- zu mindestens 90 (95, 97)% ähnlich: Darunter ist zu verstehen, daß die mit erfaßten Polynucleotide in ihrem kodierenden Bereich beuzüglich der Basenabfolge zu mindestens 90% (95%, 97%) identisch mit der Referenz (Abbildung etc.) sind und die mit erfaßten Peptide und Proteine in ihrer Primärstruktur, der Abfolge der Aminosäuren zu mindestens 90% (95%, 97%) mit der Referenz identisch sind.
- Gen: Mit dem Begriff Gen wird ein Genomabschnitt mit einer definierten Nukleotidsequenz bezeichnet, der die Information zur Synthese einer m- oder prä-mRNA oder einer sonstigen RNA (z.B. tRNA, rRNA, snRNA etc.) enthält. Es besteht aus kodierenden und nicht kodierenden Abschnitten.
- Genfragment: Nukleinsäureabschnitt, der in seiner Basenabfolge einen Teilbereich eines Gens beinhaltet
- Biomolekül: Allgemeiner Begriff für Nukleinsäuren oder Polyaminosäuren, insbesondere auch DNA, RNA, Peptide und Proteine, wobei diese Moleküle auch künstlich verändert sein dürfen. Im Sinne dieser Erfindung vorzugsweise Peptide und Proteine.
- Bindung an das Peptid oder Protein: Wechselwirkung zwischen Substanz und Peptid oder Protein, die zu Fixierung führt.
- funktionelle Parameter: darunter versteht man Meßgrößen eines Experimentes, die mit der Funktion eines Proteins (lonenkanal, Rezeptor, Enzym) korrelieren
- gentechnisch manipuliert: Manipulation von Zellen, Geweben oder Organismen derart, daß hier genetisches Material eingebracht wird
- endogen exprimiert: Expression eines Proteins, die eine Zelllinie unter geeigneten Kulturbedingungen aufweist, ohne das dieses entsprechende Protein durch gentechnische Manipulation zur Expression veranlasst wurde
- G-Protein: International übliche Abkürzung für ein Gunaosintriphosphat (GTP)-bindendes Protein, das als Signalprotein durch G-Protein gekoppelte Rezeptoren aktiviert wird
- Reportergen: Generelle Bezeichnung für Gene, deren Genprodukte sich mit Hilfe einfacher biochemischer Methoden oder histochemischer Methoden einfach nachweisen lassen, wie z.B. Luziferase, alkalische Phosphatase oder Green Fluorescent Protein (GFP).
- (rekombinantes) DNA-Konstrukt: Generelle Bezeichnung für jede Art von DNA-Molekülen, die durch die in vitro-Verknüpfung von DNA-Molekülen entstanden sind.
- Klonierungsvektor: Generelle Bezeichnung für Nukleinsäure-Moleküle, die beim Klonieren als Träger von Fremdgenen oder Teilen dieser Gene dienen.
- Expressionsvektor. Bezeichnung für speziell konstruierte Klonierungsvektoren, die nach Einbringen in eine geeignete Wirtszelle die Transcription und Translation des in den Vektor einklonierten Fremdgens erlauben.
- LTR-Sequenz: Abkürzung für Long terminal repeat. Generelle Bezeichnung für längere Sequenzbereiche, die an beiden Enden eines linearen Genoms zu finden sind. Derartige Sequenzbereiche kommen z.B. in den Genomen von Retroviren und an den Enden eukaryontischer Transposons vor.
- Poly-.A-Schwanz: die am 3'-Ende von messenger-RNAs durch Polyadenylierung angeheftenen Adenyl-Reste (ca. 20-250).
- Promotor-Sequenz: Bezeichnung für einen DNA-Sequenzbereich, von dem aus die Transkription eines Gens, d.h. die Synthese der mRNA, gesteuert wird.
- ORI-Sequenz: Abkürzung für Origin of replication. Die ORI-Sequenz erlaubt einem DNA-Molekül, sich als autonome Einheit in der Zelle zu vermehren.
- Enhancer-Sequenz: Bezeichung für relativ kurze, zum Teil als Repetitionen auftretende, genetische Elemente, die in der Regel die Expression mancher Gene in unterschiedlichem Maße verstärken.
- Transkriptionsfaktor: Bezeichnung für ein Protein, das über eine Bindung an spezifische DNA-Sequenzen, die Transkription eines Gens beeinflußt.
- kultivieren: Zellen oder Gewebe unter geeigneten Kulturbedingungen halten
- Bedingungen, die eine Expression erlauben, darunter versteht man die Auswahl und Anwendung von Kulturbedingungen die eine Expression des interessierenden Proteins erlauben, darunter gehören Temperaturänderung, Mediumwechsel, Zusatz von induzierenden Substanzen, Weglassen hemmender Substanzen.
- Inkubationszeit: Zeitdauer für die Zellen oder Gewebe inkubiert, d.h. einer definierten Temperatur ausgesetzt werden.
- Selektionsdruck: Anwendungen von Kulturbedingungen die Zellen mit einem bestimmtem Genprodukt, dem sog. Selektionsmarker, einen Wachstumsvorteil verschaffen.
- Amphibienzelle, Zelle aus einem Tier der Klasse der Amphibia
- Bakterienzelle, Zelle die dem Überreich der Eubacteria oder Archaebacteria zuzuordnen ist, oder von ihr abstammt.
- Hefezelle, Zelle die der Ordnung der Endomycetalse zuzuordnen ist, oder von ihr abstammt.
- Insektenzelle, Zelle die der Ordnung der Hexapoda zuzuordnen ist, oder von ihr abstammt.
- native Säugetierzelle, aus einem Säugetier stammende Zelle, die in ihren relevanten Merkmalen der im Organismus befindlichen Zelle entspricht.
- immortalisierte Säugetierzelle: Zelle die durch die angewendeten Kulturbedingungen oder gentechnische Manipulation die Eigenschaft erlangt hat, sich über die normalerweise übliche Teilungshäufigkeit hinaus (ca.100) in der Kultur zu teilen.
- markiert: durch entsprechende Modifizierung oder Derivatisierung für eine Nachweisreaktion zugänglich gemacht. Beispielsweise radioaktiv, fluoreszierend oder lumineszierend.
- Ligand: Substanz, die an ein im Körper oder einer Zelle befindliches Molekül, im speziellen einen Rezeptor, bindet
- Verdrängung: vollständiges oder partielles Entfernen eines Liganden von seiner Bindungsstelle
- gebundene Aktivität: Biochemisch oder physikalisch erfaßter Meßwert, der mit der an einem Rezptor gebundenen Ligandenmenge korreliert
- Regulation: die als Teil eines Regelprozese erfolgte Hemmung oder Aktivierung eines Vorgangs
- Hemmung: als Sonderfall der Regulation die Verhinderung/Minderung eines Vorgangs
- Aktivierung: als Sonderfall der Regulation die Verstärkung eines Vorgangs
- Rezeptoren. Im weitesten Sinne alle im pro- oder eukaryoten Organismus vorhandenen Moleküle, an die ein Wirkstoff binden kann. Im engeren Sinne membrangebundene Proteine oder Komplexen mehrerer Proteine, die durch Bindung eines Wirkstoffes eine Änderung in der Zelle hervorrufen.
- lonenkanäle: Membrangebundene Proteine oder Komplexe mehrerer Proteine, durch die Kationen oder Anionen durch die Membran hindurchgelangen können.
- Enzyme: Bezeichnung für Proteine oder Komplexe aus einer aktivierenden Nichteiweißkomponente mit einem Protein, die katalytische Eigenschaften besitzen.
- Genexpression (exprimieren/exprimierbar): das Übersetzen der genetischen Information eines Genes in RNA (RNA-Expression) oder in Protein (Proteinexpression).
- Ionenmilieu: Ionenkonzentration eines oder mehrerer Ionen in einem bestimmten Kompartiment.
- Membranpotential: Spannungsdifferenz über eine Membran aufgrund eines Überschusses an Kationen auf der einen Seite und Anionen auf der anderen Seite der Membran.
- Veränderung der Enzymaktivität: Hemmung oder Induktion der katalytischen Aktivität eines Enzyms.
- 2^{nd} messenger: kleines Molekül, das als Antwort auf ein extrazelluläres Signal entweder im Cytosol gebildet wird oder in das Cytosol hineinwandert und dabei hilft die Information an das Zelliniere weiterzugeben, wie zum Beispiel cAMP, IP₃.
- (Gen-)Sonde: Bezeichnung für jede Art von Nukleinsäuren, mit deren Hilfe man ein gesuchtes Gen oder eine bestimmte DNA-Sequenz nachweisen kann. Durch Derivatisierung der Gensonde (z.B. Biotin, magnetische Beads, Digoxinin) können zudem DNA-Moleküle aus einem Gemisch herausgezogen werden. Als Sonden werden klonierte Gene, Genfragmente, chemisch synthetisierte Oligonukleotide und auch RNA verwendet, die meist radioaktiv markiert ist.
- DNA: Internationale Bezeichnung für Desoxyribonukleinsäure
- genomische DNA: Generelle Bezeichnung für die bei eukaryontischen Organismen aus dem Zellkern einer Zelle stammenden DNA.
- cDNA: Abkürzung für complementary DNA. Bezeichnung für die einzel- bzw. doppelsträngige DNA-Kopie eines RNA-Moleküls.
- cDNA-Bank/Bibliothek: Bezeichung für eine Sammlung von willkürlich klonierten cDNA-Fragmenten, die zusammengenommen die Gesamtheit aller von einer Zelle oder einem Gewebe synthetisierten RNA repäsentieren.
- cDNA-Klon: Bezeichnung für eine Population genetisch einheitlicher Zellen, die sich von einer einzigen Zelle ableiten, derart, daß diese Zelle eine künstlich eingebrachtes cDNA-Fragment enthält.
- Hybridisierung: Durch Basenpaarung bewirkte Ausbildung eines doppelsträngigen Nukleinsäuremoleküls aus zwei getrennten Einzelsträngen.
- stringente Bedingungen: Bedingungen, unter denen nur perfekt basengepaarte Nukleinsäure-Stränge gebildet werden und stabil bleiben.
- isolieren: ein gesuchtes Molekül aus einem Gemisch herausfinden und abtrennen.
- DNA-Seguenzierung: Bestimmung der Abfolge der von Basen in einem DNA-Molekül.
- Nukleinsäuresequenz: Bezeichnung für die Primärstruktur eines DNA-Moleküls, d.h. die Abfolge der einzelnen Basen, aus denen sich eine DNA zusammensetzt.
- Genspezifische Oligonukleotid Primer: Oligonukleinsäuren, also 10-40 Basen lange Nukleinsäurefragmente, die in ihrer Basenzusammensetzung eine stringente Hybridisierung an das gesuchte Gen oder die gesuchte cDNA erlauben.
- Ermitteln von Oligonukleotid Primern: Die manuelle oder Computerunterstützte Suche von Oligonukleotiden zu einer vorgegebenen DNA-Sequenz, die für eine Hybridisierung und/oder eine Polymerase-Ketten Reaktion optimal geeignet sind.
- PCR: Abkürzung für Polymerase-Kettenreaktion. In vitro-Verfahren zur selektiven Anreicherung von Nucleinsäure-Bereichen definierter Länge und definierter Sequenz aus einem Gemisch von NukleinsäureMolekülen.
- DNA-Template: Nukleinsäuremolekül oder ein Gemisch von Nukleinsäuremolekülen, aus denen ein DNA-Abschnitt mit Hilfe der PCR (s.o.) vervielfältigt wird.
- RNA: International gebräuchliche Abkürzung für Ribonukleinsäuren
- mRNA: International gebräuchliche Abkürzung für messenger-Ribonukleinsäuren, die am Transfer der genetischen Information aus dem Kern in die Zelle beteiligt sind und die Information für die Synthese eines Polypetids oder eines Proteins beinhalten.
- Antisense Polynukleotid: Eine aus mehreren natürlichen oder modifizierten Nukleinsäuren bestehendes Molekül, deren Basenabfolge komplementär zur Basenabfolge eines Teilbereiches einer in der Natur vorkommenden RNA ist.
- PNA: International gebräuchliche Abkürzung für peptidic Nucleic Acids. Hierbei bilden peptidisch verknüpfte Aminosäuren eine Kette, wobei die Aminosäuren als Seitenkette eine für die Hybridisierung mit DNA oder RNA fähigen Base trägt.
- Sequenz: Abfolge von Nukleotiden oder Aminosäuren. Im spezifischen Sinne dieser Erfindung ist damit die Nukleinsäuresequenz gemeint.
- Ribozym: Bezeichnung für eine katalytisch aktive Ribonukleinsäure (z.B. Ligase, Endonuklease, Polymerase, Exonuklease)
- DNA-Enzym: Bezeichnung für ein DNA-Molekül, das katalytische Aktivität beinhaltet (z.B. Ligase, Endonuklease, Polymerase, Exonuklease)
- katalytische RNA/DNA: generelle Bezeichnung für Ribozyme bzw. DNA-Enzyme (s.o.).
- Adenovirus: bei Vertebraten vorkommender cytopathogener Virus
- Adenoassozüertes Virus (AAV): Gehört zur Familie der Parvoviren. Für eine effektive Vermehrung des AAV ist eine Coinfektion der Wirtszellen mit Helferviren (z.B. Herpes-, Vaccina- oder Adenoviren) erforderlich. Die Eigenschaft von AAV, stabil in das Wirtsgenom zu integrieren, macht es als Transduktionsvektor für Säugetierzellen besonders interessant.
- Herpesvirus: Viraler Erreger der Herpes-Infektion
- posttranslationale Modifikation: Veränderung an Proteinen oder Polypetiden, die nach der Translation durchgeführt wird, hierzu zählen z.B. Phosphorylierung, Glykosylierung, Amidierung, Acetylierung oder Proteolyse.
- glykosylieren: Bezeichnung für das Anhängen von einzelnen Zuckermolekülen oder ganzen Zuckerketten an Proteine.
- phosphorylieren: Bezeichnung für das Anhängen von einem oder mehreren Phosphatresten an ein Protein, bevorzugt an die OH-Gruppen der Aminosäuren Serin, Threonin oder Tyrosin.
- amidieren. Die Bezeichnung für das Umwandeln einer Carboxylfunktion in eine Amidfunktion, z.B. an den carboxyterminalen Aminosäurerest eines Peptides oder Proteins.
- mit Membrananker versehen: Posttranslationelle Modifikation eines Proteins, oder eines anderen organischen Moleküls, derart daß es durch Anhängen eines hydrophoben Moleküls, geeigneterweise eine Fettsäure oder ein Derivat derselben, an die Lipiddoppelschicht-Membran von Zellen verankert wird.
- spalten: in diesem spezifischen Fall die Spaltung eines Peptids oder Proteins in mehrere Untersequenzen.
- verkürzen: Ein aus mehreren Einzelteilen bestehendes Molekül um eine oder mehrere Teile zu verkürzen.
- Antikörper: Lösliche, oder an Zellmembranen gebundene, als Immunglobuline bezeichnete Proteine mit einer spezifischen Bindungsstelle für Antigene.
- monoklonaler Antikörper: sind gegen eine einzige antigene Determinante eines Antigens gerichtete Antikörper mit extrem hoher Selektivität.
- polyklonaler Antikörper: Gemisch aus Antikörpern, die gegen mehrere Determinanten eines Antigens gerichtet sind.
- transgen: genetisch verändert.
- nichthumanes Säugetier: Die Gesamtheit der Säugetiere (Klasse der Mammalia) mit Ausnahme der Spezies Mensch.
- Keim-Zelle: Zelle mit haploidem Genom, die durch Verschmelzung mit einer zweiten Keimzelle die Bildung eines neuen Organismus ermöglicht.
- somatische Zelle: diploide Zelle als Bestandteil eines Organismus.
- chromosomale Einbringung: Eingriff in die Nukleotidsequenz auf chromosomaler Ebene.
- Genom: Allgemeine Beschreibung für die Gesamtheit aller Gene in einem Organismus.
- Vorfahr des Tieres: Ein Tier (der Vorfahr), das auf natürliche oder künstliche Weise durch Weitergabe an seinem genetischen Material in direkter Linie mit einem anderen Tier (dem Nachfahren) verwandt ist.
- exprimierbar: Ein Nukleinsäuremolekül ist dann exprimierbar, wenn es die Information zur Synthese eines Proteins oder Polypetids beinhaltet und mit ensprechenden regulatorischen Sequenzen versehen ist, die eine Synthese dieses Proteins oder Polypeptids in vitro oder in vivo erlauben. Wenn diese Voraussetzungen nicht mehr gegeben sind, beispielsweise durch Eingriff in der kodierenden Sequenz, ist das Nukleinsäuremolekül nicht mehr exprimierbar.
- Nagetier: Tier aus der Ordnung der Rodentia, z.B. Ratte oder Maus.
- als schmerzregulierende Substanz identifizierbar. Substanz die bei Einbringung in einen lebenden Organismus eine Verhaltensänderung bewirkt, die der Fachmann als schmerzhemmend bezeichnet (antinozizeptiv, antihyperalgetisch oder antiallodynisch). Im Falle des Screeningverfahrens bezieht sich das darauf, daß die Substanz beim Screening durch stärkere Bindung oder Auslösung einer Änderung eines funktionellen Parameters deutlich, beispielsweise 100 %, die Bindung oder Wechselwirkung des Durchschnitts der getesteten Substanzen übertrifft.
- Verbindung: ein anderer Name für Molekül, als aus mehreren Atomen bestehend, hier ein durch das erfindungsgemäße Verfahren identifiziertes Molekül.
- Wirkstoff: Eine Verbindung, die bei Anwendung an einem Organismus eine Veränderung in diesem Organismus hervorruft. Im Besonderen werden darunter organisch-chemisch synthetisierte Moleküle verstanden, die auf den Organismus eine heilende Wirkung ausüben. Hier insbesondere Moleküle, die an die erfindungsgemäßen Proteine und Peptide binden.
- niedermolekular: Molekül mit einem Molekulargewicht < 2kDa.
- Arzneimittel: ein Stoff entsprechend der Definition im Artikel 1 §2 des Gesetzes über den Verkehr mit Arzneimitteln.
- Diagnostikum: Verbindung oder Verfahren, das verwendet werden kann, um eine Krankheit zu diagnostizieren.
- Behandlung von Schmerz: Verfahren, mit dem Ziel Schmerzen zu lindem oder aufzuheben, oder das zu erwartende Auftreten von Schmerzen zu hemmen (preemptive Analgesie).
- chronischer Schmerz: eine Schmerzempfindung von länger anhaltender Dauer, oft dadurch gekennzeichnet, daß sie über Zeitpunkt und Ort des initialen Stimulus hinausreicht, die Schmerzempfindlichkeit des Körpers steigert.
- Gentherapie: Unter Gentherapie versteht man alle Verfahren, die das Ziel haben, genetische Erkrankungen durch geeignete Veränderungen des Genoms kausal zu behandeln.
- In-vivo-Gentherapie: Einbringen von genetischem Material in den lebenden Organismus mit dem Ziel der Gentherapie. Man kann zwischen somatischem und Keimbahn-Eingriff unterscheiden, der einmal an diploiden Zellen und einmal an haploiden Zellen stattfindet.
- In-vitro-Gentherapie: Einbringen von genetischem Material in Zellen außerhalb des menschlichen Körpers mit dem Ziel diese nachher wieder durch Einbringen in den menschlichen Körper zur Gentherapie zur verwenden.
- Diagnostik: Verfahren, um eine Krankheit zu identifizieren.
- Wirksamkeitsuntersuchung: Untersuchung mit dem Ziel die Wirksamkeit einer Verbindung nach Einwirkung auf einen lebenden Organismus zu untersuchen.

In einer bevorzugten Ausführungsform des Verfahrens wird die Zelle vor dem Schritt (a) gentechnisch manipuliert. Dabei wird genetisches Material in die Zelle eingebracht, insbesondere eine oder mehrere Polynukleotidsequenzen. In einer weiter bevorzugten Variante dieser Ausführungsform erlaubt die gentechnische Manipulation die Messung mindestens eines der durch die Testsubstanz veränderten funktionellen Parameter. In dieser Ausführungsform werden durch gentechnische Manipulation Voraussetzungen geschaffen unter denen die Veränderung eines funktionellen Parameters überhaupt oder verbessert gemessen werden kann. Dabei ist es insbesondere bevorzugt, daß durch die gentechnische Manipulion eine in der Zelle nicht endogen exprimierte Form eines G-Proteins exprimiert oder ein Reportergen eingeführt wird. Darunter ist insbesondere die gentechnische Einführung eines endogen nicht vorhandenen oder physiologisch nicht exprimierten G-Proteins (GTPbindenden Proteins) in die Zelle zu verstehen, beispielsweise die Einführung eines chimären G-Proteins, das eine Veränderung des Signalweges erlaubt oder eines promiskuitiven G-Proteins, das sehr bindungsfreudig ist Die Einführung eines Reportergens wiederum erlaubt die Messung einer (extrazellulär ausgelösten) induzierten Expression des Genproduktes.

In einer weiteren bevorzugten Ausführungsform wird die Zelle gentechnisch so manipuliert wird, daß die Zelle mindestens ein Polynukleotid gemäß einer der Abbildungen 1a), 1c), 1e), 1g) oder ein dazu zu mindestens 90 %, vorzugsweise zu mindestens 95 %, insbesondere zu mindestens 97 % ähnliches Polynukleotid enthält. Damit kann beispielsweise erreicht werden, daß ein Protein, das in der im Verfahren verwendeten Zelle oder Präparation nicht endogen exprimiert wird, von der Zelle synthetisiert wird. Dabei ist es insbesondere bevorzugt, wenn das Polynukleotid in einem rekombinanten DNA-Konstrukt enthalten ist. Unter einem (rekombinanten) DNA-Konstrukt versteht man ein in-vitro hergestelltes DNA-Molekül.

Wenn beim Verfahren vor dem Schritt a) die Zelle gentechnisch manipuliert wird, ist es bevorzugt, daß die Zelle nach der gentechnischen Manipulation und vor dem Schritt (a) unter Bedingungen, die eine Expression erlauben, kultiviert wird, gegebenenfalls unter Selektionsdruck. Unter kultivieren versteht man, Zellen oder Gewebe bei Bedingungen, die ein Überleben der Zellen, bzw. deren Plachfolgegeneration sichern, zu halten. Dabei sollten die Bedingungen hier so gewählt werden, daß eine Expression des durch die gentechnische Manipulation eingefügten Materials ermöglicht wird. Dazu sollten pH, Sauerstoffgehalt, und Temperatur physiologisch gehalten sein und ausreichend Nährstoffe und notwendige Cofaktoren beigefügt sein. Der Selektionsdruck erlaubt, nur die Zellen weiter zu kultivieren, bei denen die gentechnische Manipulation zumindest teilweise erfolgreich war. Dazu gehört beispielsweise die Einführung einer Antibiotikaresistenz über das DNA-Konstrukt.

Es ist bei dem erfindungsgemäßen Verfahren besonders bevorzugt, wenn die verwendete Zelle eine Amphibienzelle, Bakterienzelle, Hefezelle, Insektenzelle oder eine immortalisierte oder native Säugetierzelle ist Beispiele für Amphibienzellen sind Xenopus Oocyten, für Bakterienzellen E-coli-Zellen, für Hefezellen auch Saccharomyces cerevisiae, für Insektenzellen Sf9-Zellen, für immortalisierte Säugetierzelle HeLa-Zellen und für native Säugetierzellen die CHO (Chinese Hamster Ovary)-Zelle.

Bei einer verwendeten Meßmethode zur Feststellung der Bindung der Substanz an das Biomolekül oder Protein im erfindungsgemäßen Verfahren erfolgt die Messung der Bindung über die Verdrängung eines bekannten markierten Liganden des Biomoleküls, bzw. des Proteins und/oder über die daran gebundene Aktivität einer markierten Testsubstanz. Dabei ist ein Ligand ein mit hoher Spezifität an das Protein bindendes Molekül, das durch eine ebenfalls bindende, zu testende Substanz aus der Bindungsstelle verdrängt wird. Unter Markierung ist eine den Nachweis erleichternde künstliche Modifikation am Molekül zu verstehen. Beispiele sind radioaktive, fluoreszierende oder lumineszierende Markierung.

Bei einer anderen verwendeten Meßmethode zur Feststellung der durch die Bindung der Substanz an Protein im erfindungsgemäßen Verfahren ausgelösten Veränderung der funktionellen Parameter, erfolgt die Messung mindestens eines der durch die Testsubstanz veränderten funktionellen Parameter über Messung der Regulation, Hemmung und/oder Aktivierung von Rezeptoren, lonenkanälen und/oder Enzymen, insbesondere Ober Messung der Veränderung der Genexpression, des Ionenmilieus, des pH oder des Membranpotentials, über Veränderung der Enzymaktivität oder der Konzentration der 2^{nd} messenger. Damit ist auf der einen Seite direkt die Messung der Wirkung der Substanz über die Beeinflußung von Rezeptoren, lonenkanälen und/oder Enzymen erfaßt, auf der anderen Seite als bevorzugt zu messende Beispiele sich ändernder Parameter wie Genexpression, lonenmilleu, pH, Membranpotential, Enzymaktivität oder Konzentration der 2^{nd} messenger. Dabei versteht man unter lonenmilieu insbesondere die Konzentration eines oder mehrer Ionen in einem Zellkompartiment, insbesondere dem Cytosol, unter Membranpoten6al die Ladungsdiffferenz zwischen zwei Seiten einer Biomembran und unter 2^{nd} messenger Botenstoffe des intrazellulären Signalwegs wie z.B. zyklisches AMP (cAMP), Inosototthphosphat (IP3) oder Diacylglycerol (DAG).

Ein besonders bevorzugter Gegenstand der Erfindung ist ein erfindungsgemäßes Verfahren, bei dem ein erstes erfindungsgemäßes Verfahren mit einem zweiten erfindungsgemäßen Verfahren derart gekoppelt wird, daß die Meßwerte und Ergebnisse des ersten Verfahrens hinsichtlich der zu messenden Substanz mit den Meßwerten und Ergebnissen des zweiten Verfahrens hinsichtlich der zu messenden Substanz verglichen werden, dadurch gekennzeichnet, daß in einem der zwei Verfahren, im folgenden Hauptverfahren genannt, im Schritt (a) die zu testende Substanz
mit einem Biomolekül ausgewählt aus Gruppe II:
dem Protein BNPI und/oder einem Protein gemäß einer der Abbildungen 1b), 1d), 1f) oder 1h) und/oder einem zu einem dieser vorgenannten Proteine zu mindestens 90 % ähnlichen Protein und/oder einem Protein, für das ein Polynukleotid gemäß einer der Abbildungen 1a), 1c), 1e) oder 1g) oder ein dazu zu mindestens 90 % ähnlichen Polynukleotid kodiert, und/oder einem Protein, das durch eine Nukleinsäure kodiert wird, die unter stringenten Bedingungen an ein Polynukleotid gemäß einer der Abbildungen 1a), 1c), 1e) oder 1g) oder deren Antisense Polynukleotide bindet, und/oder einer Zelle und/oder einer Präparation aus einer solchen Zelle, die mindestens eines der vorgenannten Proteine und Teilproteine, bzw. Biomoleküle aus Gruppe II, synthetisiert hat,
und
daß im anderen der zwei Verfahren, im folgenden Nebenverfahren genannt, im Schritt (a) die zu testende Substanz mit einem Biomolekül aus der Gruppe I oder' mit einem Biomolekül aus Gruppe II inkubiert wird, aus der das Biomolekül, mit der die Substanz im Hauptverfahren inkubiert wird, nicht ausgewählt ist.

Unter dieser besonders bevorzugten Ausführungsform ist insbesondere die Kombination der Messung der Bindung an BNPI oder davon abgeleiteten Biomolekülen oder der Messung der daraus entstehenden Modifikation zellulärer Parameter zu verstehen, da gerade ein Vergleich angesichts der vollkommen getrennten aber eng aneinanderliegenden Verteilung der beiden Kanäle im Gewebe einen wichtigen Aufschluß über physiologische Funktionen geben kann. Damit erlaubt aber der differentielle Abgleich der Daten die Identifizierung optimiert pharmazeutisch bzw. medizinisch wirksamer Substanzen.

Durch ein erfindungsgemässes Screeningverfahren kann eine Verbindung identifiziert werden als pharmazeutisch relevante Substanz mit Wirksamkeit in mindestens einer der vorgenannten Indikationen. Hierbei bezieht sich Verbindung insbesondere auf niedermolekulare Wirkstoffe, aber auch auf Peptide, Proteine und Nukleinsäuren. Dabei bedeutet identifizierbar, daß die Verbindung das Merkmal aufweist, daß es beim erfindungsgemäßen Screeningverfahren bezüglich der Bindung deutlich stärker, vorzugsweise doppelt so stark bindet wie der Durchschnitt der zu testenden Substanzen oder bezüglich der Änderung der funktionellen Parameter deutlich vom Durchschnitt der zu testenden Substanzen abweicht. Besonders bevorzugt ist es, wenn die durch ein erfindungsgemäßes Verfahren identifizierte Verbindung eine niedermolekulare Verbindung ist.

Mit dem im erfindungsgemäßen Verfahren eingesetzten Polynukleotid sind auch die dargestellten Genfragmente selbst umfaßt, wie auch ein Polynukleotid, das entweder vollständig oder zumindest Teilen der kodierenden Sequenz des dem Fragment entsprechenden Gens entspricht. Damit sind auch Polynukleotide gemeint, die mindestens 90%-ige, vorzugsweise 95%-ige, insbesondere wenigstens 97%-ige Übereinstimmung in der Basenabfolge mit der kodierenden Sequenz der abgebildeten Polynukleotide oder der kodierenden Sequenz der Gens aufweisen. Es ist weiter bevorzugt, daß es sich bei dem Polynukleotid um RNA bzw. ein- oder doppelstängige DNA, insbesondere mRNA oder cDNA handelt. Ebenso ist es bevorzugt, daß es sich bei dem Polynukleotid um ein Antisense-Polynukleotid oder PNA handelt, das eine Sequenz aufweist, die in der Lage ist, spezifisch an ein erfindungsgemäßes Polynukleotid zu binden. Dabei versteht man unter PNA "peptidic nucleic acid" (peptidische Nukleinsäure), die zwar die Basenpaare trägt, aber dessen Rückrat peptidisch gebunden ist. Ein Antisense-Polynukleotid zeigt die komplementäre Basenabfolge zu mindestens einem Teil einer Basis-Nukleinsäure. Ebenfalls bevorzugt ist es, daß das Polynukleotid Teil eines Ribozym oder sonstigen DNA-Enzyms oder einer katalytischen RNA bzw. DNA ist. Unter Ribozym ist eine katalytisch aktive Ribonukleinsäure zu verstehen, unter DNA-Enzym ein entsprechende Desoxyribonukleinsäure, also katalytische RNA beziehungsweise DNA.

Ebenfalls eingesetzt werden kann ein Polynukleotid oder auch Oligonukleotid, bei dem mindestens eines der Nukleotide, insbesondere mehrere der Nukleotide, "Locked Nucleic Acids" ("LNA's") sind oder mindestens eines der Nukleotide, insbesondere alle Nukleotide, Phosphorothioate sind, vorzugsweise ein solches, bei dem mehrere der Nukleotide "Locked Nucleic Acids" ("LNA's") sind. "Locked nucleic acids" ("LNA's") sind Ribonukleotide, die eine Methylen-Brücke enthalten, die den 2'-Sauerstoff der Ribose mit dem 4'-Kohlenstoff verbindet (s. Abb. 27). Einen Überblick über die LNA's geben Braasch D.A. und Corey, D.R. (2001), Locked nucleic acids (LNA); fine-tuning the recognition of DNA und RNA. Chem. Biol. 8, 1-7. LNA's werden beispielsweise von der Firma Proligo, Boulder, CO, USA angeboten. Auch Phosphorothiate sind dem Fachmann bekannt und können beispielsweise bei MWG-Biotech AG, Ebersberg, Germany bestellt werden.

Bevorzugt ist es für ein im erfindungsgemäßen Verfahren eingesetztes Protein bzw. ein daraus abgeleitetes Teilprotein, wenn dieses posttranslational modifiziert wurde, es insbesondere glykosiliert, phosphoryliert, amidiert, methyliert, acetyliert, ADP-ribosyliert, hydroxyliert, mit einem Membrananker versehen, gespalten oder verkürzt wurde. Posttranslationale Modifikationen sind beispielsweise dem Voet/Voet, Biochemistry, 1^{st} Edition, 1990, S. 935-938 zu entnehmen.

Dabei kann das eingesetzte Polynukleotid (gegebenfalls gemäß Punkt a) und/oder Punkt b)) eine RNA oder eine ein- oder doppelstängige DNA, insbesondere mRNA oder cDNA sein.

Dabei kann das eingesetzte Polynukleotid (gegebenfalls gemäß Punkt b)) Teil eines Ribozyms oder sonstigen DNA-Enzyms oder einer katalytischen RNA oder DNA sein.

Die folgenden Beispiele und Abbildungen sollen die Erfindung erläutern, ohne sie darauf zu beschränken.

### Abbildungen und Beispiele

### Abbildungen:

- Fig. 1a): cDNA-Sequenz von BNPI, human; AN: NM_020309
- Fig. 1b): Aminosäure-Sequenz von BNPI, human; AN: NM_020309
- Fig. 1c): cDNA-Sequenz von BNPI, human; Nr.: AAT42064 aus WO96/34288
- Fig. 1d): Aminosäure-Sequenz von BNPI, human; Nr.: AAT42064 aus WO96/34288
- Fig. 1e): cDNA-Sequenz von BNPI, Ratte; AN: U07609
- Fig. 1f): Aminosäure-Sequenz von BNPI, Ratte; AN: U07609
- Fig. 1g): cDNA-Sequenz von BNPI, Maus; AN: XM_133432
- Fig. 1h): Aminosäure-Sequenz von BNPI, Maus; AN: XM_133432

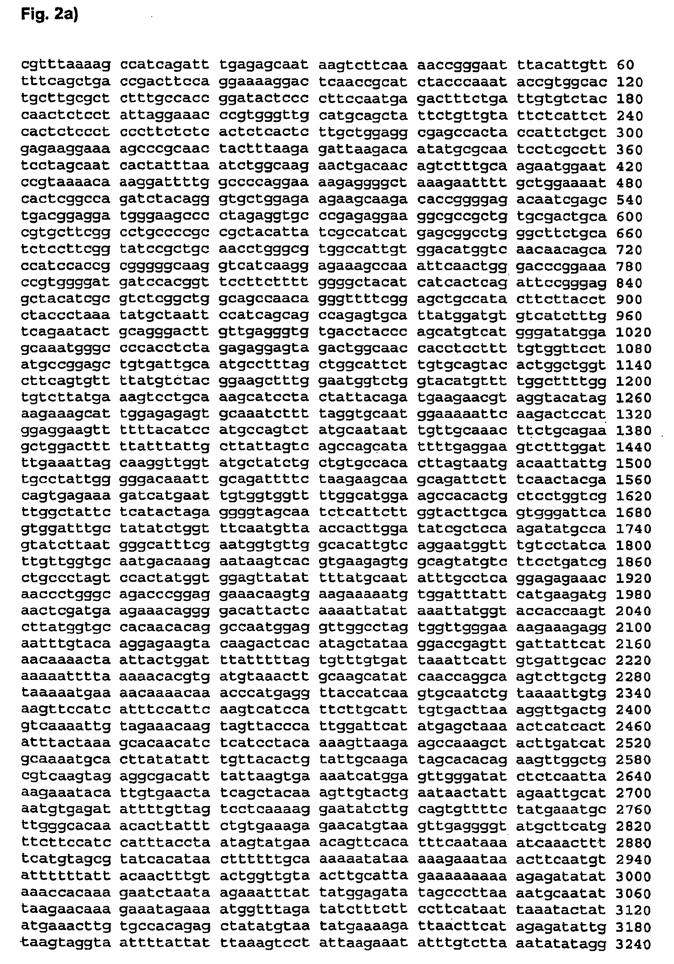

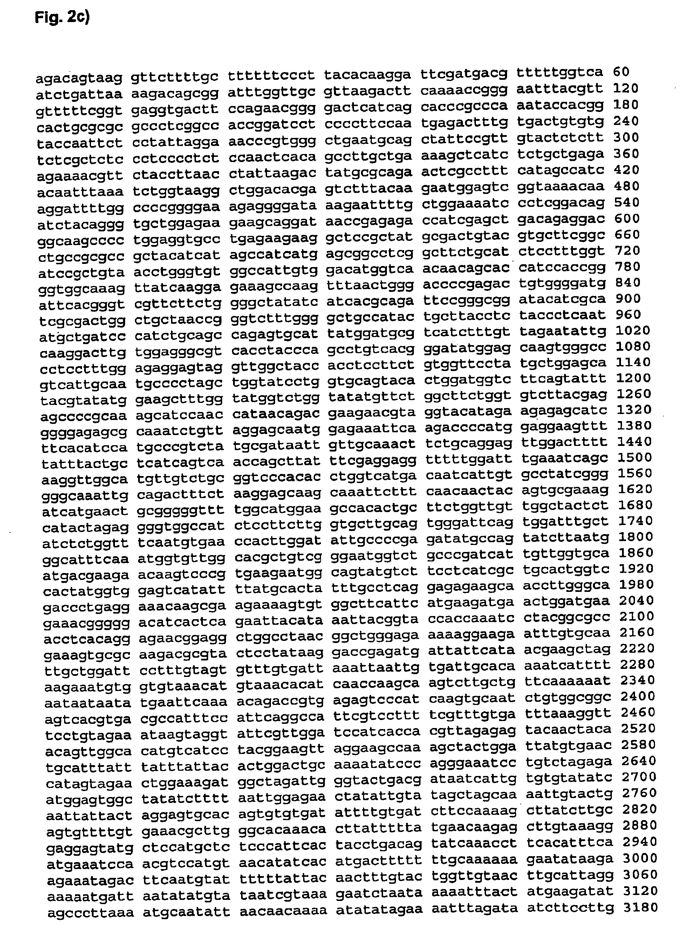

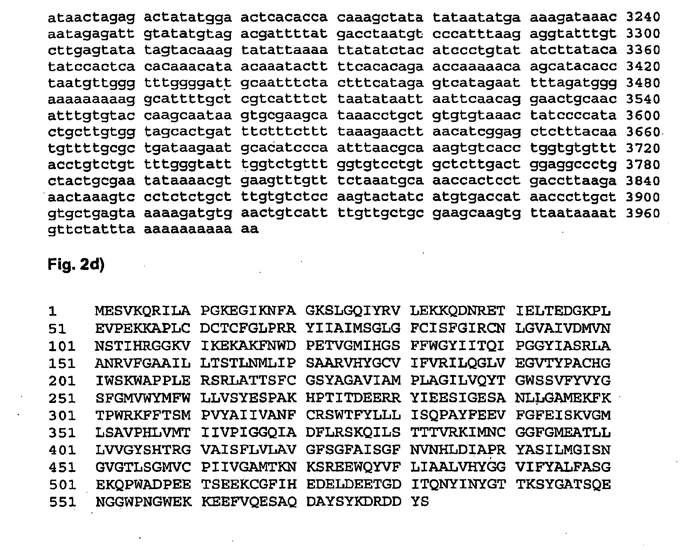

- Fig. 2a): cDNA-Sequenz von DNPI, human; AN: AB032435

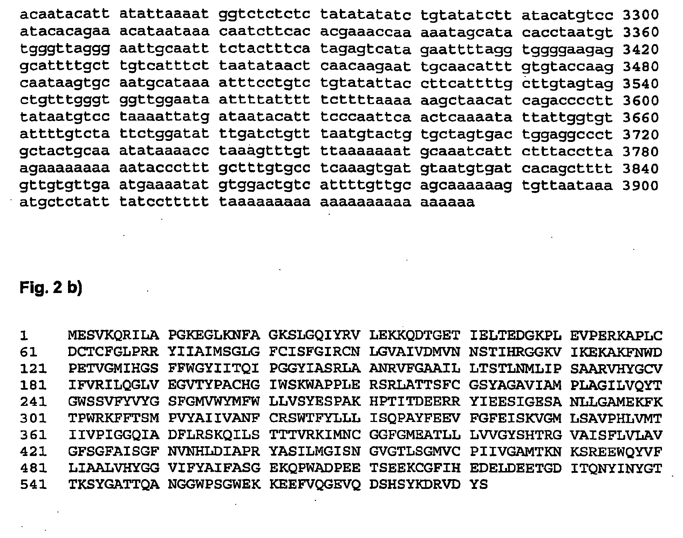

- Fig. 2b): Aminosäure-Sequenz von DNPI, human; AN: AB032435
- Fig. 2c): cDNA-Sequenz von DNPI, Ratte; AN: AF271235
- Fig. 2d): Aminosäure-Sequenz von DNPI, Ratte; AN: AF271235

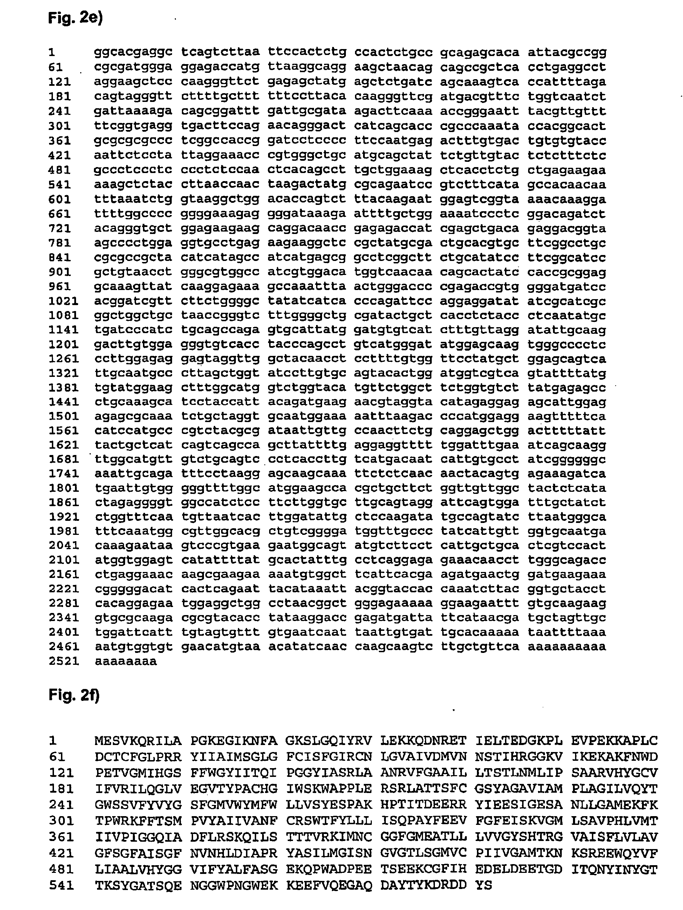

- Fig. 2e): cDNA-Sequenz von DNPI, Maus; AN: NM_080853
- Fig. 2f): Aminosäure-Sequenz von DNPI, Maus;AN: NM_080853
- Fig. 3): Differentielle Expression von DNPI und BNPI in Synapsen und motorischen Arealen des lumbalen Rückenmarks der Ratte (s. Beispiel 2a)
- Fig. 4): Differentielle Expression von DNPI und BNPI in Synapsen der Dorsalhom-Arealen des lumbaren Rückenmarks der Ratte (s. Beispiel 2b)
- Fig. 5): Differentielle Expression von DNPI und BNPI in Synapsen des sakralen Rückenmarks der Ratte (s. Beispiel 2c)
- Fig. 6): Differentielle Expression von DNPI und BNPI in Synapsen der medullo-cervicospinalen Leitung des Trigeminalnervs der Ratte (s. Beispiel 2d)
- Fig. 7): Differentielle Expression von DNPI und BNPI in Hinregionen der Ratte (s. Beispiel 2e)
- Fig. 8): Differentielle Expression von DNPI und BNPI in Hinregionen der Ratte (s. Beispiel 2f)
- Fig. 9): Differentielle Expression von DNPI und BNPI in Hinregionen der Ratte (s. Beispiel 2g)
- Fig. 10): Differentielle Expression von DNPI und BNPI in Hinregionen der Ratte (s. Beispiel 2h)
- Fig. 11): Differentielle Expression von DNPI und BNPI in Hinregionen der Ratte (s. Beispiel 2i)
- Fig. 12): Differentielle Expression von DNPI und BNPI in Hinregionen der Ratte (s. Beispiel 2j)
- Fig. 13): Differentielle Expression von DNPI und BNPI in Hinregionen der Ratte (s. Beispiel 2k)
- Fig. 14): Differentielle Expression von DNPI und BNPI in Hinregionen der Ratte (s. Beispiel 21)
- Fig. 15): Differentielle Expression von DNPI und BNPI in Hinregionen der Ratte (s. Beispiel 2m)
- Fig. 16): Differentielle Expression von DNPI und BNPI in Hinregionen der Ratte (s. Beispiel 2n)
- Fig. 17): Differentielle Expression von DNPI und BNPI in Hinregionen der Ratte (s. Beispiel 20)
- Fig. 18): Differentielle Expression von DNPI und BNPI in Hinregionen der Ratte (s. Beispiel 2p)
- Fig. 19): Differentielle Expression von DNPI und BNPI in Hinregionen der Ratte (s. Beispiel 2q)

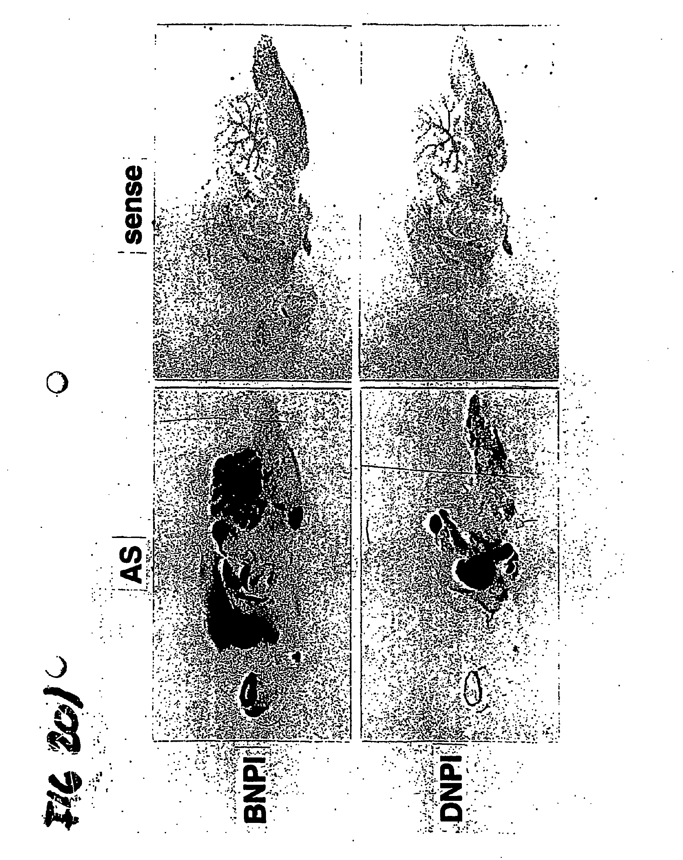

- Fig. 20): Differentielle Expression von DNPI und BNPI in Hinregionen der Ratte, dabei heißt AS Anti-sense und bezieht sich auf die Färbung.

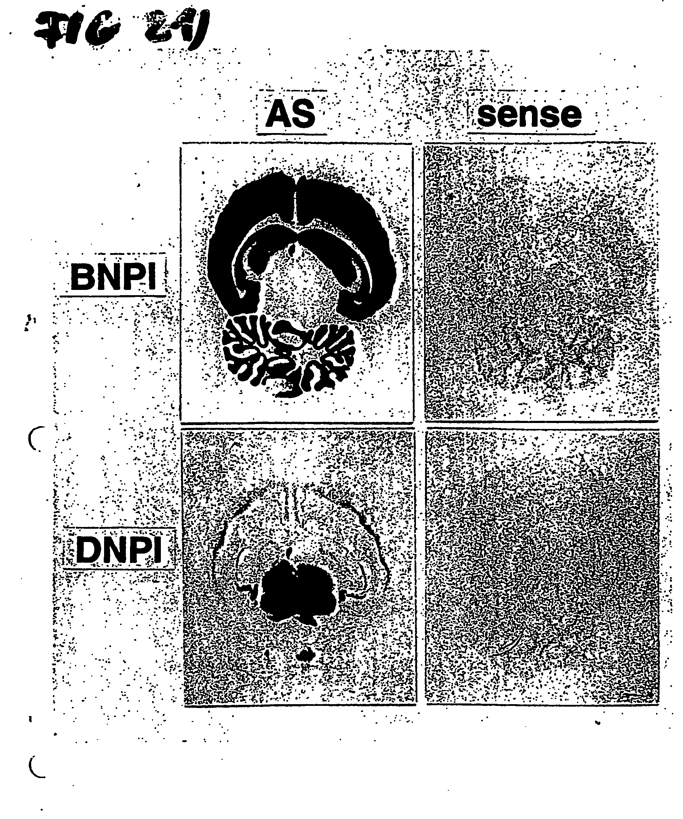

- Fig. 21): Differentielle Expression von DNPI und BNPI in Hinregionen der Ratte, dabei heißt AS Anti-sense und bezieht sich auf die Färbung.

### Beispiele:

### Beispiel 1

### Differentielle Betrachtung der Expression zwischen DNPI und BNPI über immuncytochemische Färbung

Zur immunhistochemischen Anfärbung wurden polyklonale Kanichen-Antiseren gegen das rekombinante DNPI- bzw. BNPI-Fusionsprotein verwendet. Generell wurden Schnitte verschiedener Regionen des ZNS angelegt und die Expression von DNPI mit der von BNPI verglichen. Das Vorgehen entsprach bezüglich der Schnitte und der Färbung dem bei Persson S., Schäfer MK-H., Nohr D., Ekström G., Post C., Nyberg F. und Weihe E. (1994), Neuroscience 63; 313-326 bzw. Nohr D., Schäfer MK-H., Romeo H., Persson S., Nyberg F. Post C. und Weihe E. (1999), Neuroscience 93; 759-773 beschriebenen Verfahren, wobei die Offenbarung, Beschreibung bzw. technische Lehre dieser Artikel ausdrücklich zum Teil der hier vorgelegten Offenbarung, Beschreibung bzw. technischen Lehre dieser Erfindung gemacht wird.

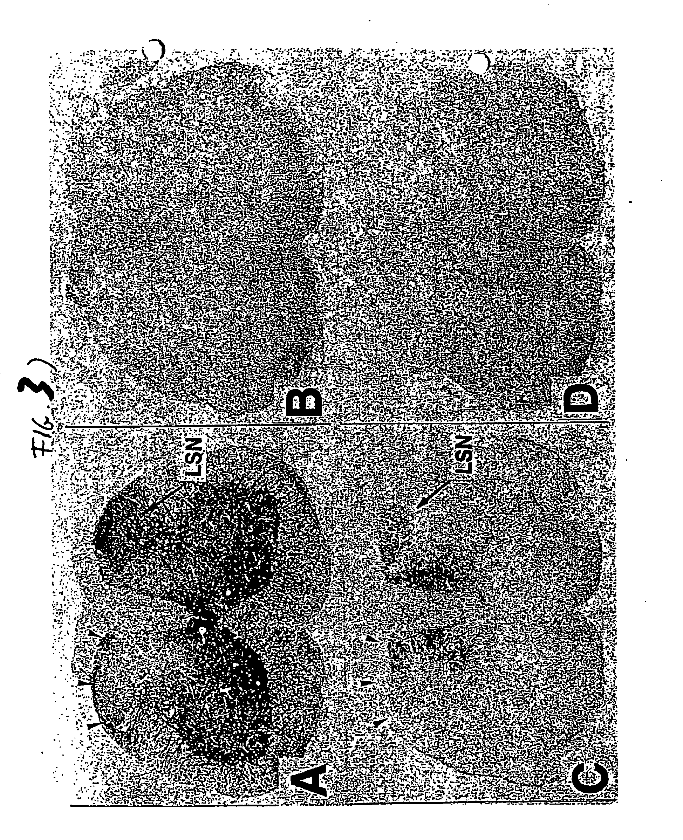

### Beispiel 2a zu Abbildung 3)

Es ist die differentielle Verteilung der Immunreaktivität von BNPI und DNPI im lumbaren Rückenmark der Ratte zu sehen. Die angrenzenden deparafinierten Abschnitte A- bis D sind wie folgt gefärbt:
A = anti-DNPI;
B = anti-DNPI präadsorbiert mit DNPI-Fusionsprotein;
C = anti-BNPI;
D = anti-BNPI präadsorbiert mit BNPI-Fusionsprotein;

Die DNPI- (A) und BNPI- (C) Immunfarbstoffe waren voll mit homologem rekombinanten BNPI- (D) und BNPI- (B) Fusionsprotein präadsorbierbar, was die Spezifität der Immunreaktion beweist.

Bemerkenswert ist das gegenseitig ausschließende Verteilungsmuster von DNPI und BNPI-Immunfärbung im äußeren und tiefen Dorsalhorn. (A;C).Punktierte Immunfärbung von DNPI ist in den synaptischen Emndungen des äußeren Dorsalhoms (Lamina 1 und Substantia gelatinosaa) (Pfeil in A), während BNPI Immunreaktivität vollständig fehlt (Pfeile in B). Akkumulation von starker positiver punktierter BNPI-Immunfärbung liegt im tieferen Dorsalhom vor, während DNPI-Färbung relativ niedrig ist. DNPI ist präsent in der lateralen spinalen Nukleus (LSN in A), während BNPI völlig fehlt (LSN in C). DNPI ist in der Lamina X um den zentralen Kanal abundant, während BNPI selten ist. BNPI Immunfärbung ist im lateralen Ventralhom schwach und gering oder fehlend im medialen Ventralhorn. Durch das ganze Ventralhorn ist punktförmige DNPI-Färbung abundant, etwas weniger im lateralen Horn im Vergleich zum medialen Ventralhorn. Es gibt eine schwache BNPI und DNPI Färbung in einigen Zellkörpem der im Ventralhorn gelegenen Motoneurone, was aber nicht durch die homologen transporter Fusionsproteine präadsorbiert wurde und daher als nichtspezifisch eingestuft wurde.

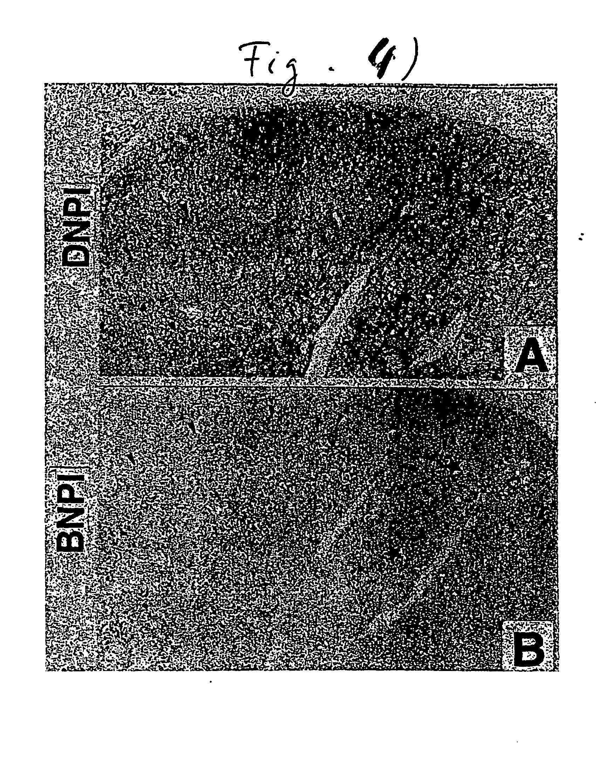

### Beispiel 2b zu Abbildung 4)

Es ist die differentielle Verteilung der Immunreaktivität von BNPI und DNPI im linken lateralen oberflächlichen dorsalen lumbaren Rückenmark (left lateral superficial dorsal lumbar spinal cord) der Ratte zu sehen. A und B jeweils für BNPI (A) und DNPI (B) gefärbt zeigen viele punktförmige Anfärbungen für DNPI, die in der Lamina I und substantia gelatinosa konzentriert sind wo BNPI fast vollständig fehlt. Weiter sind dichte Komplexe von DNPI positiven Punkten im lateralen spinalen Nukleus zu sehen, wo BNPI fast vollständig fehlt. Feine DNPI positive Punkte sind auch in den tieferen dorsalen Horn zu finden, wenn auch mit geringerer Dichte.

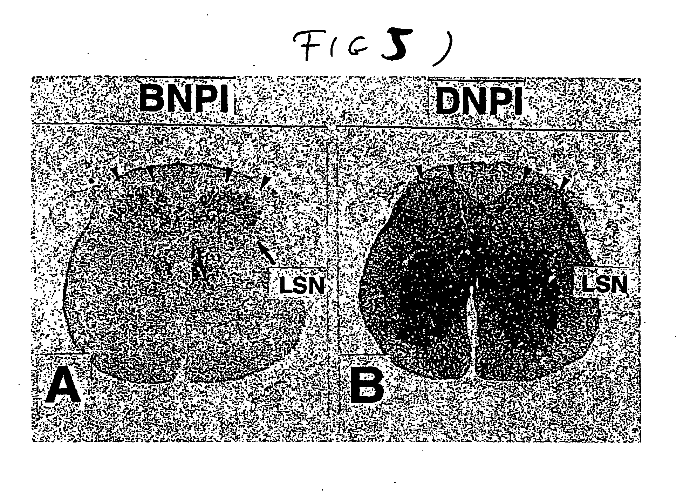

### Beispiel 2c zu Abbildung 5)

Es ist die differentielle Verteilung der Immunreaktivität von BNPI und DNPI im sakralen Rückenmark der Ratte zu sehen. Die angrenzenden Abschnitte A und B jeweils für BNPI (A) und DNPI (B) gefärbt, zeigen gegenseitige Exclusions-Zonen punktierter DNPI- und BNPI-Immunfärbung im Dorsalhom. DNPI ist in der gesamten Grey Matter präsent und ist in den sehr äußeren Schichten des Dorsalhoms konzentriert, wo es es eine schmale Bande an der Grenze zu White Matter bildet DNPI ist abundant im lateralen spinalen Nukleus und in der Lamina X wie auch in der Lamina V/VI und im ganzen ventralen Horn. BNPI ist abundant im tiefen Dorsalhorn und selten in Ventralhorn.

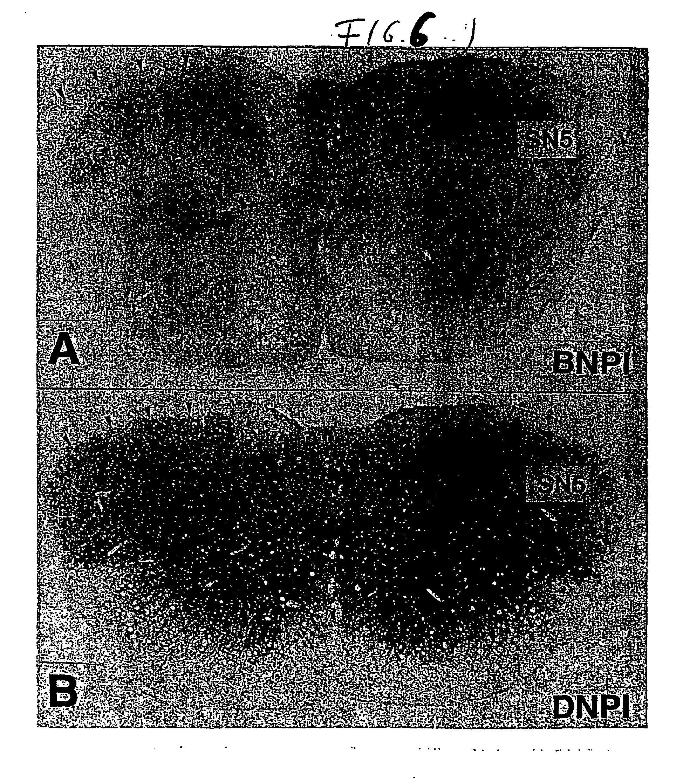

### Beispiel 2d zu Abbildung 6)

Es ist die differentielle Verteilung der Immunreaktivität von BNPI und DNPI in der unteren Medulla oblongate am Übergang zum cervicalen Rückenmark zu sehen. Die angrenzenden Abschnitte A und B jeweils für BNPI (A) und DNPI (B) gefärbt zeigen eine bevorzugte Akkumulation der BNPI-Färbung im medialen Teil des spinalen trigeminalen Nukleus und in dem mittleren und unteren Teil der dorsalen Medulla. es ist nur eine sehr schwache Färbung mit BNPI in den ventralen Medulla zu sehen. DNPI ist abundant in Grey Matter der Medulla. DNPI-Färbung überlappt mit der BNPI-Färbung im inneren spinalen Nucleus V. Es ist zu beachten, daß BNPI auch im oberen spinalen trigeminalen Nukleus, der gleich der spinalen substantia gelatinosa ist, zu finden ist. DNPI-Färbung ist in Gebieten schwächer, in denen BNPI präsent ist, schwächer als in Gebieten, wo BNPI niedrig ist oder fehlt. Einige wenige BNPI Punkte sind im ventralen Grey Motor Gebiet zu sehen.

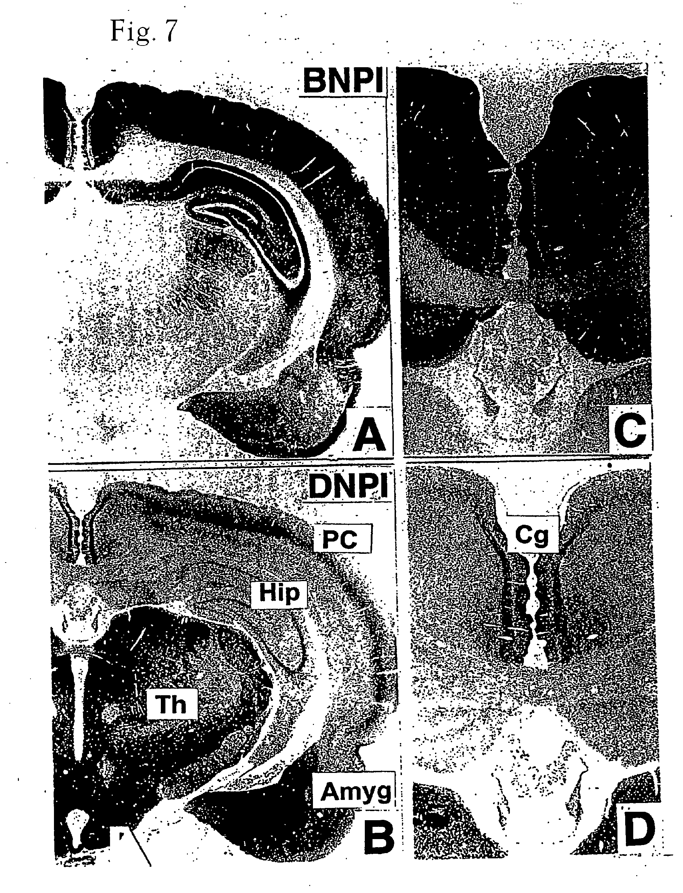

### Beispiel 2e zu Abbildung 7)

Komplementär differentielle Verteilung von DNPI und BNPI Imunreaktivität in 2 Folgeschnitten des Rattenhims in schmerzrelevanten Himregionen wie sensorischer parietaler Cortex; cingulärer Cortex, Thalamus, Corpus amygdaloideum sowie auch Hypothalamus. DNPI ist im Cortex in den granulären sensorischen Schichten insbesondere in Lamina IV konzentriert; BNPI ist im Cortex abundant aber schwächer in der Lamina IV als in anderen Laminae. Im cingulären Cortex (C vs D als Hochvergrößerung) ist die Verteilung von DNPI und BNPI komplementär wechselseitig excludierend bzw. reziprok in der Dichte der jeweiligen Synapsen. DNPI überwiegt im Thalamus eindeutig über BNPI, BNPI ist im Hypothalamus spärlich, DNPI abundant. Abundantes BNPI überwirgt im Hypocampus über spärliches DNPI bei wechselseitig komplementärer Verteilung.
Thalamus = Th,
Amygdala = Amyg.
Hippocampus = Hip,
Cinguläerer Cortex = Cg,
Hypothalamus = Hy,
parietaler Cortex = PC.

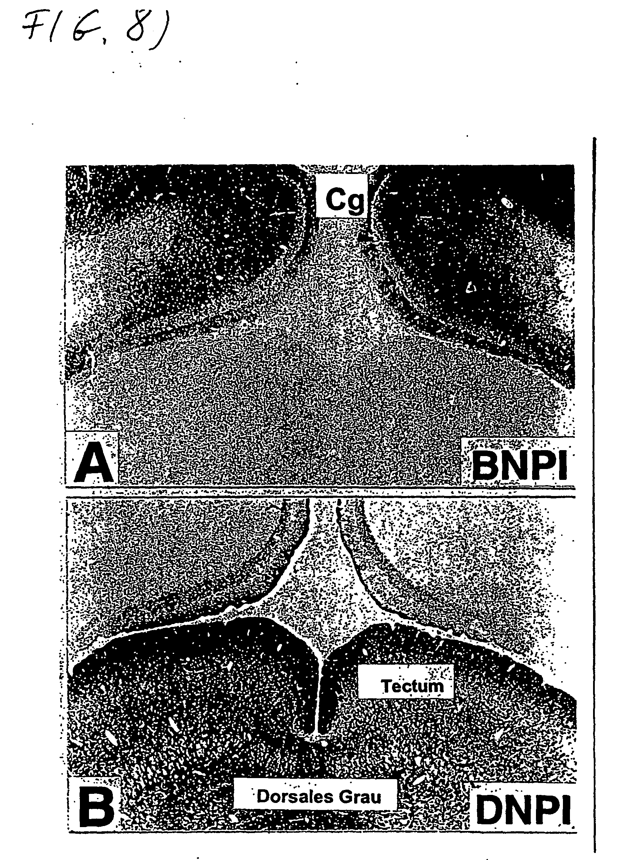

### Beispiel 2f zu Abbildung 8)

Komplementär differentielle Verteilung von DNPI und BNPI Immunreaktivität in schmerzrelevanten Himregionen wie cingulärer Cortex (Cg) und Tectum sowie dorsalem periaquäductalen Grau. DNPI Dominanz im Tectum und dorsalem Grau. Folgeschnitte eines Rattenhims durch das obere Mesencephalon.

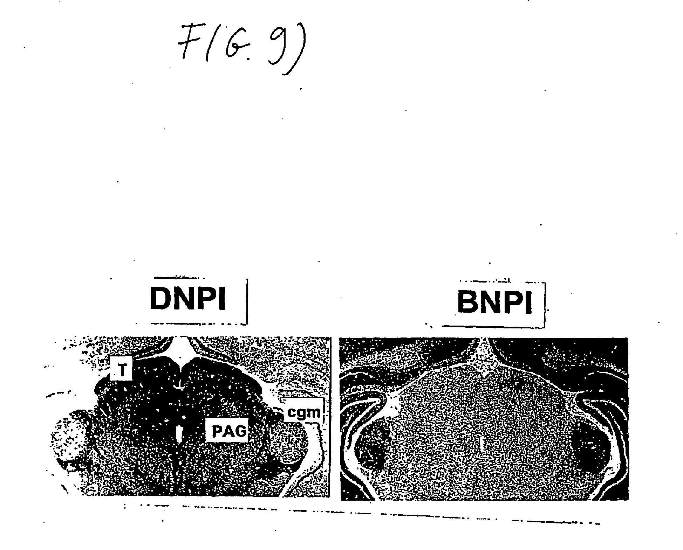

### Beispiel 2g zu Abbildung 9)

Komplementär differentielle Verteilung von DNPI und BNPI Immunreaktivität in schmerzrelevanten Himregionen wie Tectum (T) sowie periaquäductalen Grau (PAG). DNPI Dominanz im Tectum und dorsalem Grau. Man notiere differentielle Verteilung von DNPI und BNPI im corpus geniculatum mediale (cgm) der Hörbahn. Folgeschnitte eines Rattenhims durch das obere Mesencephalon; Ebene colliculus superior.

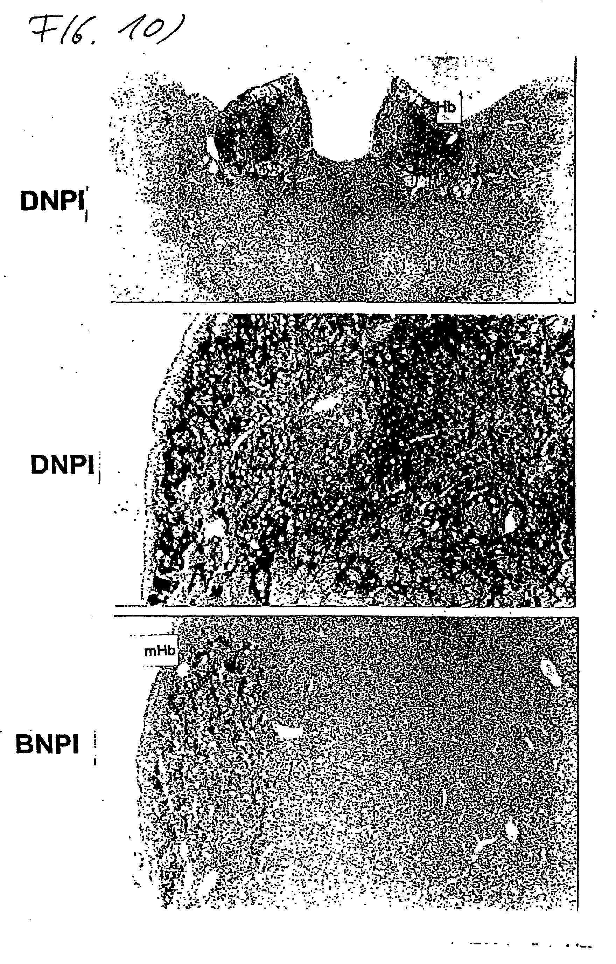

### Beispiel 2h zu Abbildung 10)

Abundanz von DNPI über BNPI in den Habenulae (Hb). DNPI ist präsent im gesamten Habenularkomplex (niedrige Vergrößerung, obere Abbildung; hohe Vergrößerung, mittlere Abb.). BNPI ist nur im medialen Habenularkem (mHb untere Abb., Folgeschnitt zu mittlerer Abbildung).

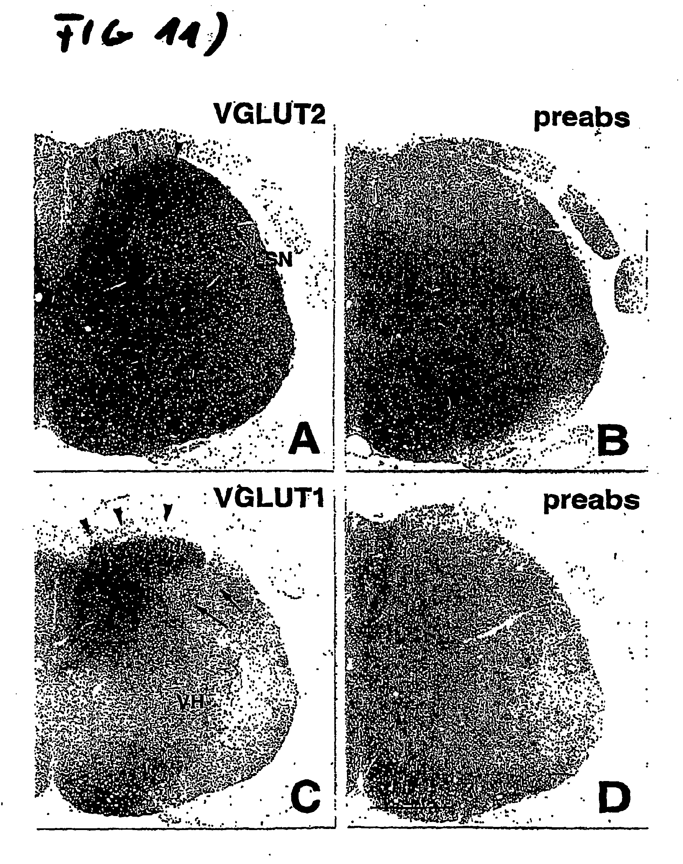

In den folgenden Beispielen 2i bis 2q und den zugehörigen Abbildungen 11- 19 wird der Begriff VGLUT1 für BNPI und VGLUT2 für DNPI verwendet. Die Begriffe sind jeweils vollkommen synonym; inhaltlich völlig gleich und betreffen den gleichen Gegenstand, also VGLUT1 = BNPI und VGLUT2 = DNPI. "preabs" bedeutet die Präabsorption mit VGLUT1-oder VGLUT2-Fusionsprotein vor der lmmunfärbung.

### Beispiel 2i zu Abbildung 11)

Dies ist ein Vergleich zwischen VGLUT1 und VGLUT2 bezüglich der Verteilung und der Spezifität im lumbaren Rückenmark über die Immunoreaktivität.
Die angrenzenden Abschnitte A-D sind abwechselnd mit anti-VGLUT2 (A), anti-VGLUT2, präabsorbiert mit VGLUT2-Fusionsprotein (B), anti-VGLUT1 (C) und anti-VGLUT1, präabsorbiert mit VGLUT1-Fusionsprotein (D) gefärbt. Die Immunoreaktionen in (A) und (C) werden vollständig mit homologem rekombinanten Fusionsprotein präabsorbiert (B) und (D), was die Spezifität der Immunreaktion zeigt. Zu beachten ist das differentielle Verteilungsmuster im oberflächlichen und tiefen Dorsalhorn (A;C). Punktförmige Immunfärbung für VGLUT2 ist im oberflächlichen Dorsalhorn zu erkennen (Pfeile kennzeichnen in A Lamina 1 und Substantia gelatinosa), wo die lmmunreaktivität mit VGLUT1 minimal ist (Pfeile in C). Zu beachten ist weiter die Akkumulation stark positiver punktförmiger VGLUT1 im tiefen Dorsalhorn, wo VGLUT2 relativ schwach ist. VGLUT2 ist im lateralen spinalen Nukleus vorhanden (LSN; Pfeile in A), wo VGLUT1 nur gering vertreten ist (Pfeile in C).VGLUT2 ist stark in Lamina X um den zentralen Kanal herum vertreten, wo VGLUT1 selten ist Die Immunfärbung von VGLUT1 ist schwach bis mittelmäßig i lateralen ventralen Hom und sehr dünn im medialen ventralen Horn. Eine feine punktförmige VGLUT2-Anfärbung ist dicht und reichlich im Ventralhom (VH).

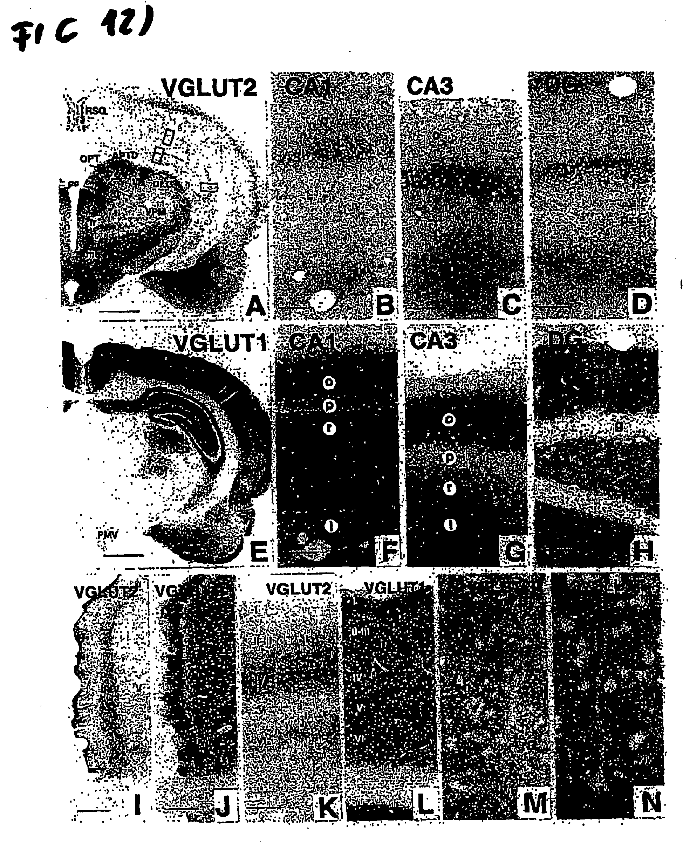

### Beispiel 2j zu.Abbildung 12)

Dies ist ein Vergleich zwischen VGLUT1 und VGLUT2 bezüglich der Verteilung im Vorderhim (1).

Paare von Low-Power- und High-Power-Micrographen von zwei angrenzenden Frontalsektionen sind alternativ für VGLUT2 (A-D, I, K, M) und VGLUT1 (E-H, J, L, N) gefärbt. Dies zeigt die deutliche Differenz und die teilweise gegenseitige Auschließlichkeit in der Verteilung, Dichte und Intensität von VGLUT1 und VGLUT2 Immunoreaktivität (ir) in ausgewählten corticalen, hippocampalen, diencephalischen und limbischen Bereichen.
**Hypothalamus und Thalamus (A,E):** Punktförmige VGLUT2-ir is relativ stark über den ganzen Hypothalamus und Thalamus, wo VGLUT-2 eine beschränkte Verteilung auf den hypothalamischen, ventralen premammillaren Nukleus (PMV) und auf Teile des thalamischen Nukleus inclusive des laterialen hinteren thalamischen Nukleus (LP), dem dorsalen lateralen geniculaten Nukleus (DLG) und dem ventralen posteromedialen thalamischen Nukleus (VPM) zeigt. Olivarer prtectaler Nukleus (OPT); dorsaler vorderer pretectaler Nukleus (APTD); precommisuraler Nukleus (PrC).
**Cortex (A; E; I; J; K; L: M; N):** Die VGLUT2-Färbung ist in einem Band des Neocortex enthaltend Lamina IV moderat. Sie ist schwach in einem neocorticalem Band enthalten Lamina VI und minimal in den anderen neocorticalen Schichten. Intensive punktförmige VGLUT1-ir ist sehr stark im gesamten Cortex, inclusive des pririform Cortex (Pir) und etwas schwächer ausgeprägt im neocortikalen Band der Lamina VI, wo moderate VCGLUT2-Färbung akkumuliert. Zu beachten ist der gegenseitige Ausschluß von VGLUT1- und VGLUT2-Färbung in den Schichten des retrospenialen granularen Cortex (RSG in A und E), die in starker Vergrößerung in (I) und (J) gezeigt werden. Die großen Vergrößerungen M und N aus der Lamina IV in K und L zeigen verschiedene Dichten der punktförmigen VGLUT1- und VGLUT2-ir im Vergleich zwischen immunonegativen neuronalen Zellkörpem und -Prozessen.
**Hippocampus (A, E; B-D, F-H):** Dünne VGLUT2-ir-Punkte sind meist beschränkt auf die granularen Schichten (g) des dentaten Gyrus (DG) und der pyramidalen Schicht (p) der Felder CA1, CA2 und CA3 des Hippocampus. Dichte V-GLUT1-ir-Punkte sind sehr stark über den gesamten Hippocampus vertreten mit Ausnahme der granularen (g) und pyramidalen (p) Zell-Schichten. Mit Rechtecken gekennzeichnete Abschnitte in A und entsprechende Abschnitte auf angrenzenden Sektionen in E werden mit großer vergrößerung jeweils in B-D und F-H gezeigt. zu beachten sind die differentielle Verteilung und Dichte der VGLUT1-ir und VGLUT2-ir in der Oriens-Schicht (o), pyramidalen Schicht (p), im Stratum radiatum (r) und dem Stratum lacunosum molekulare (I) von CA1 (B,F) und CA3 (C, G) und in der molekularen (m), granularen (g) und polymorphen (p) Schicht des dentaten Gyrus (DG) (D,H).
**Amygdala Complex:** Hier ist eine gewisse Überlappung festzustellen, aber die differentielle Dichte und Intensität der Immunfärbung von VGLUT1-ir und VGLUT2-ir Punkten im hinteren basomedialen amygdaloiden Nukleus (BMP), im lateralen amygdaloiden Nukleus (La) und im cortikalen amygdaloiden Nukleus (Co.) wie auch im angrenzenden dorsalen endopiriformen Nukleus (DEn) ist deutlich zu sehen.

Es ist weiter festzustellen, daß "White Matter" und Faser Trakte VGLUT1- und VGLUT2-negativ sind (hintere commisure (pc), fomix (f), fasciculus retroflexus (fr), mammillothalamischer Trakt (mt).

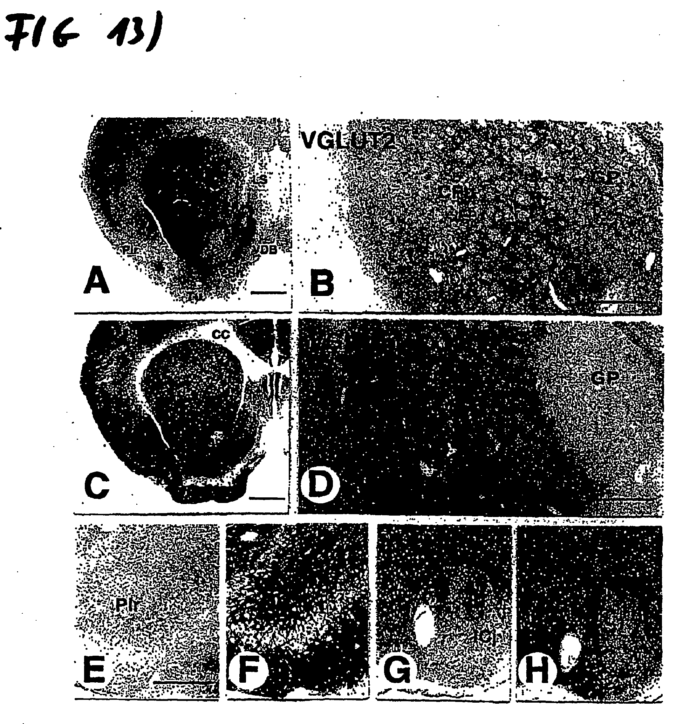

### Beispiel 2k zu Abbildung 13)

Dies ist ein Vergleich zwischen VGLUT1 und VGLUT2 bezüglich der Verteilung im Vorderhirn (2).
Paare von Low-Power- und High-Power-Micrographen von zwei angrenzenden Frontalsektionen sind alternativ für VGLUT2 (A-B, E, G) und VGLUT1 (C-D, F, H) gefärbt. Dies zeigt die deutliche Differenz und die teilweise gegenseitige Auschließlichkeit in der Verteilung, Dichte und Intensität von VGLUT1- und VGLUT2-Immunoreaktivität (ir) im Neocortex (Lamina IV, VI) Caudate Putamen (CPu), Globulus pallidum (GP), piriform cortex (Pir), Nukleus accumbens Kern (AcC), Nukleus accumbens Rand (AcSh), ventralem Pallidum (VP), olfaktorischem Tuberkel (Tu), Inseln von Calleja (ICj), dem ventralen diagonalen Band (VDB) und dem lateralen Septum (LS). Im CPu is VGLUT1 etwas schwächer vertreten als VGLUT2. VGLUT2 ist im Globus pallidum (GP) vorhanden (B), wo VGLUT1 fast vollständig fehlt (D). Im piriformen Cortex (Pir) und den Inseln von Calleja (ICj) ist die punktförmige VGLUT1-ir stärker und dichter als für VGLUT2-ir. Zu beachten ist die Akkumulation schwacher bis moderater VGLUT1-ir in den pyramidalen Zellschichten in (E), wo VGLUT2-ir fast völlig fehlt (F). Zu beachten ist auch eine gewisse Überlappung und Reziprozität in der Färbung von VGLUT1 und VGLUT2 in den ICj (G,H) wie auch die Abwesenheit von VGLUT1-ir und VGLUT2-ir im commissuralem FaserTrakten /Corpus callosum (cc(, vordere commissure (ac).
Balken in A, C = 1 mm, in B, D = 500 µm; in E-H = 200 µm.

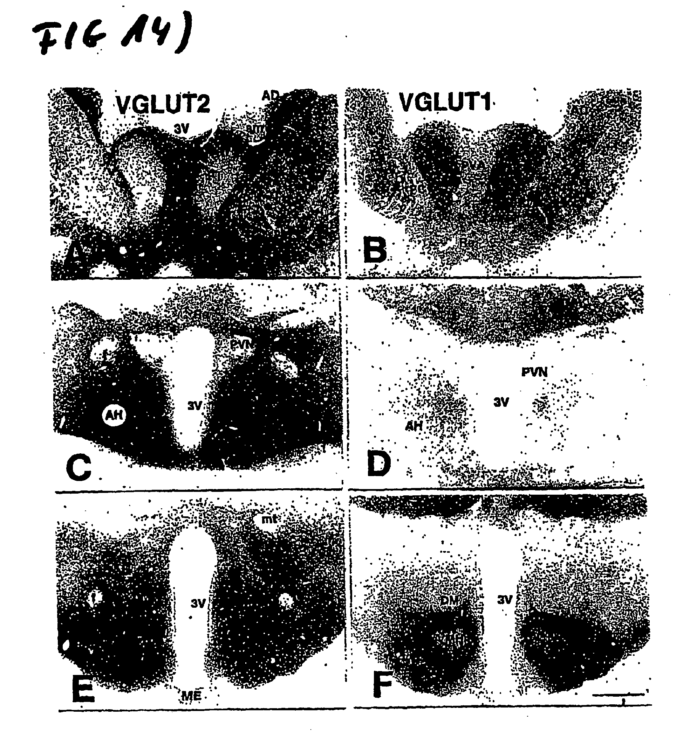

### Beispiel 2l zu Abbildung 14)

Dies ist ein Vergleich zwischen VGLUT1 und VGLUT2 bezüglich der Verteilung im thalamischen und hypothalamischen Nucleus.
Angrenzende frontale Sektionen (A,B) des Diencephalons zeigen die nukleus-spezifische differentielle starke Vorkommen von VGLUT2 (A) und VGLUT1 (B) im Thalamus und Hypothalamus. Zu beachten ist das sehr starke Vorkommen von VGLUT2-ir (A) im paraventrikulären thalamischen Nukleus (PVA), reunienten thalamischen Nukleus (Re), reticularen thalamischen Nukleus (Rt), paracentralem thalamischen Nukleus (PC) und anterodorsalem thalamischen Nukleus (AD). Hier fehlt VGLUT1-ir (B) fast vollständig oder kommt nur in niedriger Konzentration vor. VGLUT1 (B) kommt moderat im hinteren thalamischen Nukleus (PT) vor, wo VGLUT2 (A) selten ist. VGLUT1 ist fehlt fast völlig in der stria medullaris (sm), wo VGLUT2-ir selten ist.
Angrenzende frontale Abschnitte C, D des Diencephalons zeigen die Häufigkeit von VGLUT2 (C) im vorderen hypothalamischen Nukleus (AH) aber die Seltenheit im paraventrikulären Nukleus (PVN) und extreme Seltenheit von VGLUT1-ir im vorderen hypothalamischen Nukleus (AH) und völliges Fehlen im PVN. Zu beachten ist auch die Gegenwart von VGLUT1 (D) im Gegensatz zur Abwesenheit von CGLUT2 (C) im ventromedialen thalamischen Nukleus (VM) und dem Reuniens thalamischen Nukleus (Re).
Angrenzende frontale Sektionen des Hypothalamus (E,F) zeigen die Häufigkeit von VGLUT2 im LH, im ventromedialen thalamischen Nukleus (VHM) und dem dorsomedialen thalamischen Nukleus (DM) und die Seltenheit von VGLUT1 im Kern des VMH aber moderates Vorkommen in seinem Rand. Zu beachten ist die schwache Färbung von VGLUT2 in der medianen Emminenz (ME). VGLUT1 und VGLUT2 fehlen in den Fasertrakten des Formix (f) und des mammillothalamischen Traktes (mt). Dritter ventrikel (3V); Balken = 500 µm (für A-F).

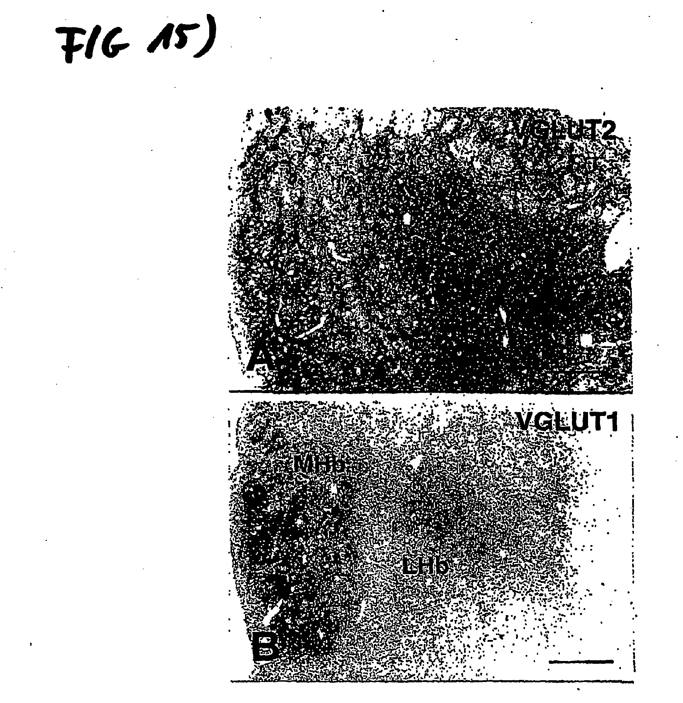

### Beispiel 2m zu Abbildung 15)

Dies ist ein Vergleich zwischen VGLUT1 und VGLUT2 bezüglich der Verteilung im Epithalamus.
High-Power-Micrographen von angrenzenden Sektionen, die alternativ für VGLUT2 (A) und VGLUT1 (B) gefärbt sind, zeigen den Überfluß an VGLUT2 in sowohl dem medialen habenularen Nukleus (MHb) und dem lateralen habenularen Nukleus (LHb). Es ist zu beachten, daß VGLUT1-ir im MHb weniger dicht ist als VGLUT2-in CGLUT1 fehlt fast vollständig im LHb.
Balken = 100 µm (für A,B).

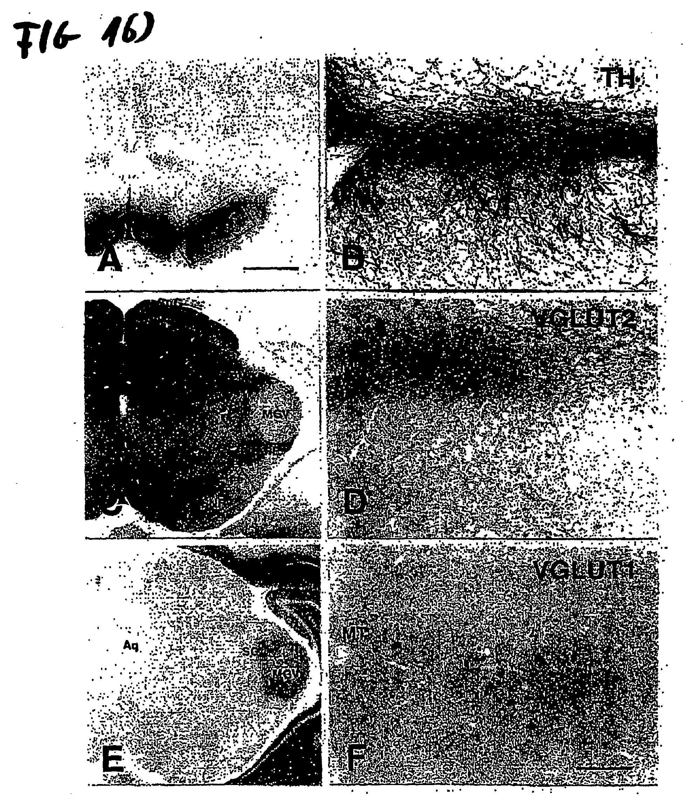

### Beispiel 2n zu Abbildung 16)

Dies ist ein Vergleich zwischen VGLUT1, Tyrosin-Hydroxylase und VGLUT2 bezüglich der Verteilung im Mesencephalon und Metathalamus.
Low-Power- (A,C,E) und High-Power-Micrographen (B,D,F) von drei angrenzenden Sektionen sind alternierend für Tyrosin-Hydroxylase (TH), VGLUT2 und VGLUT1 gefärbt und zeigen die differentielle Verteilung, Dichte und Intensität der Immunfärbung für VGLUT1 und VGLUT2 im Vergleich zu TH. VGLUT2-ir Punkte sind im Tectum konzentriert, wobei die höchsten Mengen in der supeficialen "Grey-Schicht des oberen Colliculus (SuG) und geringere Mengen in der intermediären "Grey"-Schicht des oberen Colliculus (InG) zu finden sind, während diese in der optischen Nukleus-Schicht des oberen Colliculus (Op) selten sind. VGLUT2-ir Punkte liegen im gesamten Tegmentum inclusive des Nukleus ruber (R) und der TH-positiven pars compacta der substantia nigra (SNC) vor und sind im dorsalen periaquäductalem Grey (PAG) besonders angereichert und insbesondere im medialen terminalen Nukleus des "accessory optic tract" (MT), des optischen Zugangstrakts, wie auch im mediocaudalen Teil des lateralen hinteren Nukleus (LPMC), im hinteren intralaminaren thalamischen Nukleus (PIL) im peripeduncularen Nukleus (PP) und im suprageniculaten thalamischen Nukleus (SG). VGKUT1-ir ist hier minimal. VGLUT 1-Färbung zeigt sich in moderaten Mengen im ventralen medialen geniculaten Nukleus (MGV), wo VGLUT2-Mengen minimal sind. VGLUT1 ist im gesamten Tectum, dem periaquaeductalem Grey und dem tegmetum nur minimal vorhanden und fehlt fast vollständig in der substantia nigra pars compacta (SNC) und der pars reticulais (SNR) Schwache VGLUT2-Färbung ist in den neuronalen Perikarya und Puncta in der SNR vorhanden (D, high-powered Micrograph aus der durch ein Rechteck gekennzeichneten in (C), wo VGLUT1 fast vollständig fehlt (korrespondierend in F)). Mesencephalischer Aquaedukt (Aq.) Balken in A, C, E =1mm; in B, D, F = 200 µm.

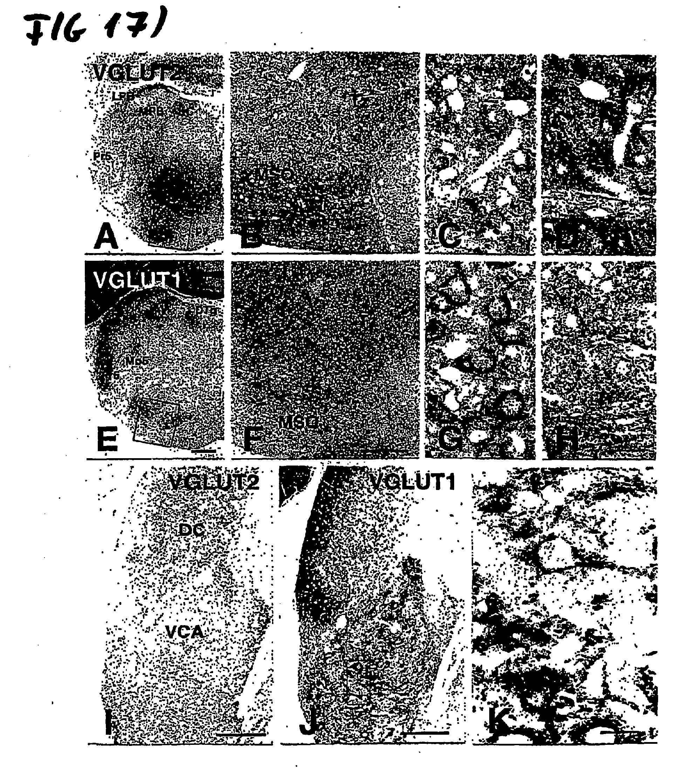

### Beispiel 2o zu Abbildung 17)

Dies ist ein Vergleich zwischen VGLUT1 und VGLUT2 bezüglich der Verteilung im pontomedullären Himstamm.
Low-Power- und High-Power-Micrographen von zwei angrenzenden Frontalsektionen sind alternierend für VGLUT2 (A-D) und VGLUT1 (E-H) gefärbt. Dies zeigt eine starke Menge punktförmiger VGLUT2-Immunoreaktivität (ir) (B, D) in der medialen oberen Olive (MSO), wo VGLUT1-ir niedrig ist (F, H). VGLUT2-ir ist relativ schwach im Nukleus des trapezoiden Körpers (TZ) (B-C) ausgeprägt, wo VGLUT1-ir in großer Menge vorliegt (F-G). Es ist zu beachten, daß deutlich VGLUT1-positive confluente große Punkte immunonegative neuronale Zellkörper im TZ (G) umschließen. Starke VGLUT1-ir-Punkte sind in den zentralen sensorischen Nukleus des trigeminal Nervs (Pr5), wo VGLUT2-ir sehr niedrig ist. Moderat positive VGLUT1 Punkte sind in dem motorischen trigeminalen Nukleus (Mo5) vorhanden, wo VGLUT2-ir niedrig ist. VGLUT1-ir und VGLUT2-ir sind mit gerringer Menge im lateralen medialen parabrachialen Nukleus vertreten (LPB, MPB). Moderate VGLUT2-ir akkumuliert im locus coerulus (LC), wo VGLUT1-ir sehr gering ist. VGLUT1-ir und VGLUT2-ir fehlen im pyramidalen Trakt (pyr). Angrenzende Sektoren (I,J) alternierend für VGLUT2 (I) und VGLUT1 (J) gefärbt, zeigen eine starke Menge punktförmiger VGLUT1-Immunoreaktivität (ir) im vorderen ventralen cochlearen Nukleus (VCA), wo VGLUT2 im Prinzip völlig fehlt. Ein High-Power-Micrograph (K) von J zeigt stark positive VGLUT-ir Punkte, die immunonegative neuronale Zellkörper und Prozesse einschliessen. VGLUT1-ir Punkte sind in größerer Zahl im dorsalen cochtearen Nukleus (DC) als VGLUT2-ir-positive Punkte.
Balken in A, E = 500 µm; in B, F, I, J = 200 µm; in C, D, G, H, K = 25 µm.

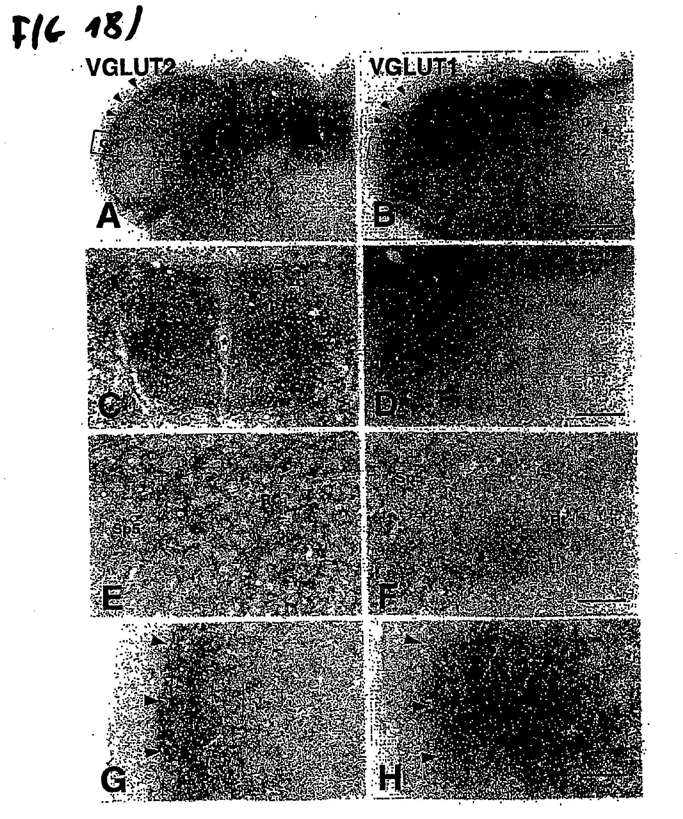

### Beispiel 2p zu Abbildung 18)

Dies ist ein Vergleich zwischen VGLUT1 und VGLUT2 bezüglich der Verteilung im unteren Himstamm.

Low-Power- und High-Power-Micrographen von zwei angrenzenden Frontalsektionen sind alternierend für VGLUT2 (A, C, E, G) und VGLUT1 (B, D, F, H) gefärbt, zeigen eine moderate Menge kleiner punktförmiger VGLUT2-Immunoreaktivität (ir) im oberflächlichen spinalen trigeminalen Nukleus (Sp5), was durch Pfeile markiert ist (A, G), wo VGLUT1-ir im Prinzip völlig fehlt (Pfeile in B, H). VGLUT2 ist im doorsalen motorischen Nukleus des Vagus (10), hypoglossalen Nukleus (12), der retikularen Formation (Rt) und im ventralen Teil des solitären Traktes (SoIV) (A,C,E,) moderat vorhanden, wo VGLUT1 im Prinzip völlig fehlt (B, D, F). VGLUT2-ir ist im dorsalen solitären Trakt (SolD) sehr niedrig. Zu beachten ist das Übergewicht VGLUT1 im tiefen Sp5 (B, F, H), im Cuneat (Cu) und dem grazilen Nukleus (GR), wo VGLUT2-ir niedrig ist. Sterne markieren den zentralen Kanal.
Balken in A, B = 500 µm; C,D = 200 µm, E,F = 100 µm; G,H = 100 µm.

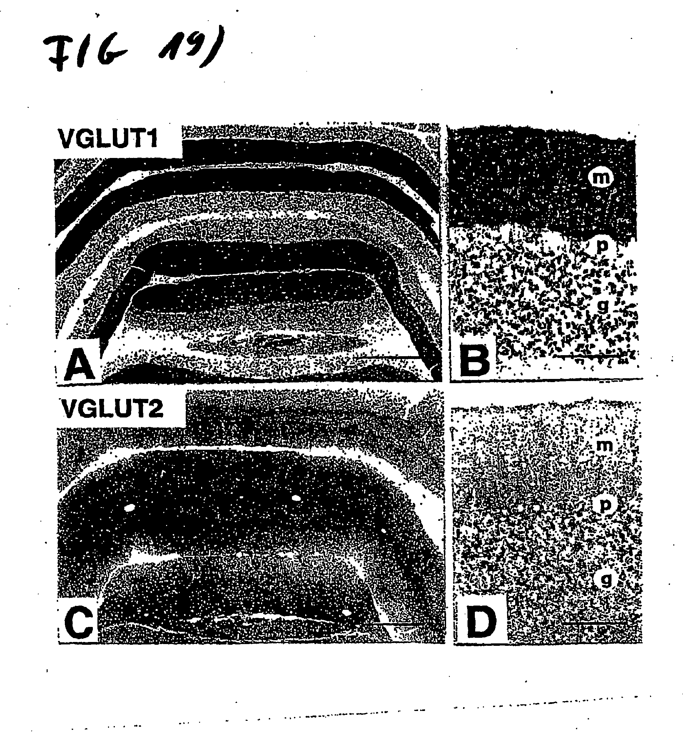

### Beispiel 2q zu Abbildung 19)

Dies ist ein Vergleich zwischen VGLUT1 und VGLUT2. bezüglich der Verteilung im Cerebellum.
Low-Power und High-Power-Micrographen von zwei angrenzenden Frontalsektionen, alternierend für VGLUT2 (A, B) und VGLUT1 (C, D) gefärbt, zeigen eine extreme Dichte intensiv gefärbter VGLUT1-positiver Punkte in der molekularen Schicht (m), sehr wenige VGLUT1 Punkte um Somata der Purkinje Zelle in der Purkinje Zellschicht (p) und dichte glomeruli-ähnliche Akkumulation stark gefärbter konfluenter VGLUT1 Punkte in der granularen Schicht (g). VGLUT2-ir Punkte sind viel weniger dicht in der molekularen Schicht, wo Sie in einer bandförmigen Art angeordnet sind. VGLUT2-ir Punkte, die glomerula-ähnliche Strukturen in der glomerularen Schicht (g) bilden, sind weniger dicht als die, die füpr VGLUT1 färben.

Balken in A, C = 500 µm; in B,D = 100 µm.

### Diskussion und Analyse zu Beispiel 2 allgemein:

Die differentielle Verteilung von BNPI und DNPI in Synapsen des primärafferenten, spinalen trigeminalen und supraspinalen Systems ist eine starke Evidenz für eine selektive Beeinflußbarkeit sensorischer Funktionen durch selektive Modulation des DNPI bzw. BNPI-vermittelten Glutamat-Transports.

Die Präsenz von BNPI und DNP im DRG weist auf die Möglichkeit hin, periphere neurogene Entzündungen selektiv durch selektive Intervention am DNPI oder BNPI-Target zu beeinflussen. Die Präsenz im DRG indiziert auch eine immunmodulatorische Rolle und entsprechende Targetierung. Eine Präsenz von BNPI und DNPI im sensorischen Vagus oder Glossopharyngeus Ganglion indiziert das Target für Baroafferenz, Chemoafferenz, cardiovaskuläre oder cardiorespiratorische Funktion, inklusive Asthma, Hypertonie etc., wie auch für Emesis. Interessant ist die Verteilung auch für die Darm-Gehim-Achse, also Regulalion der Sättigung, der "Inflammatory bowel disease" oder Morbus Crohn ebenso wie für Autoimmunität im zentralen odere peripheren Nervensystem, autoimmuner Diabetes, alkoholischer Neuropathie, Alkohol induzierter chronischer Pankreatitis mit Neuroproliferation (Fink et al. mit Weihe; Neuroscience). Alleine die Verteilung im ZNS und PNS macht diese Targets zu interessanten Objekten in den restlichen bereits oben genannten Indikationen.

Ein entscheidender Punkt war aber auch, daß BNPI und DNPI in den afferenten Bereichen zu den sensorischen Bereichen des Auges und des Ohres nachgewiesen werden konnten, was in Kombination mit den anderen Erkenntnissen eine wichtige Rolle bei Sehstörungen, Retinitis pigmentosa, Opticus Degeneration, Katarakt, Netzhautablösung, Retinadegeneration, Glaukom oder Nystagmus oder Hörstörungen, Tinitus, M. Menière, Hörsturz, Erkrankungen des Hör- und/oder Gleichgewichtsorgans oder Erkrankungen der Hörbahn oder Vesibularbahn nahelegt.

Entscheidend ist weiter, daß die Verteilung von VGLUT2 (=DNPI) in den meisten Regionen des Gehirns und des Rückenmarks komplementär und gegenseitig ausschließend zur Expression von VGLUT1 (=BNPI) ist. Zusammen könnten die beiden Glutamat-Transporter für die Aufnahme von Glutamat durch synaptische Vesikel aller centralen glutamatergen Neuronen verantwortlich sein.

Es wurde hier gefunden, daß die thalamischen und Gehimstamm-Relais-Zentren des visuellen und statoakkustischen Pfades durch differentielle VGLUT1- und VGLUT2- gesteuerte Signale getrieben werden. Thalamische und mesenephalische Relay-Zentren des visuellen Systems wie der colliculus superior und der dorsolaterale geniculate Nukleus und der mediale terminale Nukleus des "Accessory optic tracts", des zugehene optischen Sytems sind spezifisch VGLUT2-gesteuert, was nahelegt, daß die retinalen ganglionischen Zellen, die das dritte Neuron des optischen Sinnes repräsentieren, zumindest teilweise mit VGLUT2 beschichtet sind. Im Gegensatz dazu erhalten der Hirnstamm cochlear, olivary trapezoid und das metathalamische mediale geniculate Relay-Zenter des Gehör-Pfades starken Input von VGLUT1-beschichteten glutamatergen Synapsen.
Verschiedene Nuklei des Gehimstamm visuellen Systems erhalten einen starken Input durch VGLUT2 synaptische Punkte. Daher scheint der Gehimstamm des optischen Systems ausschließlich durch VGLUT2 glutamaterge Synapsen versorgt zu werden.
Es ergeben sich folgende Ergebnisse und Folgerungen aus den Untersuchungen: DNPI ist ein Marker für glutamaterge synaptische Vesikel, wobei es 2 unterschiedliche Typen von Neuronen, bzw. Synapsen gibt. VGLUT1 und VGLUT2 zeigen ein differentielles Verteilungsmuster.
Insgesamt läßt sich aus der Verteilung der BNPI und DNPI im ZNS und PNS zeigen, daß diese eine Rolle bei den verschiedenen bereits oben genannten Indikationen spielen, für die mit dem erfindungsgemäßen Verfahren an diesen Targets ansetzende arzneilich wirksame Verbindungen gesucht werden.

### Beispiel 3:

### Durchführung des Screeningverfahrens mit Messung der Bindung über die Verdrängung eines radioaktiv markierten Liganden

Ein Nukleinsäureabschnitt, der für BNPI kodiert, wird in einem Expressionsvektor kloniert, der eine konstitutive Expression (z.B. CMV-Promoter) oder eine induzierbare Expression in eukaryonten Zellen erlaubt. Die DNA wird mit geeignetem Transfektionsverfahren, z.B. mit Lipofectamin (Fa. Roche Diagnostics) in eukaryontischen Zellen (z.B. CHO-Zellen, HEK293-Zellen oder NIH-3T3-Zellen) hineingebracht Die Zellen werden in Anwesenheit eines Selektionsreagenzen (z.B. Zeocin, Hygromycin oder Neomycin) kultiviert so daß nur die Zellen überleben, die das DNA-Konstrukt aufgenommen haben, und bei länger andauernder Selektion, auch in das Genom inkorporiert haben.
Ausgehend von diesen Zellen werden Membranfraktionen gewonnen, die BNPI in großer Menge enthalten und für einen Bindungsassay verwendet werden können. Dieser besteht aus 1.) dem BNPI enthaltenden Membranen 2.) einem radioaktiv markierten Liganden 3.) einem Bindungspuffer (z.B. 50 mM HEPES pH 7.4, 1 mM EDTA) und dem auf Bindung zu untersuchenden Liganden. Nach Inkubation der oben genannten Reaktionsgemische (für z.B. 30-60 min) bei einer geeigneten Temperatur (meistens Raumtemperatur) werden die nichtgebundenen radioaktiven Ligandenmoleküle abfiltriert. Die Restmenge an gebundenem Liganden wird nach Zugabe eines Szintillationscocktails in einem β-Counter (z.B. Trilux, Fa. Wallac) vermessen. Zeigt die Testsubstanz eine Bindung an BMPI, so wird dies als verringerter radioaktiver Einbau detektiert. Dieses Verfahren wird geeignetermaßen so miniaturisiert, daß es in (96-, 384- oder 1536-well) Mikrotiterplatten durchgeführt werden kann, um dieses Verfahren mittels eines Roboters im sogenannten Hightroughput-Screening (HTS)-Verfahren durchzuführen.

### Beispiel 4:

### Durchführung des erfindungsgemäßen Screeningverfahrens mit BNPI und Messung der durch die Bindung der Substanz veränderten funktionellen Parameter

Ein Nukleinsäureabschnitt, der für BNPI kodiert, wird in einem Expressionsvektor kloniert, der eine induzierbare Expression in Prokaryonten, wie z.B. E.coli erlaubt. Hierbei wird der Nukleinsäureabschnitt so modifiziert, daß er als Fusionsprotein mit einer zusätzlichen N- oder C-terminalen Aminosäuresequenz exprimiert wird. Diese Sequenz sollte bei unveränderter Funktion des BNPI eine Aufreinigung über ein spezifisches Verfahren erlauben, z.B. Glutathion S-Transferasefragment, das über Bindung an Glutathion eine Isolierung aus dem Proteingemisch erlaubt. Nach Transfektion der Bakterien, Induktion des Genes (z.B. mit IPTG beim Iao-Promoter) und Aufschließen der Bakterien werden die Fusionsproteine aufgereinigt und in einem in vitro-Kinase Experiment eingesetzt. Hierbei werden 5 µg Protein bei 30°C für 30 Minuten in 50 µl Kinasepuffer (20 mM PIPES, pH 7.0, 5 mM MnCl₂, 7 mM β-Mercaptoethanol, 0.4 mM Spermin, 10 mM rATP) ergänzt mit 10 µCi [γ³²P] ATP. Als Substrate werden gereinigtes Histon H1-Protein (Fa. Sigma) oder bakteriell exprimiertes GST-NFATc1-Fusionsprotein hinzugegeben. Nach der Inkubationszeit wird das nicht-inkorpierte [γ-³²P] ATP abfiltriert und die Menge an eingebautem ³²Phosphat durch β-Szintillation (Trilux, Fa. Wallac) bestimmt. In einem Experiment zum Aufspüren neuer BNPI-Inhibitoren werden in diesem Ansatz die Testsubstanzen mitinkubiert und eine Abnahme der ³²P-Inkorporation als Indikator für einen Inhibitor benutzt. Dieses Verfahren wird geeigneter maßen so miniaturisiert, daß es in (96-, 384- oder 1536-well) Mikrotiterplatten durchgeführt werden kann, um dieses Verfahren mittels eines Roboters im sogenannten Hightroughput-Screening (HTS)-Verfahren durchzuführen.

### Beispiel 5.

### Beispiel für ein Arzneimittel zur Tinitus-Behandlung oder zur Behandlung motoneuronischer Störungen enthaltend eine durch ein erfindungsgemäßes Verfahren identifizierte Verbindung - Tablettenformulierung

Tabletten können durch direktes Verpressen von Mischungen der erfindungsgemä&en Verbindung mit entprechenden Hilfsstoffen oder durch Verpressen von verbindungshaltigen Granulaten (mit gegebenenfalls weiteren Hilfsstoffen) hergestellt werden. Die Granulate können dabei entweder durch Feuchtgranulation mit z.B. wäßrigen Granulierflüssigkeften und anschließender Trocknung dieser Granulate oder durch Trockengranulation z.B. über Kompaktierung hergestellt werden.
■ Direktverpressung

| | | |
|---|---|---|
| z.B. pro Tablette: | 25 mg | durch erfindungsgemäßes Verfahren identifizierte Verbindung |
| | 271 mg | LudipressTM (Granulat zur Direkttablettierung aus Lactose monohydrat, Povidon K30 und Crospovidon) |
| | 4 mg | Magnesiumstearat |
| | 300 mg | Gesamt |

Homogene Mischung des Wirkstoffes mit den Hilfsstoffen herstellen und diese auf einer Tablettenpresse zu Tabletten mit einem Ø von 10 mm verpressen.
■ Trockengranulation

| | | |
|---|---|---|
| z.B. pro Tablette: | 25 mg | gemäß dem erfindungsgemäßem Verfahren identifizierte Verbindung |
| | 166 mg | Microcristalline Cellulose |
| | 80 mg | Niedrig substituierte Hydroxypropylcellulose (I-HPC LH 11™) |
| | 5 mg | Hochdisperses Siliziumdioxid |
| | 4 mg | Magnesiumstearat |
| | 280 mg | Gesamt |

Homogene Mischung der Verbindung mit der Mikrokristallinen Cellulose und der I-HPC herstellen und diese Kopaktieren. Nach dem Sieben der Komprimate wird das entstandene Granulat mit Magnesiumstearat und Siliziumdioxid gemischt und auf einer Tablettenpresse zu Tabletten mit einem Ø von 9 mm verpreßt.
■ Feuchtgranulation

| | | |
|---|---|---|
| z.B. pro Tablette: | 25 mg | durch erfindungsgemäßes Verfahren identifizierte Verbindung |
| | 205 mg | Mikrokristalline Cellulose |
| | 6 mg | Povidon K30 |
| | 10 mg | Crospovidon |
| | 4 mg | Magnesiumstearat |
| | 250 mg | Gesamt |

Homogene Mischung der Verbindung mit der Mikrokristallinen Cellulose und dem Crospovidon herstellen und diese in einem Granulator mit einer wäßrigen Lösung des Povidons granulieren. Das feuchte Granulat wird anschließend nachgranuliert und nach der Trocknung im Trockenschrank (50°C) 10 h getrocknet. Das trockene Granulat wird mit dem Magnesiumstearat zusammen gesiebt, endgemischt und auf einer Tablettenpresse zu Tabletten mit einem Ø von 8 mm verpreßt.

### Beispiel 6:

### Beispiel für ein Arzneimittel zur Tinitus-Behandlung oder zur Behandlung motoneuronischer Störungen enthaltend eine nach einem erfindungsgemäßen Verfahren identifizierte Verbindung- parenterale Lösung

1 g einer nach einem erfindungsgemäßen Verfahren identifizierten Verbindung wird in 1 l Wasser für Injektionszwecke bei Raumtemperatur gelöst und anschließend durch Zugabe von NaCl (Natriumchlorid) auf isotone Bedingungen eingestellt.

### Literatur:

Aihara Y, Mashima H. Onda H. Hisano Setsuji, Kasuya H., Hori T. Yamada S., Tomura H. Yamada Y., Inoue I., Kojima I. und Takeda J. (2000), J. Neurochem. 74: 2622 - 2625
Akopian AN, Sivilotti L & Wood JN (1995) Nature 379: 257-262
Ausubel FM, Brent R, Kingdton RE, Moore DD, Seidman JG, Smith JA & Struhl K eds.(1190) Current protocols in molecular biology. John Wiley &Sons, Inc. New York, NY.
Baba H, Doubell TP, Woolf CJ 1999: Peripheral inflammation facilitates Aβ fiber-mediated synaptic input to the substantia gelatinosa of the adult rat spinal cord. J Neurosci 19: 859-867
Bauer D, Müller H, Reich J, Riedel H, Ahrenkiel V, Warthoe P & Strauss M (1993): Identification of differentially expressed mRNA species by an improved display technique (DDRT-PCR) Nucl Acids Res 21: 4272-4280.
Bonini A, Anderson SM, Steiner DF (1997) Molecular cloning and tissue expression of a novel orphan G Protein-coupled receptor from rat lung. Biochem Biophys Res Comm 234: 190-193.
Chih-Cheng et al., (1995): A P2X prinoceptor expressed by a subset of sensory neurons. Nature 377:428-432
Corderre TJ, Katz J, Vaccarino AL, Melzack R (1993): Contribution of central plasticity to pathological pain: review of clinical and experimental evidence. Pain 52: 259-285.
Dickenson (1995) Novel pharmacological targets in the treatment of pain. Pain Rev., **2,** 1-12.
Dubuisson et al., 1997 Pain, 4:161-174.
Feng Y & Gregor P (1997) Cloning of a novel member of the G Protein-coupled receptor family related to peptide receptors. Biochem Biophys Res Comm 231: 651-654.
Furukawa T, Yang Y, Nakamoto B, Stamatoyannopoulos G, Papayannopoulou T (1996): Identification of new genes expressed in a human erythroleukemia cell line. Bloods Cell Mol & Dis 22:11-22.
Gunasekar PG, Kanthasamy, AG, Borowitz JL, Isom GE 1995: NMDA receptor activation produces concurrent generation of nitric oxide and reactive oxygen species: implication for cell death. J Neurochem 65: 2016-2021.
Hawes BE, Fried S, Yao X, Weig B, Graziano MP 1998: Nociceptin (ORL1) and µ-Opioid receptors mediate mitogen-activated protein kinase activation in CHO cells through a Gi-coupled signaling pathway: evidence for distinct mechanisms of agonist-mediated desensitization. J Neurochem 71: 1024-1033.
Hubank M & Schatz DG (1994): Identifying differences in mRNA expression by representational difference analysis of cDNA. Nucl Acids Res 22: 5640-5648.
Klußmann S et al., 1996: Nature Biotechnology 14: 1112-1115.
Li L-Y & Chang K-J 1996: The stimulatory effect of opioids on mitogen-activated protein kinase in chinese hamster ovary cells transfected to express µ-opioid receptors. Mol Pharm 50:599-602.
Liang P & Pardee AB 1992: Differential Display of eukaryotic messenger RNA by means of the polymerase chain reaction. Science 257:967-971.
Methner A, Hermey G, Schinke B, Hermanns-Borgmeyer I (1997): A novel G Protein-coupled receptor with homology to neuropeptide and chemoattractant receptors expressed during bone development. Biochem Biophys Res Comm 233: 336-342.
Mohit AA, Martin JH & Miller CA 1995: p493F12 Kinase: a novel MAP kinase expressed in a subset of neurons in the human nervous system. Neuron 14: 67-78.
Poirier GM-C, Pyati J, Wan JS, Erlander MG 1997: Screening differentially expressed cDNA clones obtained by differential display using amplified RNA. Nucleic Acids Research 25: 913-914.
Sambrook J, Fritsch EF & Maniatis T 1989: Molecular Cloning: A Laboratory Manual. Second Edition. Cold Spring Harbor Laboratory Press, Cold Spring Harbor.
Sompayrac L, Jane S, Bum TC, Tenen DG & Danna KJ 1995: Overcoming limitations of the mRNA differential display technique. Nucleic Acids Research 23: 4738-4739.
Tal M 1996: A novel antioxidant alleviates heat hyperalgesia in rats with an experimental painful neuropathy. Neurreport 7: 1382-1384.
Tölle TR (1997): Chronischer Schmerz. In: Klinische Neurobiologie, Herdergen T, Tölle TR, Bähr M (Hrsg.): S. 307-336; Spektrum Verlag, Heidelberg.
U.S.Patent 5.262.311
Velculescu VE, Zhang L, Vogelstein B, Kinzler KW (1995): Serial analysis of gene expression. Science 270: 484-487.
Wan JS, Sharp JS et al. (1996): Cloning differentially expressed mRNAs Nature Biotech 14:1685-1691.
Watson JB & Margulies JE (1993) Differential cDNA screening strategies to identify novel stage-specific proteins in the developing mammalian brain. Dev Neurosci 15: 77-86.
Wilks AF (1989) Two putative protein-tyrosine kinases identified by application of the polymerase chain reaction. Poc Natl Acad Sci USA 86: 1603-1607.
WO96/34288
Woolf CJ, Shortland P, Coggeshall RE 1992: Peripheral nerve injury triggers central sprouting of myelinated afferents. Nature 355:75-78.
Zimmermann, M & Herdegen, T (1996): Plasticity of the nervous system at the systemic, cellular and molecular levels: a mechanism of chronic pain and hyperalgesia. Progr Brain Res 110: 233-259

## Patentansprüche

1. Verfahren zur Auffindung pharmazeutisch relevanter Substanzen mit Wirksamkeit in den Indikationen oder zur Behandlung von amyotropher Lateralsklerose, Erkrankungen des spinalen Motoneurons, Muskelatrophien, Muskeldystrophien oder multipler Sklerose mit folgenden Verfahrensschritten:
(a) Inkubation einer zu testenden Substanz unter geeigneten Bedingungen mit mindestens einem Biomolekül aus Gruppe I ausgewählt aus:
dem Protein BNPI und/oder einem Protein gemäss einer der Abbildungen 1 b), 1 d), 1f) oder 1h) und/oder einem zu einem dieser vorgenannten Proteine zu mindestens 90 % ähnlichen Protein und/oder einem Protein, für das ein Polynukleotid gemäss einer der Abbildungen 1a), 1c), 1e) oder 1g) oder ein dazu zu mindestens 90 % ähnliches Polynukleotid kodiert, und/oder einem Protein, das durch eine Nukleinsäure kodiert wird, die unter stringenten Bedingungen an ein Polynukleotid gemäss einer der Abbildungen 1a), 1c), 1e) oder 1g) oder deren Antisense Polynukleotide bindet,
und/oder einer Zelle und/oder einer Präparation aus einer solchen Zelle, die mindestens eines der vorgenannten Proteine, bzw. Biomoleküle aus Gruppe 1, synthetisiert hat,
(b) Messung der Bindung der Testsubstanz an dem oder den gegebenenfalls von einer Zelle synthetisierten Biomolekül/en aus Gruppe I oder an einer Zelle und/oder einer Präparation aus einer solchen Zelle, die mindestens eines der vorgenannten Biomoleküle aus Gruppe 1 synthetisiert hat, oder Messung mindestens eines der durch die Bindung der Testsubstanz an dem oder den gegebenenfalls von einer Zelle synthetisierten Biomolekül/en aus Gruppe I oder an einer Zelle und/oder einer Präparation aus einer solchen Zelle, die mindestens eines der vorgenannten Biomoleküle aus Gruppe I synthetisiert hat, veränderten funktionellen Parameter, über Messung der Regulation, Hemmung und/oder Aktivierung von Rezeptoren, lonenkanälen und/oder Enzymen, insbesondere über Messung der Veränderung der Genexpression, des lonenmilieus, des pH oder des Membranpotentials, über Veränderung der Enzymaktivität oder der Konzentration der 2nd messenger,
oder
über Messung der Bindung über die Verdrängung eines bekannten markierten Liganden des Biomoleküls und/oder Proteins und/oder über die daran gebundene Aktivität einer markierten Testsubstanz.

2. Verfahren gemäss Anspruch 1, **dadurch gekennzeichnet, dass** die Zelle vor dem Schritt (a) gentechnisch manipuliert wird.

3. Verfahren gemäss Anspruch 2, **dadurch gekennzeichnet, dass** die gentechnische Manipulation die Messung mindestens eines der durch die Testsubstanz veränderten funktionellen Parameter erlaubt.

4. Verfahren gemäss Anspruch 3, **dadurch gekennzeichnet, dass** durch die gentechnische Manipulation eine in der Zelle nicht endogen exprimierte Form eines G-Proteins exprimiert oder ein Reportergen eingeführt wird.

5. Verfahren gemäss einem der Ansprüche 2-4, **dadurch gekennzeichnet, dass** die Zelle gentechnisch so manipuliert wird, dass die Zelle mindestens ein Polynukleotid gemäss einer der Abbildungen 1a), 1c), 1e) oder 1g) oder ein dazu zu mindestens 90 %, vorzugsweise zu mindestens 95 %, insbesondere zu mindestens 97 % ähnliches Polynukleotid enthält.

6. Verfahren gemäss Anspruch 5, **dadurch gekennzeichnet, dass** das Polynukleotid in einem rekombinanten DNA-Konstrukt enthalten ist.

7. Verfahren gemäss einem der Ansprüche 2 bis 6, **dadurch gekennzeichnet, dass** die Zelle nach der gentechnischen Manipulation gemäss Anspruch 2 und vor dem Schritt (a) gemäss Anspruch 1 unter Bedingungen, die eine Expression erlauben, kultiviert wird, gegebenenfalls unter Selektionsdruck.

8. Verfahren gemäss einem der Ansprüche 1 bis 7, **dadurch gekennzeichnet, dass** die Zelle eine Amphibienzelle, Bakterienzelle, Hefezelle, Insektenzelle oder eine immortalisierte oder native Säugetierzelle ist.

9. Verfahren gemäss einem der Ansprüche 1 bis 8, bei dem ein erstes Verfahren gemäss einem der Ansprüche 1 bis 8 mit einem zweiten Verfahren gemäss einem der Ansprüche 1 bis 8 derart gekoppelt wird, dass die Messwerte und Ergebnisse des ersten Verfahrens hinsichtlich der zu messenden Substanz mit den Messwerten und Ergebnissen des zweiten Verfahrens hinsichtlich der zu messenden Substanz verglichen werden, **dadurch gekennzeichnet, dass** in einem der zwei Verfahren, im folgenden Hauptverfahren genannt, im Schritt (a) die zu testende Substanz
mit einem Biomolekül ausgewählt aus Gruppe II:
dem Protein BNPI und/oder einem Protein gemäss einer der Abbildungen 1b), 1d), 1f) oder 1h) und/oder einem zu einem dieser vorgenannten Proteine zu mindestens 90 % ähnlichen Protein und/oder einem Protein, für das ein Polynukleotid gemäss einer der Abbildungen 1a), 1c), 1e) oder 1g) oder ein dazu zu mindestens 90 % ähnliches Polynukleotid kodiert, und/oder einem Protein, das durch eine Nukleinsäure kodiert wird, die unter stringenten Bedingungen an ein Polynukleotid gemäss einer der Abbildungen 1a), 1c), 1e) oder 1g) oder deren Antisense Polynukleotide bindet,
und/oder einer Zelle und/oder einer Präparation aus einer solchen Zelle, die mindestens eines der vorgenannten Proteine bzw. Biomoleküle aus Gruppe II, synthetisiert hat
und
dass im anderen der zwei Verfahren, im folgenden Nebenverfahren genannt, im Schritt (a) die zu testende Substanz mit einem Biomolekül aus der Gruppe I oder mit einem Biomolekül aus Gruppe II inkubiert wird, aus der das Biomolekül, mit der die Substanz im Hauptverfahren inkubiert wird, nicht ausgewählt ist.

## Claims

1. Process for finding pharmaceutically relevant substances having activity.in the following indications or for the treatment of amyotrophic lateral sclerosis, diseases of the spinal motor neurone, muscular atrophies, muscular dystrophies or multiple sclerosis, comprising the following process steps:
(a) incubation of a substance to be tested under suitable conditions with at least one biomolecule of group I selected from:
the protein BNPI and/or a protein according to one of Figures 1b), 1d), 1f) or 1h) and/or a protein that is at least 90 % similar to one of the above-mentioned proteins and/or a protein coded for by a polynucleotide according to one of Figures 1a), 1c), 1e) or 1g) or by a polynucleotide that is at least 90 % similar thereto, and/or a protein coded for by a nucleic acid that binds under stringent conditions to a polynucleotide according to one of Figures 1a), 1c), 1e) or 1g) or their antisense polynucleotides,
and/or a cell and/or a preparation of such a cell that has synthesised at least one of the above-mentioned proteins, or biomolecules of group I,
(b) measurement of the binding of the test substance to the biomolecule(s) of group I optionally synthesised by a cell or to a cell and/or a preparation of such a cell that has synthesised at least one of the above-mentioned biomolecules of group I, or measurement of at least one functional parameter changed by the binding of the test substance to the biomolecule(s) of group I optionally synthesised by a cell or to a cell and/or a preparation of such a cell, that has synthesised at least one of the above-mentioned biomolecules of group I, via measurement of the regulation, inhibition and/or activation of receptors, ion channels and/or enzymes, especially via measurement of the change in the gene expression, the ionic environment, the pH or the membrane potential, via change in the enzyme activity or the concentration of the 2nd messenger,
or
via measurement of the binding via the displacement of a known labelled ligand of the biomolecule and/or protein and/or via the activity of a labelled test substance bonded thereto.

2. Process according to claim 1, **characterised in that** the cell is manipulated by genetic engineering before step (a).

3. Process according to claim 2, **characterised in that** the manipulation by genetic engineering permits the measurement of at least one of the functional parameters changed by the test substance.

4. Process according to claim 3, **characterised in that**, by means of the manipulation by genetic engineering, a form of a G-protein that is not expressed endogenously in the cell or a reporter gene is introduced.

5. Process according to any one of claims 2 to 4, **characterised in that** the cell is manipulated by genetic engineering in such a manner that the cell contains at least one polynucleotide according to one of Figures 1a), 1c), 1e) or 1g) or a polynucleotide that is at least 90 %, preferably at least 95 %, especially at least 97 %, similar thereto.

6. Process according to claim 5, **characterised in that** the polynucleotide is contained in a recombinant DNA construct.

7. Process according to any one of claims 2 to 6, **characterised in that**, after the manipulation by genetic engineering according to claim 2 and before step (a) according to claim 1, the cell is cultivated under conditions permitting expression, optionally under selection pressure.

8. Process according to any one of claims 1 to 7, **characterised in that** the cell is an amphibian cell, bacterial cell, yeast cell, insect cell or an immortalised or native mammalian cell.

9. Process according to any one of claims 1 to 8, in which a first process according to any one of claims 1 to 8 is coupled with a second process according to any one of claims 1 to 8 in such a manner that the measured values and results of the first process with regard to the substance to be measured are compared with the measured values and results of the second process with regard to the substance to be measured, **characterised in that** in one of the two processes, referred to hereinbelow as the main process, in step (a) the substance to be tested is
with a biomolecule selected from group II:
the protein BNPI and/or a protein according to one of Figures 1b), 1d), 1f) or 1h) and/or a protein that is at least 90 % similar to one of these above-mentioned proteins and/or a protein coded for by a polynucleotide according to one of Figures 1a), 1c), 1e) or 1g) or by a polynucleotide that is at least 90 % similar thereto, and/or a protein coded for by a nucleic acid that binds under stringent conditions to a polynucleotide according to one of Figures 1a), 1c), 1e) or 1g) or their antisense polynucleotides, and/or a cell and/or a preparation of such a cell that has synthesised at least one of the above-mentioned proteins or biomolecules of group II,
and
**in that** in the other of the two processes, referred to hereinbelow as the subsidiary process, in step (a) the substance to be tested is incubated with a biomolecule from group I or with a biomolecule from that group selected from group II from which the biomolecule with which the substance is incubated in the main process has not been chosen.

## Revendications

1. Méthode de recherche de substances présentant un intérêt pharmaceutique, douées d'activité dans les indications ou pour le traitement de la sclérose latérale amyotrophique, de maladies du motoneurone spinal, d'atrophies musculaires, de dystrophies musculaires ou de la sclérose en plaques, comprenant les étapes suivantes :
(a) incubation d'une substance à tester, dans des conditions convenables, avec au moins une biomolécule du Groupe I, choisie entre :
la protéine BNPI et/ou une protéine selon les figures 1b), 1d), 1f) ou 1h) et/ou une protéine semblable à au moins 90 % à l'une des protéines mentionnées ci-dessus, et/ou une protéine codée par un polynucléotide selon l'une des figures 1a), 1c), 1e) ou 1g) ou par un polynucléotide qui y est semblable à 90 % au moins, et/ou une protéine qui est codée par un acide nucléique qui se lie dans des conditions stringentes à un polynucléotide selon l'une des figures 1a), 1c), 1e) ou 1g) ou leurs polynucléotides anti-sens,
et/ou avec une cellule et/ou une préparation d'une cellule qui a synthétisé au moins l'une des protéines mentionnées ci-dessus, c'est-à-dire des biomolécules du Groupe I,
(b) mesure de la liaison de la substance testée à la biomolécule ou aux biomolécules du Groupe I éventuellement synthétisée(s) par une cellule, ou à une cellule et/ou à une préparation obtenue à partir d'une cellule qui a synthétisé au moins l'une des biomolécules du Groupe I mentionnées ci-dessus, ou mesure d'au moins l'un des paramètres fonctionnels modifiés par la liaison de la substance testée à la biomolécule ou aux biomolécules du Groupe I éventuellement synthétisée(s) par une cellule, ou à une cellule et/ou à une préparation obtenue à partir d'une cellule qui a synthétisé au moins l'une des biomolécules du Groupe I mentionnées ci-dessus, par l'intermédiaire de la mesure de la régulation, de l'inhibition et/ou de l'activation de récepteurs, de canaux ioniques et/ou d'enzymes, en particulier par l'intermédiaire de la mesure de la variation de l'expression génique, du milieu ionique, du pH ou du potentiel de membrane, par l'intermédiaire de la variation de l'activité enzymatique ou de la concentration du second messager,
ou
par mesure de la liaison au moyen du déplacement d'un ligand marqué connu de la biomolécule et/ou de la protéine, et/ou au moyen de l'activité qui y est associée d'une substance testée marquée.

2. Méthode selon la revendication 1, **caractérisée en ce que** la cellule est manipulée par génie génétique avant l'étape (a).

3. Méthode selon la revendication 2, **caractérisée en ce que** la manipulation par génie génétique permet la mesure d'au moins l'un des paramètres fonctionnels modifiés par la substance testée.

4. Méthode selon la revendication 3, **caractérisée en ce qu'**une forme d'une protéine G d'expression non endogène est exprimée dans la cellule ou un gène rapporteur est introduit.

5. Méthode selon l'une des revendications 2-4, **caractérisée en ce que** la cellule est manipulée par génie génétique de façon telle qu'elle contienne au moins un polynucléotide selon l'une des figures 1a), 1c), 1e) ou 1g) ou un polynucléotide qui y est semblable en une proportion d'au moins 90 %, avantageusement d'au moins 95 %, notamment d'au moins 97 %.

6. Méthode selon la revendication 5, **caractérisée en ce que** le polynucléotide est contenu dans un produit d'assemblage d'ADN recombiné.

7. Méthode selon l'une des revendications 2 à 6, **caractérisée en ce que** la cellule est cultivée, après la manipulation de génie génétique selon la revendication 2 et avant l'étape (a) selon la revendication 1, dans des conditions qui permettent une expression, le cas échéant sous pression de sélection.

8. Méthode selon l'une des revendications 1 à 7, **caractérisée en ce que** la cellule est une cellule d'amphibien, une cellule de bactérie, une cellule de levure, une cellule d'insecte ou une cellule immortalisée ou native de mammifère.

9. Méthode selon l'une des revendications 1 à 8, dans laquelle une première méthode, selon l'une des revendications 1 à 8, est couplée à une seconde méthode, selon l'une des revendications 1 à 8, de façon telle que les valeurs mesurées et les résultats de la première méthode, en ce qui concerne la substance à mesurer, sont comparés aux valeurs de mesure et aux résultats de la seconde méthode en ce qui concerne la substance à mesurer, **caractérisée en ce que** dans l'une des deux méthodes, appelée méthode principale ci-après, au cours de l'étape (a), la substance à tester
est liée à une molécule choisie dans le Groupe II :
la protéine BNPI et/ou une protéine selon l'une des figures 1b), 1d), 1f) ou 1h) et/ou une protéine semblable à au moins 90 % à l'une de ces protéines mentionnées ci-dessus, et/ou une protéine codée par un polynucléotide selon l'une des figures 1a), 1c), 1e) ou 1g) ou par un polynucléotide qui y est semblable à 90 % au moins, et/ou une protéine qui est codée par un acide nucléique qui se lie dans des conditions stringentes à un polynucléotide selon l'une des figures 1a), 1c), 1e) ou 1g) ou leurs polynucléotides anti-sens,
et/ou avec une cellule et/ou avec une préparation obtenue à partir d'une cellule, qui a synthétisé au moins l'une des protéines, c'est-à-dire des biomolécules du Groupe II mentionnées ci-dessus, et
**en ce que**, dans l'autre des deux méthodes, appelée ci-après méthode secondaire, au cours de l'étape (a), la substance à tester est mise à incuber avec une biomolécule du Groupe I ou avec une biomolécule du Groupe II dans lequel la biomolécule avec laquelle la substance qui est en incubation dans la méthode principale n'est pas choisie.
